# FASCICULE DE BREVET EUROPEEN

(11) **EP 0 781 342 B1**
(45) Date de publication et mention de la délivrance du brevet: **09.08.2006**
(21) Numéro de dépôt: 95931256.2
(22) Date de dépôt: 15.09.1995
(51) Int. Cl.: C12N 15/82, C12N 15/54, C12N 15/34, C07K 14/01, A01N 63/02, A01H 5/00

(54) **PLANTES ET SEMENCES TRANSGENIQUES RESISTANTES AUX VIRUS A ADN PHYTOPATHOGENES, ET LEURS PROCEDES D'OBTENTION**
PHYTOPATHOGENE DNA-VIRUS-RESISTENTE TRANSGENE PFLANZEN UND SAATEN, UND VERFAHREN ZU IHRER HERSTELLUNG
PHYTOPATHOGENIC DNA VIRUS RESISTANT TRANSGENIC PLANTS AND SEEDS AND METHODS FOR OBTAINING SAME

(30) Priorité: 15.09.1994 FR 9411040
(43) Date de publication de la demande: 02.07.1997
(73) Titulaire: CENTRE NATIONAL DE LA RECHERCHE SCIENTIFIQUE, 75016 Paris Cédex (FR)
(72) Inventeur: GRONENBORN, Bruno, F-91190 Gif-sur-Yvette (FR)
(74) Mandataire: Grosset-Fournier, Chantal Catherine
(86) Numéro de dépôt international: PCT/FR1995/001192
(87) Numéro de publication internationale: WO 1996/008573

(56) Documents cités:
- WO-A-95/03404
- MOL. BIOL. TOMATO [PROC. SYMP.] (1993), 227-38. EDITOR(S): YODER,JOHN I. PUBLISHER: TECHNOMIC, LANCASTER, PA., DE KOUCHKOVSKY, F., ET AL. 'Molecular biology of tomato yellow leaf curl virus (TYLCV) and potential ways to control the disease'
- PROCEEDINGS OF THE NATIONAL ACADEMY OF SCIENCES OF USA, vol. 90, WASHINGTON US, pages 3134-3141, WILSON, T.M.A. 'STRATEGIES TO PROTECT CROP PLANTS AGAINST VIRUSES: PATHOGEN-DERIVED RESISTANCE BLOSSOMS'
- ANNUAL MEETING OF THE AMERICAN PHYTOPATHOLOGICAL SOCIETY, ST. LOUIS, MISSOURI, USA, AUGUST 17-21, 1991. PHYTOPATHOLOGY 81 (10). 1991. 1247., HANSON S F ET AL 'SITE-SPECIFIC MUTATIONS IN CODONS OF THE PUTATIVE NTP-BINDING MOTIF OF THE AL1 GENE OF BEAN GOLDEN MOSAIC GEMINIVIRUS ABOLISH INFECTIVITY.'
- VIROLOGY, vol. 187, pages 555-564, VON ARNIM, A., ET AL. 'INHIBITION OF AFRICAN CASSAVA MOSAIC VIRUS SYSTEMIC INFECTION BY A MOVEMENT PROTEIN FROM THE RELATED GEMINIVIRUS TOMATO GOLDEN MOSAIC VIRUS' cité dans la demande
- PROCEEDINGS OF THE NATIONAL ACADEMY OF SCIENCES OF USA, vol. 88, WASHINGTON US, pages 6721-6725, DAY, A.G., ET AL. 'EXPRESSION OF AN ANTISENSE VIRAL GENE IN TRANSGENIC TOBACCO CONFERS RESISTANCE TO THE DNA VIRUS TOMATO GOLDEN MOSAIC VIRUS' cité dans la demande
- PROCEEDINGS OF THE NATIONAL ACADEMY OF SCIENCES OF USA, vol. 87, WASHINGTON US, pages 6291-6295, STANLEY, J., ET AL. 'DEFECTIVE VIRAL DNA AMELIORATES SYMPTOMS OF GEMINIVIRUS INFECTION IN TRANSGENIC PLANTS'
- BIOTECHNOLOGY, vol. 12, no. 5, NEW YORK US, pages 500-504, KUNIK, T., ET AL. 'TRANSGENIC TOMATO PLANTS EXPRESSING THE TOMATO YELLOW LEAF CURL VIRUS CAPSID PROTEIN ARE RESISTANT TO THE VIRUS'

(1) INFORMATION GENERALE:
   (i) DEPOSANT:
      (A) NOM: CENTRE NATIONAL DE LA RECHERCHE SCIENTIFIQUE
      (B) RUE: 3, rue Michel-Ange
      (C) VILLE: PARIS
      (E) PAYS: FRANCE
      (F) CODE POSTAL: 75794 CEDEX 16
   (ii) TITRE DE L' INVENTION: PLANTES ET SEMENCES TRANSGENIQUES RESISTANTES AUX VIRUS A ADN PHYTOPATHOGENES, ET LEURS PROCEDES D'OBTENTION
   (iii) NOMBRE DE SEQUENCES: 8
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
   (v) DONNEES DE LA DEMANDE ACTUELLE:
      NUMERO DE DEPOT: PCT/FR95/01192
   (vi) DONNEES DE LA DEMANDE ANTERIEURE:
      (A) NUMERO DE DEPOT: FR 94.11040
      (B) DATE DE DEPOT: 15-SEP-1994
(2) INFORMATION POUR LA SEQ ID NO: 1:
   (i) CARACTERISTIQUES DE LA SEQUENCE:
      (A) LONGUEUR: 1080 paires de bases
      (B) TYPE: acide nucléique
      (C) NOMBRE DE BRINS: double
      (D) CONFIGURATION: linéaire
   (ii) TYPE DE MOLECULE: ADN génomique
   (ix) CARACTERISTIQUE ADDITIONELLE:
      (A) NOM/CLE: CDS
      (B) EMPLACEMENT: 1..1077
   (xi) DESCRIPTION DE LA SEQUENCE: SEQ ID NO: 1:
   ATG CCA AGA TCA GGT CGT TTT AGT ATC AAG GCT AAA AAT TAT TTC CTT          48
   Met Pro Arg Ser Gly Arg Phe Ser Ile Lys Ala Lys Asn Tyr Phe Leu
     1               5                  10                  15
   ACA TAT CCC AAA TGT GAT TTA ACA AAA GAA AAT GCA CTT TCC CAA ATA          96
   Thr Tyr Pro Lys Cys Asp Leu Thr Lys Glu Asn Ala Leu Ser Gln Ile
                20                  25                  30
   ACA AAC CTA CAA ACA CCC ACA AAC AAA TTA TTC ATC AAA ATT TGC AGA       144
   Thr Asn Leu Gln Thr Pro Thr Asn Lys Leu Phe Ile Lys Ile Cys Arg
                35                  40                  45
   GAA CTA CAT GAA AAT GGG GAA CCT CAT CTC CAT ATT CTC ATC CAA TTC       192
   Glu Leu His Glu Asn Gly Glu Pro His Leu His Ile Leu Ile Gln Phe
        50                  55                  60
   GAA GGA AAA TAC AAT TGT ACC AAT CAA CGA TTC TTC GAC CTG GTA TCC       240
   Glu Gly Lys Tyr Asn Cys Thr Asn Gln Arg Phe Phe Asp Leu Val Ser
    65                  70                  75                  80
   CCA ACC AGG TCA GCA CAT TTC CAT CCG AAC ATT CAG GGA GCT AAA TCG       288
   Pro Thr Arg Ser Ala His Phe His Pro Asn Ile Gln Gly Ala Lys Ser
                    85                  90                  95
   AGC TCC GAC GTC AAG TCC TAT ATC GAC AAG GAC GGA GAT GTT CTT GAA       336
   Ser Ser Asp Val Lys Ser Tyr Ile Asp Lys Asp Gly Asp Val Leu Glu
               100                 105                 110
   TGG GGT ACT TTC CAG ATC GAC GGA CGA TCT GCT AGG GGA GGA CAA CAG       384
   Trp Gly Thr Phe Gln Ile Asp Gly Arg Ser Ala Arg Gly Gly Gln Gln
           115                 120                 125
   ACA GCC AAC GAC GCT TAC GCA AAG GCA ATT AAC GCA GGA AGT AAG TCG       432
   Thr Ala Asn Asp Ala Tyr Ala Lys Ala Ile Asn Ala Gly Ser Lys Ser
       130                 135                 140
   CAG GCT CTT GAT GTA ATT AAA GAA TTA GCG CCT AGA GAT TAC GTT CTA       480
   Gln Ala Leu Asp Val Ile Lys Glu Leu Ala Pro Arg Asp Tyr Val Leu
   145                 150                 155                 160
   CAT TTT CAT AAT ATA AAT AGT AAT TTA GAT AAG GTT TTC CAG GTG CCT       528
   His Phe His Asn Ile Asn Ser Asn Leu Asp Lys Val Phe Gln Val Pro
                   165                 170                 175
   CCG GCA CCT TAT GTT TCT CCT TTT TTA TCT TCT TCT TTC GAT CAA GTT       576
   Pro Ala Pro Tyr Val Ser Pro Phe Leu Ser Ser Ser Phe Asp Gln Val
               180                 185                 190
   CCT GAT GAA CTT GAA CAC TGG GTT TCC GAG AAC GTC ATG GAT GCC GCT       624
   Pro Asp Glu Leu Glu His Trp Val Ser Glu Asn Val Met Asp Ala Ala
           195                 200                 205
   GCG CGG CCT TGG AGA CCG GTG AGT ATA GTG ATT GAG GGT GAC AGC CGG       672
   Ala Arg Pro Trp Arg Pro Val Ser Ile Val Ile Glu Gly Asp Ser Arg
       210                 215                 220
   ACA GGA AAG ACA ACG TGG GCC CGT TCA TTA GGC CCA CAT AAT TAT TTG       720
   Thr Gly Lys Thr Thr Trp Ala Arg Ser Leu Gly Pro His Asn Tyr Leu
   225                 230                 235                 240
   TGC GGC CAT CTT GAC CTC AGT CAA AAA GTA TAC AGC AAT AAT GCT TGG       768
   Cys Gly His Leu Asp Leu Ser Gln Lys Val Tyr Ser Asn Asn Ala Trp
                   245                 250                 255
   TAT AAC GTC ATT GAT GAC GTC GAC CCG CAT TAT TTA AAA CAC TTT AAA         816
   Tyr Asn Val Ile Asp Asp Val Asp Pro His Tyr Leu Lys His Phe Lys
               260                 265                 270
   GAA TTT ATG GGG GCC CAA AGA GAT TGG CAA AGC AAC ACA AAG TAT GGC         864
   Glu Phe Met Gly Ala Gln Arg Asp Trp Gln Ser Asn Thr Lys Tyr Gly
           275                 280                 285
   AAG CCC ATT CAA ATT AAA GGA GGC ATT CCC ACT ATC TTC CTA TGC AAT         912
   Lys Pro Ile Gln Ile Lys Gly Gly Ile Pro Thr Ile Phe Leu Cys Asn
       290                 295                 300
   CCA GGC CCA CAA TCA TCA TTT AAA GAA TAT CTC GAC GAA GAA AAA AAT         960
   Pro Gly Pro Gln Ser Ser Phe Lys Glu Tyr Leu Asp Glu Glu Lys Asn
   305                 310                 315                 320
   CAA GCA TTA AAA AAC TGG GCT ACT AAG AAT GCA ATC TTC GTC ACC ATC        1008
   Gln Ala Leu Lys Asn Trp Ala Thr Lys Asn Ala Ile Phe Val Thr Ile
                   325                 330                 335
   CAC CAG CCA TTG TTC GCA GAT ACC AAT CAA AAT ACA ACA TCA CAT CGC        1056
   His Gln Pro Leu Phe Ala Asp Thr Asn Gln Asn Thr Thr Ser His Arg
               340                 345                 350
   CAA GAA GAG GCA AGT GAG GCG TAG                                        1080
   Gln Glu Glu Ala Ser Glu Ala
           355
(2) INFORMATION POUR LA SEQ ID NO: 2:
   (i) CARACTERISTIQUES DE LA SEQUENCE:
      (A) LONGUEUR: 359 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Pro Arg Ser Gly Arg Phe Ser Ile Lys Ala Lys Asn Tyr Phe Leu
    1               5                  10                  15
   Thr Tyr Pro Lys Cys Asp Leu Thr Lys Glu Asn Ala Leu Ser Gln Ile
               20                  25                  30
   Thr Asn Leu Gln Thr Pro Thr Asn Lys Leu Phe Ile Lys Ile Cys Arg
           35                  40                  45
   Glu Leu His Glu Asn Gly Glu Pro His Leu His Ile Leu Ile Gln Phe
       50                  55                  60
   Glu Gly Lys Tyr Asn Cys Thr Asn Gln Arg Phe Phe Asp Leu Val Ser
    65                 70                  75                  80
   Pro Thr Arg Ser Ala His Phe His Pro Asn Ile Gln Gly Ala Lys Ser
                   85                  90                  95
   Ser Ser Asp Val Lys Ser Tyr Ile Asp Lys Asp Gly Asp Val Leu Glu
               100                 105                 110
   Trp Gly Thr Phe Gln Ile Asp Gly Arg Ser Ala Arg Gly Gly Gln Gln
           115                 120                 125
   Thr Ala Asn Asp Ala Tyr Ala Lys Ala Ile Asn Ala Gly Ser Lys Ser
       130                 135                 140
   Gln Ala Leu Asp Val Ile Lys Glu Leu Ala Pro Arg Asp Tyr Val Leu
   145                 150                 155                 160
   His Phe His Asn Ile Asn Ser Asn Leu Asp Lys Val Phe Gln Val Pro
                   165                 170                 175
   Pro Ala Pro Tyr Val Ser Pro Phe Leu Ser Ser Ser Phe Asp Gln Val
               180                 185                 190
   Pro Asp Glu Leu Glu His Trp Val Ser Glu Asn Val Met Asp Ala Ala
           195                 200                 205
   Ala Arg Pro Trp Arg Pro Val Ser Ile Val Ile Glu Gly Asp Ser Arg
       210                 215                 220
   Thr Gly Lys Thr Thr Trp Ala Arg Ser Leu Gly Pro His Asn Tyr Leu
   225                 230                 235                 240
   Cys Gly His Leu Asp Leu Ser Gln Lys Val Tyr Ser Asn Asn Ala Trp
                   245                 250                 255
   Tyr Asn Val Ile Asp Asp Val Asp Pro His Tyr Leu Lys His Phe Lys
               260                 265                 270
   Glu Phe Met Gly Ala Gln Arg Asp Trp Gln Ser Asn Thr Lys Tyr Gly
           275                 280                 285
   Lys Pro Ile Gln Ile Lys Gly Gly Ile Pro Thr Ile Phe Leu Cys Asn
       290                 295                 300
   Pro Gly Pro Gln Ser Ser Phe Lys Glu Tyr Leu Asp Glu Glu Lys Asn
   305                 310                 315                 320
   Gln Ala Leu Lys Asn Trp Ala Thr Lys Asn Ala Ile Phe Val Thr Ile
                   325                 330                 335
   His Gln Pro Leu Phe Ala Asp Thr Asn Gln Asn Thr Thr Ser His Arg
               340                 345                 350
   Gln Glu Glu Ala Ser Glu Ala
           355
(2) INFORMATION POUR LA SEQ ID NO: 3:
   (i) CARACTERISTIQUES DE LA SEQUENCE:
      (A) LONGUEUR: 1080 paires de bases
      (B) TYPE: acide nucléique
      (C) NOMBRE DE BRINS: double
      (D) CONFIGURATION: linéaire
   (ii) TYPE DE MOLECULE: ADN génomique
   (ix) CARACTERISTIQUE ADDITIONELLE:
      (A) NOM/CLE: CDS
      (B) EMPLACEMENT: 1..1077
   (xi) DESCRIPTION DE LA SEQUENCE: SEQ ID NO: 3:
   ATG CCA AGA TCA GGT CGT TTT AGT ATC AAG GCT AAA AAT TAT TTC CTT       48
   Met Pro Arg Ser Gly Arg Phe Ser Ile Lys Ala Lys Asn Tyr Phe Leu
     1               5                  10                  15
   ACA TAT CCC AAA TGT GAT TTA ACA AAA GAA AAT GCA CTT TCC CAA ATA       96
   Thr Tyr Pro Lys Cys Asp Leu Thr Lys Glu Asn Ala Leu Ser Gln Ile
                20                  25                  30
   ACA AAC CTA CAA ACA CCC ACA AAC AAA TTA TTC ATC AAA ATT TGC AGA      144
   Thr Asn Leu Gln Thr Pro Thr Asn Lys Leu Phe Ile Lys Ile Cys Arg
            35                  40                  45
   GAA CTA CAT GAA AAT GGG GAA CCT CAT CTC CAT ATT CTC ATC CAA TTC      192
   Glu Leu His Glu Asn Gly Glu Pro His Leu His Ile Leu Ile Gln Phe
        50                  55                  60
   GAA GGA AAA TAC AAT TGT ACC AAT CAA CGA TTC TTC GAC CTG GTA TCC      240
   Glu Gly Lys Tyr Asn Cys Thr Asn Gln Arg Phe Phe Asp Leu Val Ser
    65                  70                  75                  80
   CCA ACC AGG TCA GCA CAT TTC CAT CCG AAC ATT CAG GGA GCT AAA TCG      288
   Pro Thr Arg Ser Ala His Phe His Pro Asn Ile Gln Gly Ala Lys Ser
                    85                  90                  95
   AGC TCC GAC GTC AAG TCC TAT ATC GAC AAG GAC GGA GAT GTT CTT GAA      336
   Ser Ser Asp Val Lys Ser Tyr Ile Asp Lys Asp Gly Asp Val Leu Glu
               100                 105                 110
   TGG GGT ACT TTC CAG ATC GAC GGA CGA TCT GCT AGG GGA GGA CAA CAG      384
   Trp Gly Thr Phe Gln Ile Asp Gly Arg Ser Ala Arg Gly Gly Gln Gln
           115                 120                 125
   ACA GCC AAC GAC GCT TAC GCA AAG GCA ATT AAC GCA GGA AGT AAG TCG      432
   Thr Ala Asn Asp Ala Tyr Ala Lys Ala Ile Asn Ala Gly Ser Lys Ser
       130                 135                 140
   CAG GCT CTT GAT GTA ATT AAA GAA TTA GCG CCT AGA GAT TAC GTT CTA      480
   Gln Ala Leu Asp Val Ile Lys Glu Leu Ala Pro Arg Asp Tyr Val Leu
   145                 150                 155                 160
   CAT TTT CAT AAT ATA AAT AGT AAT TTA GAT AAG GTT TTC CAG GTG CCT      528
   His Phe His Asn Ile Asn Ser Asn Leu Asp Lys Val Phe Gln Val Pro
                   165                 170                 175
   CCG GCA CCT TAT GTT TCT CCT TTT TTA TCT TCT TCT TTC GAT CAA GTT      576
   Pro Ala Pro Tyr Val Ser Pro Phe Leu Ser Ser Ser Phe Asp Gln Val
               180                 185                 190
   CCT GAT GAA CTT GAA CAC TGG GTT TCC GAG AAC GTC ATG GAT GCC GCT        624
   Pro Asp Glu Leu Glu His Trp Val Ser Glu Asn Val Met Asp Ala Ala
           195                 200                 205
   GCG CGG CCT TGG AGA CCG GTG AGT ATA GTG ATT GAG GGT GAC AGC CGG        672
   Ala Arg Pro Trp Arg Pro Val Ser Ile Val Ile Glu Gly Asp Ser Arg
       210                 215                 220
   ACA GGA GCG ACC ACG TGG GCC CGT TCA TTA GGC CCA CAT AAT TAT TTG        720
   Thr Gly Ala Thr Thr Trp Ala Arg Ser Leu Gly Pro His Asn Tyr Leu
   225                 230                 235                 240
   TGC GGC CAT CTT GAC CTC AGT CAA AAA GTA TAC AGC AAT AAT GCT TGG        768
   Cys Gly His Leu Asp Leu Ser Gln Lys Val Tyr Ser Asn Asn Ala Trp
                   245                 250                 255
   TAT AAC GTC ATT GAT GAC GTC GAC CCG CAT TAT TTA AAA CAC TTT AAA        816
   Tyr Asn Val Ile Asp Asp Val Asp Pro His Tyr Leu Lys His Phe Lys
               260                 265                 270
   GAA TTT ATG GGG GCC CAA AGA GAT TGG CAA AGC AAC ACA AAG TAT GGC        864
   Glu Phe Met Gly Ala Gln Arg Asp Trp Gln Ser Asn Thr Lys Tyr Gly
           275                 280                 285
   AAG CCC ATT CAA ATT AAA GGA GGC ATT CCC ACT ATC TTC CTA TGC AAT        912
   Lys Pro Ile Gln Ile Lys Gly Gly Ile Pro Thr Ile Phe Leu Cys Asn
       290                 295                 300
   CCA GGC CCA CAA TCA TCA TTT AAA GAA TAT CTC GAC GAA GAA AAA AAT        960
   Pro Gly Pro Gln Ser Ser Phe Lys Glu Tyr Leu Asp Glu Glu Lys Asn
   305                 310                 315                 320
   CAA GCA TTA AAA AAC TGG GCT ACT AAG AAT GCA ATC TTC GTC ACC ATC       1008
   Gln Ala Leu Lys Asn Trp Ala Thr Lys Asn Ala Ile Phe Val Thr Ile
                   325                 330                 335
   CAC CAG CCA TTG TTC GCA GAT ACC AAT CAA AAT ACA ACA TCA CAT CGC       1056
   His Gln Pro Leu Phe Ala Asp Thr Asn Gln Asn Thr Thr Ser His Arg
               340                 345                 350
   CAA GAA GAG GCA AGT GAG GCG TAG                                       1080
   Gln Glu Glu Ala Ser Glu Ala
           355
(2) INFORMATION POUR LA SEQ ID NO: 4:
   (i) CARACTERISTIQUES DE LA SEQUENCE:
      (A) LONGUEUR: 359 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 4:
   Met Pro Arg Ser Gly Arg Phe Ser Ile Lys Ala Lys Asn Tyr Phe Leu
    1               5                  10                  15
   Thr Tyr Pro Lys Cys Asp Leu Thr Lys Glu Asn Ala Leu Ser Gln Ile
               20                  25                  30
   Thr Asn Leu Gln Thr Pro Thr Asn Lys Leu Phe Ile Lys Ile Cys Arg
           35                  40                  45
   Glu Leu His Glu Asn Gly Glu Pro His Leu His Ile Leu Ile Gln Phe
       50                  55                  60
   Glu Gly Lys Tyr Asn Cys Thr Asn Gln Arg Phe Phe Asp Leu Val Ser
   65                  70                  75                  80
   Pro Thr Arg Ser Ala His Phe His Pro Asn Ile Gln Gly Ala Lys Ser
                   85                  90                  95
   Ser Ser Asp Val Lys Ser Tyr Ile Asp Lys Asp Gly Asp Val Leu Glu
               100                 105                 110
   Trp Gly Thr Phe Gln Ile Asp Gly Arg Ser Ala Arg Gly Gly Gln Gln
           115                 120                 125
   Thr Ala Asn Asp Ala Tyr Ala Lys Ala Ile Asn Ala Gly Ser Lys Ser
       130                 135                 140
   Gln Ala Leu Asp Val Ile Lys Glu Leu Ala Pro Arg Asp Tyr Val Leu
   145                 150                 155                 160
   His Phe His Asn Ile Asn Ser Asn Leu Asp Lys Val Phe Gln Val Pro
                   165                 170                 175
   Pro Ala Pro Tyr Val Ser Pro Phe Leu Ser Ser Ser Phe Asp Gln Val
               180                 185                 190
   Pro Asp Glu Leu Glu His Trp Val Ser Glu Asn Val Met Asp Ala Ala
           195                 200                 205
   Ala Arg Pro Trp Arg Pro Val Ser Ile Val Ile Glu Gly Asp Ser Arg
       210                 215                 220
   Thr Gly Ala Thr Thr Trp Ala Arg Ser Leu Gly Pro His Asn Tyr Leu
   225                 230                 235                 240
   Cys Gly His Leu Asp Leu Ser Gln Lys Val Tyr Ser Asn Asn Ala Trp
                   245                 250                 255
   Tyr Asn Val Ile Asp Asp Val Asp Pro His Tyr Leu Lys His Phe Lys
               260                 265                 270
   Glu Phe Met Gly Ala Gln Arg Asp Trp Gln Ser Asn Thr Lys Tyr Gly
           275                 280                 285
   Lys Pro Ile Gln Ile Lys Gly Gly Ile Pro Thr Ile Phe Leu Cys Asn
       290                 295                 300
   Pro Gly Pro Gln Ser Ser Phe Lys Glu Tyr Leu Asp Glu Glu Lys Asn
   305                 310                 315                 320
   Gln Ala Leu Lys Asn Trp Ala Thr Lys Asn Ala Ile Phe Val Thr Ile
                   325                 330                 335
   His Gln Pro Leu Phe Ala Asp Thr Asn Gln Asn Thr Thr Ser His Arg
               340                 345                 350
   Gln Glu Glu Ala Ser Glu Ala
           355
(2) INFORMATION POUR LA SEQ ID NO: 5:
   (i) CARACTERISTIQUES DE LA SEQUENCE:
      (A) LONGUEUR: 1080 paires de bases
      (B) TYPE: acide nucléique
      (C) NOMBRE DE BRINS: double
      (D) CONFIGURATION: linéaire
   (ii) TYPE DE MOLECULE: ADN génomique
   (ix) CARACTERISTIQUE ADDITIONELLE:
      (A) NOM/CLE: CDS
      (B) EMPLACEMENT: 1..1077
   (xi) DESCRIPTION DE LA SEQUENCE: SEQ ID NO: 5:
   ATG CCA AGA TCA GGT CGT TTT AGT ATC AAG GCT AAA AAT TAT TTC CTT          48
   Met Pro Arg Ser Gly Arg Phe Ser Ile Lys Ala Lys Asn Tyr Phe Leu
     1               5                  10                  15
   ACA TAT CCC AAA TGT GAT TTA ACA AAA GAA AAT GCA CTT TCC CAA ATA          96
   Thr Tyr Pro Lys Cys Asp Leu Thr Lys Glu Asn Ala Leu Ser Gln Ile
                20                  25                  30
   ACA AAC CTA CAA ACA CCC ACA AAC AAA TTA TTC ATC AAA ATT TGC AGA         144
   Thr Asn Leu Gln Thr Pro Thr Asn Lys Leu Phe Ile Lys Ile Cys Arg
            35                  40                  45
   GAA CTA CAT GAA AAT GGG GAA CCT CAT CTC CAT ATT CTC ATC CAA TTC         192
   Glu Leu His Glu Asn Gly Glu Pro His Leu His Ile Leu Ile Gln Phe
        50                  55                  60
   GAA GGA AAA TAC AAT TGT ACC AAT CAA CGA TTC TTC GAC CTG GTA TCC         240
   Glu Gly Lys Tyr Asn Cys Thr Asn Gln Arg Phe Phe Asp Leu Val Ser
    65                  70                  75                  80
   CCA ACC AGG TCA GCA CAT TTC CAT CCG AAC ATT CAG GGA GCT AAA TCG         288
   Pro Thr Arg Ser Ala His Phe His Pro Asn Ile Gln Gly Ala Lys Ser
                    85                  90                  95
   AGC TCC GAC GTC AAG TCC TAT ATC GAC AAG GAC GGA GAT GTT CTT GAA         336
   Ser Ser Asp Val Lys Ser Tyr Ile Asp Lys Asp Gly Asp Val Leu Glu
               100                 105                 110
   TGG GGT ACT TTC CAG ATC GAC GGA CGA TCT GCT AGG GGA GGA CAA CAG         384
   Trp Gly Thr Phe Gln Ile Asp Gly Arg Ser Ala Arg Gly Gly Gln Gln
           115                 120                 125
   ACA GCC AAC GAC GCT TAC GCA AAG GCA ATT AAC GCA GGA AGT AAG TCG         432
   Thr Ala Asn Asp Ala Tyr Ala Lys Ala Ile Asn Ala Gly Ser Lys Ser
       130                 135                 140
   CAG GCT CTT GAT GTA ATT AAA GAA TTA GCG CCT AGA GAT TAC GTT CTA         480
   Gln Ala Leu Asp Val Ile Lys Glu Leu Ala Pro Arg Asp Tyr Val Leu
   145                 150                 155                 160
   CAT TTT CAT AAT ATA AAT AGT AAT TTA GAT AAG GTT TTC CAG GTG CCT         528
   His Phe His Asn Ile Asn Ser Asn Leu Asp Lys Val Phe Gln Val Pro
                   165                 170                 175
   CCG GCA CCT TAT GTT TCT CCT TTT TTA TCT TCT TCT TTC GAT CAA GTT         576
   Pro Ala Pro Tyr Val Ser Pro Phe Leu Ser Ser Ser Phe Asp Gln Val
               180                 185                 190
   CCT GAT GAA CTT GAA CAC TGG GTT TCC GAG AAC GTC ATG GAT GCC GCT         624
   Pro Asp Glu Leu Glu His Trp Val Ser Glu Asn Val Met Asp Ala Ala
           195                 200                 205
   GCG CGG CCT TGG AGA CCG GTG AGT ATA GTG ATT GAG GGT GAC AGC CGG         672
   Ala Arg Pro Trp Arg Pro Val Ser Ile Val Ile Glu Gly Asp Ser Arg
       210                 215                 220
   ACA GGA CAT ACC ACG TGG GCC CGT TCA TTA GGC CCA CAT AAT TAT TTG         720
   Thr Gly His Thr Thr Trp Ala Arg Ser Leu Gly Pro His Asn Tyr Leu
   225                 230                 235                 240
   TGC GGC CAT CTT GAC CTC AGT CAA AAA GTA TAC AGC AAT AAT GCT TGG         768
   Cys Gly His Leu Asp Leu Ser Gln Lys Val Tyr Ser Asn Asn Ala Trp
                   245                 250                 255
   TAT AAC GTC ATT GAT GAC GTC GAC CCG CAT TAT TTA AAA CAC TTT AAA         816
   Tyr Asn Val Ile Asp Asp Val Asp Pro His Tyr Leu Lys His Phe Lys
               260                 265                 270
   GAA TTT ATG GGG GCC CAA AGA GAT TGG CAA AGC AAC ACA AAG TAT GGC         864
   Glu Phe Met Gly Ala Gln Arg Asp Trp Gln Ser Asn Thr Lys Tyr Gly
           275                 280                 285
   AAG CCC ATT CAA ATT AAA GGA GGC ATT CCC ACT ATC TTC CTA TGC AAT         912
   Lys Pro Ile Gln Ile Lys Gly Gly Ile Pro Thr Ile Phe Leu Cys Asn
       290                 295                 300
   CCA GGC CCA CAA TCA TCA TTT AAA GAA TAT CTC GAC GAA GAA AAA AAT         960
   Pro Gly Pro Gln Ser Ser Phe Lys Glu Tyr Leu Asp Glu Glu Lys Asn
   305                 310                 315                 320
   CAA GCA TTA AAA AAC TGG GCT ACT AAG AAT GCA ATC TTC GTC ACC ATC        1008
   Gln Ala Leu Lys Asn Trp Ala Thr Lys Asn Ala Ile Phe Val Thr Ile
                   325                 330                 335
   CAC CAG CCA TTG TTC GCA GAT ACC AAT CAA AAT ACA ACA TCA CAT CGC       1056
   His Gln Pro Leu Phe Ala Asp Thr Asn Gln Asn Thr Thr Ser His Arg
               340                 345                 350
   CAA GAA GAG GCA AGT GAG GCG TAG                                       1080
   Gln Glu Glu Ala Ser Glu Ala
           355
(2) INFORMATION POUR LA SEQ ID NO: 6:
   (i) CARACTERISTIQUES DE LA SEQUENCE:
      (A) LONGUEUR: 359 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 6:
   Met Pro Arg Ser Gly Arg Phe Ser Ile Lys Ala Lys Asn Tyr Phe Leu
    1               5                  10                  15
   Thr Tyr Pro Lys Cys Asp Leu Thr Lys Glu Asn Ala Leu Ser Gln Ile
               20                  25                  30
   Thr Asn Leu Gln Thr Pro Thr Asn Lys Leu Phe Ile Lys Ile Cys Arg
           35                  40                  45
   Glu Leu His Glu Asn Gly Glu Pro His Leu His Ile Leu Ile Gln Phe
       50                  55                  60
   Glu Gly Lys Tyr Asn Cys Thr Asn Gln Arg Phe Phe Asp Leu Val Ser
   65                  70                  75                  80
   Pro Thr Arg Ser Ala His Phe His Pro Asn Ile Gln Gly Ala Lys Ser
                   85                  90                  95
   Ser Ser Asp Val Lys Ser Tyr Ile Asp Lys Asp Gly Asp Val Leu Glu
               100                 105                 110
   Trp Gly Thr Phe Gln Ile Asp Gly Arg Ser Ala Arg Gly Gly Gln Gln
           115                 120                 125
   Thr Ala Asn Asp Ala Tyr Ala Lys Ala Ile Asn Ala Gly Ser Lys Ser
       130                 135                 140
   Gln Ala Leu Asp Val Ile Lys Glu Leu Ala Pro Arg Asp Tyr Val Leu
   145                 150                 155                 160
   His Phe His Asn Ile Asn Ser Asn Leu Asp Lys Val Phe Gln Val Pro
                   165                 170                 175
   Pro Ala Pro Tyr Val Ser Pro Phe Leu Ser Ser Ser Phe Asp Gln Val
               180                 185                 190
   Pro Asp Glu Leu Glu His Trp Val Ser Glu Asn Val Met Asp Ala Ala
           195                 200                 205
   Ala Arg Pro Trp Arg Pro Val Ser Ile Val Ile Glu Gly Asp Ser Arg
       210                 215                 220
   Thr Gly His Thr Thr Trp Ala Arg Ser Leu Gly Pro His Asn Tyr Leu
   225                 230                 235                 240
   Cys Gly His Leu Asp Leu Ser Gln Lys Val Tyr Ser Asn Asn Ala Trp
                   245                 250                 255
   Tyr Asn Val Ile Asp Asp Val Asp Pro His Tyr Leu Lys His Phe Lys
               260                 265                 270
   Glu Phe Met Gly Ala Gln Arg Asp Trp Gln Ser Asn Thr Lys Tyr Gly
           275                 280                 285
   Lys Pro Ile Gln Ile Lys Gly Gly Ile Pro Thr Ile Phe Leu Cys Asn
       290                 295                 300
   Pro Gly Pro Gln Ser Ser Phe Lys Glu Tyr Leu Asp Glu Glu Lys Asn
   305                 310                 315                 320
   Gln Ala Leu Lys Asn Trp Ala Thr Lys Asn Ala Ile Phe Val Thr Ile
                   325                 330                 335
   His Gln Pro Leu Phe Ala Asp Thr Asn Gln Asn Thr Thr Ser His Arg
               340                 345                 350
   Gln Glu Glu Ala Ser Glu Ala
           355
(2) INFORMATION POUR LA SEQ ID NO: 7:
   (i) CARACTERISTIQUES DE LA SEQUENCE:
      (A) LONGUEUR: 1080 paires de bases
      (B) TYPE: acide nucléique
      (C) NOMBRE DE BRINS: double
      (D) CONFIGURATION: linéaire
   (ii) TYPE DE MOLECULE: ADN génomique
   (ix) CARACTERISTIQUE ADDITIONELLE:
      (A) NOM/CLE: CDS
      (B) EMPLACEMENT: 1..1077
   (xi) DESCRIPTION DE LA SEQUENCE: SEQ ID NO: 7:
   ATG CCA AGA TCA GGT CGT TTT AGT ATC AAG GCT AAA AAT TAT TTC CTT        48
   Met Pro Arg Ser Gly Arg Phe Ser Ile Lys Ala Lys Asn Tyr Phe Leu
     1               5                  10                  15
   ACA TAT CCC AAA TGT GAT TTA ACA AAA GAA AAT GCA CTT TCC CAA ATA        96
   Thr Tyr Pro Lys Cys Asp Leu Thr Lys Glu Asn Ala Leu Ser Gln Ile
                20                  25                  30
   ACA AAC CTA CAA ACA CCC ACA AAC AAA TTA TTC ATC AAA ATT TGC AGA       144
   Thr Asn Leu Gln Thr Pro Thr Asn Lys Leu Phe Ile Lys Ile Cys Arg
            35                  40                  45
   GAA CTA CAT GAA AAT GGG GAA CCT CAT CTC CAT ATT CTC ATC CAA TTC       192
   Glu Leu His Glu Asn Gly Glu Pro His Leu His Ile Leu Ile Gln Phe
        50                  55                  60
   GAA GGA AAA TAC AAT TGT ACC AAT CAA CGA TTC TTC GAC CTG GTA TCC       240
   Glu Gly Lys Tyr Asn Cys Thr Asn Gln Arg Phe Phe Asp Leu Val Ser
    65                  70                  75                  80
   CCA ACC AGG TCA GCA CAT TTC CAT CCG AAC ATT CAG GGA GCT AAA TCG       288
   Pro Thr Arg Ser Ala His Phe His Pro Asn Ile Gln Gly Ala Lys Ser
                    85                  90                  95
   AGC TCC GAC GTC AAG TCC TAT ATC GAC AAG GAC GGA GAT GTT CTT GAA       336
   Ser Ser Asp Val Lys Ser Tyr Ile Asp Lys Asp Gly Asp Val Leu Glu
               100                 105                 110
   TGG GGT ACT TTC CAG ATC GAC GGA CGA TCT GCT AGG GGA GGA CAA CAG       384
   Trp Gly Thr Phe Gln Ile Asp Gly Arg Ser Ala Arg Gly Gly Gln Gln
           115                 120                 125
   ACA GCC AAC GAC GCT TAC GCA AAG GCA ATT AAC GCA GGA AGT AAG TCG       432
   Thr Ala Asn Asp Ala Tyr Ala Lys Ala Ile Asn Ala Gly Ser Lys Ser
       130                 135                 140
   CAG GCT CTT GAT GTA ATT AAA GAA TTA GCG CCT AGA GAT TAC GTT CTA       480
   Gln Ala Leu Asp Val Ile Lys Glu Leu Ala Pro Arg Asp Tyr Val Leu
   145                 150                 155                 160
   CAT TTT CAT AAT ATA AAT AGT AAT TTA GAT AAG GTT TTC CAG GTG CCT       528
   His Phe His Asn Ile Asn Ser Asn Leu Asp Lys Val Phe Gln Val Pro
                   165                 170                 175
   CCG GCA CCT TAT GTT TCT CCT TTT TTA TCT TCT TCT TTC GAT CAA GTT       576
   Pro Ala Pro Tyr Val Ser Pro Phe Leu Ser Ser Ser Phe Asp Gln Val
               180                 185                 190
   CCT GAT GAA CTT GAA CAC TGG GTT TCC GAG AAC GTC ATG GAT GCC GCT       624
   Pro Asp Glu Leu Glu His Trp Val Ser Glu Asn Val Met Asp Ala Ala
           195                 200                 205
   GCG CGG CCT TGG AGA CCG GTG AGT ATA GTG ATT GAG GGT GAC AGC CGG       672
   Ala Arg Pro Trp Arg Pro Val Ser Ile Val Ile Glu Gly Asp Ser Arg
       210                 215                 220
   ACA GGA AGG ACC ACG TGG GCC CGT TCA TTA GGC CCA CAT AAT TAT TTG       720
   Thr Gly Arg Thr Thr Trp Ala Arg Ser Leu Gly Pro His Asn Tyr Leu
   225                 230                 235                 240
   TGC GGC CAT CTT GAC CTC AGT CAA AAA GTA TAC AGC AAT AAT GCT TGG       768
   Cys Gly His Leu Asp Leu Ser Gln Lys Val Tyr Ser Asn Asn Ala Trp
                   245                 250                 255
   TAT AAC GTC ATT GAT GAC GTC GAC CCG CAT TAT TTA AAA CAC TTT AAA      816
   Tyr Asn Val Ile Asp Asp Val Asp Pro His Tyr Leu Lys His Phe Lys
               260                 265                 270
   GAA TTT ATG GGG GCC CAA AGA GAT TGG CAA AGC AAC ACA AAG TAT GGC      864
   Glu Phe Met Gly Ala Gln Arg Asp Trp Gln Ser Asn Thr Lys Tyr Gly
           275                 280                 285
   AAG CCC ATT CAA ATT AAA GGA GGC ATT CCC ACT ATC TTC CTA TGC AAT      912
   Lys Pro Ile Gln Ile Lys Gly Gly Ile Pro Thr Ile Phe Leu Cys Asn
       290                 295                 300
   CCA GGC CCA CAA TCA TCA TTT AAA GAA TAT CTC GAC GAA GAA AAA AAT      960
   Pro Gly Pro Gln Ser Ser Phe Lys Glu Tyr Leu Asp Glu Glu Lys Asn
   305                 310                 315                 320
   CAA GCA TTA AAA AAC TGG GCT ACT AAG AAT GCA ATC TTC GTC ACC ATC     1008
   Gln Ala Leu Lys Asn Trp Ala Thr Lys Asn Ala Ile Phe Val Thr Ile
                   325                 330                 335
   CAC CAG CCA TTG TTC GCA GAT ACC AAT CAA AAT ACA ACA TCA CAT CGC     1056
   His Gln Pro Leu Phe Ala Asp Thr Asn Gln Asn Thr Thr Ser His Arg
               340                 345                 350
   CAA GAA GAG GCA AGT GAG GCG TAG                                     1080
   Gln Glu Glu Ala Ser Glu Ala
           355
(2) INFORMATION POUR LA SEQ ID NO: 8:
   (i) CARACTERISTIQUES DE LA SEQUENCE:
      (A) LONGUEUR: 359 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 8:
   Met Pro Arg Ser Gly Arg Phe Ser Ile Lys Ala Lys Asn Tyr Phe Leu
    1               5                  10                  15
   Thr Tyr Pro Lys Cys Asp Leu Thr Lys Glu Asn Ala Leu Ser Gln Ile
               20                  25                  30
   Thr Asn Leu Gln Thr Pro Thr Asn Lys Leu Phe Ile Lys Ile Cys Arg
           35                  40                  45
   Glu Leu His Glu Asn Gly Glu Pro His Leu His Ile Leu Ile Gln Phe
       50                  55                  60
   Glu Gly Lys Tyr Asn Cys Thr Asn Gln Arg Phe Phe Asp Leu Val Ser
   65                  70                  75                  80
   Pro Thr Arg Ser Ala His Phe His Pro Asn Ile Gln Gly Ala Lys Ser
                   85                  90                  95
   Ser Ser Asp Val Lys Ser Tyr Ile Asp Lys Asp Gly Asp Val Leu Glu
               100                 105                 110
   Trp Gly Thr Phe Gln Ile Asp Gly Arg Ser Ala Arg Gly Gly Gln Gln
           115                 120                 125
   Thr Ala Asn Asp Ala Tyr Ala Lys Ala Ile Asn Ala Gly Ser Lys Ser
       130                 135                 140
   Gln Ala Leu Asp Val Ile Lys Glu Leu Ala Pro Arg Asp Tyr Val Leu
   145                 150                 155                 160
   His Phe His Asn Ile Asn Ser Asn Leu Asp Lys Val Phe Gln Val Pro
                   165                 170                 175
   Pro Ala Pro Tyr Val Ser Pro Phe Leu Ser Ser Ser Phe Asp Gln Val
               180                 185                 190
   Pro Asp Glu Leu Glu His Trp Val Ser Glu Asn Val Met Asp Ala Ala
           195                 200                 205
   Ala Arg Pro Trp Arg Pro Val Ser Ile Val Ile Glu Gly Asp Ser Arg
       210                 215                 220
   Thr Gly Arg Thr Thr Trp Ala Arg Ser Leu Gly Pro His Asn Tyr Leu
   225                 230                 235                 240
   Cys Gly His Leu Asp Leu Ser Gln Lys Val Tyr Ser Asn Asn Ala Trp
                   245                 250                 255
   Tyr Asn Val Ile Asp Asp Val Asp Pro His Tyr Leu Lys His Phe Lys
               260                 265                 270
   Glu Phe Met Gly Ala Gln Arg Asp Trp Gln Ser Asn Thr Lys Tyr Gly
           275                 280                 285
   Lys Pro Ile Gln Ile Lys Gly Gly Ile Pro Thr Ile Phe Leu Cys Asn
       290                 295                 300
   Pro Gly Pro Gln Ser Ser Phe Lys Glu Tyr Leu Asp Glu Glu Lys Asn
   305                 310                 315                 320
   Gln Ala Leu Lys Asn Trp Ala Thr Lys Asn Ala Ile Phe Val Thr Ile
                   325                 330                 335
   His Gln Pro Leu Phe Ala Asp Thr Asn Gln Asn Thr Thr Ser His Arg
               340                 345                 350
   Gln Glu Glu Ala Ser Glu Ala
           355

## Description

La présente invention a pour objet des plantes transgéniques résistantes ou tolérantes aux virus à ADN pathogènes chez les plantes.

L'invention a plus particulièrement pour objet les semences issues de ces plantes transgéniques, et susceptibles de germer en plantes présentant ce critère de résistance ou de tolérance aux virus à ADN phytopathogènes.

L'invention vise également les procédés d'obtention de ces plantes et semences.

Les virus à ADN phytopathogènes sont essentiellement représentés par les virus à ADN simple brin (ss) et les virus à ADN double brin (ds).

Les virus à ADN double brin comprennent principalement les Badnavirus et les Caulimovirus.

Les virus à ADN simple brin comprennent principalement les géminivirus et d'autres virus à ADN ss exotiques.

Parmi ces virus à ADN phytopathogènes, les géminivirus ont été particulièrement étudiés. Ce sont des virus de plantes dont l'impact sur la production agronomique est considérable dans de nombreuses régions tropicales et subtropicales (Harrison, 1985). Ce sont des virus à ADN circulaire simple brin, caractérisés par une structure unique dans le monde viral: une capside en forme de double icosaèdre (Francki *et al.,* 1980). Suivant leur organisation génomique on distingue d'une part les géminivirus à génome bipartite constitué de deux molécules d'ADN, A et B d'environ 2.8 kilobases (kb) chacune (cas de l'African Cassava Mosaic Virus (ACMV) et du Tomato Golden Mosaic Virus (TGMV)), d'autre part les géminivirus à génome monopartite à une seule molécule d'ADN d'environ 2.8 kb (cas du Maize Streak Virus (MSV), du Wheat Dwarf Virus (WDV) et du Beet Curly Top Virus (BCTV)). Selon le vecteur de transmission du virus deux sous-groupes sont identifiés. Certains géminivirus sont transmis par l'aleurode (ou mouche blanche) *Bemisia tabaci,* et jusqu'à une date récente étaient tous considérés à génome bipartite (ACMV, TGMV). Les autres géminivirus sont transmis par des ciccadelles *(Cicadulina* sp.) et ont tous un génome monopartite (MSV, WDV et BCTV); pour une revue voir Lazarowitz, 1992a.

Le virus du jaunissement et de l'enroulement des feuilles de la tomate (ou virus TYLC pour Tomato Yellow Leaf Curl Virus, encore désigné TYLCV) constitue une exception à cette classification. En effet, bien qu'il soit transmis par *Bemisia tabaci,* son génome est monopartite (Kheyr-Pour *et al*., 1991; Navot *et al*., 1991). Seul un isolat de TYLCV thailandais a été décrit comme ayant un génome bipartite (Rochester *et al.,* 1990).

Le TYLCV est responsable d'importants dégâts dans les cultures de tomates, les pertes pouvant atteindre 50 à 60% (Allex *et al.,* 1994). Sur cette espèce, les symptômes caractéristiques comportent un rabougrissement de la plante, un enroulement et un jaunissement des feuilles, un aspect buissonnant dû au raccourcissement des entre-noeuds et un arrêt de la croissance florale. Cette maladie, qui affecte déjà de nombreuses régions (Bassin méditerranéen, Moyen et Proche Orient, Extrême sud asiatique et l'Afrique sahelienne), est actuellement en expansion (Czosnek *et al.,* 1990). La France métropolitaine est, pour le moment, épargnée mais les Antilles françaises sont touchées depuis deux ans (Hostachy et Allex, 1993).

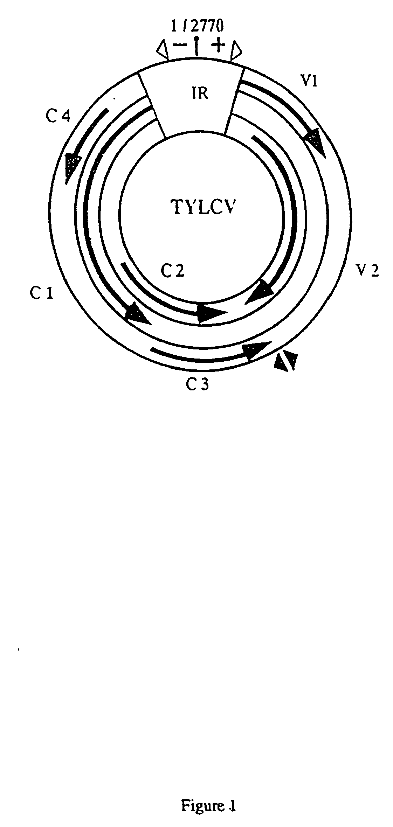

L'analyse de la séquence du génome viral du TYLCV révèle l'existence de six cadres ouverts de lectures (ou ORF pour "Open Reading Frame") pouvant coder pour des produits de taille supérieure à 10 kDa. Ces ORFs se trouvent tant sur le brin viral (ORFs V1 et V2) que sur le brin complémentaire (ORFs C1, C2, C3 et C4) (Kheyr-Pour *et al*., 1991); voir Fig.1.

La transcription du génome viral des géminivirus est bidirectionnelle (Hanley-Bowdoin *et al.,* 1988; Accotto *et al.,* 1989). Elle a lieu de part et d'autre de la région intergénique (RI), ou région commune aux deux composants d'ADN des virus à génome bipartite. La RI d'environ 200 nucléotides (nt) présente une séquence d'environ 30 nt qui pourrait adopter une structure en tige-boucle. Par analyse mutationnelle, cette structure potentielle en tige-boucle s'est révélée essentielle à la réplication de l'ADN viral.

La réplication du génome viral passe par une forme intermédiaire double brin, dans les noyaux des cellules infectées (Davies et Stanley, 1989). La présence, au niveau de la structure potentielle en tige-boucle, d'un nonanucléotide très conservé chez tous les géminivirus (TAATATTAC) et similaire au site de clivage du produit de gène A du bactériophage □X174, suggère un mode de réplication selon le modèle de cercle roulant (Stenger *et al.,* 1991; Saunders *et al.,* 1991).

Le gène C1 (ou AL1) est le seul gène viral essentiel à la réplication de l'ADN viral (Elmer *et al*., 1988). Tous les autres facteurs nécessaires à la réplication de l'ADN viral sont d'origine cellulaire. La protéine C1 ne présente aucune homologie de séquence avec les ADN polymérases connues. L'analyse de la séquence déduite en acides aminés de la protéine C1 révèle l'existence d'un motif consensus de fixation de nucléosides triphosphate (NTP) ou P-loop (pour "phosphate loop" ou boucle phosphate), **GXXXXGKT/S** (G=glycine, K=lysine, T=thréonine, S=Ser, X=tout acide aminé), qui est conservé parmi les ADN et ARN hélicases (Gorbalenya et Koonin, 1989). Les rôles de la protéine C1 dans la réplication ne sont pas encore connus. Cependant, plusieurs publications convergent dans le sens de fonctions multiples associées à la protéine C1. La protéine AL1 du TGMV se fixe à une séquence spécifique d'environ 50 nt au niveau de la région commune (Fontes *et al.,* 1992). Cette protéine possède également une activité d'autorégulation par répression de sa propre transcription (Sunter *et al.,* 1993). Une activité ATPase ainsi qu'une capacité de fixation spécifique à l'ATP *in vitro* ont été récemment montrées pour la protéine C1 du TYLCV exprimée sous forme de fusion avec la Glutathion-S-Transférase (GST) dans *Escherichia coli.* Cependant, le(s) rôle(s) possible(s) de cette activité ATPase *in vivo* n'est (ne sont) pas encore élucidé(s). Par ailleurs, une activité *in vitro* de coupure et de ligation de la protéine GST-C1 du TYLCV au niveau du nonanucléotide conservé a été montrée. Cette double activité de clivage et de ligation *in vitro* est indépendante de l'activité ATPase, puisque des mutants défectifs pour leur activité ATPase *in vitro* possèdent toujours la capacité de cliver et de lier *in vitro* l'ADN viral.

Tous les cultivars de tomate sont sensibles au TYLCV. Actuellement, la lutte contre le virus porte, sans grand succès, sur le vecteur *B. tabaci* par utilisation d'insecticides et par protection des champs et des serres avec des filets à maille très fine. Il est donc urgent de développer d'autres méthodes de lutte plus efficaces. C'est ainsi que des résistances naturelles chez des espèces sauvages de tomate *(Lycopersicon chilense, L. hirsutum, L. peruvianum)* sont recherchées en vue de les introduire dans l'espèce domestique *L. esculentum* (Zakay *et al*., 1990). Un gène de tolérance (absence de symptômes mais réplication de l'ADN viral) a été cartographié par RFLP sur le chromosome 6 de *L. chilense* et appelé Ty-1 (Eshed *et al.,* 1992). Ces programmes d'introgression sont de très longue haleine en raison des nombreux rétrocroisements à réaliser. De nouvelles stratégies utilisant le génie génétique des plantes ont donc été adoptées en parallèle à la génétique classique. Deux approches ont été utilisées pour obtenir des plantes transgéniques tolérantes ou résistantes aux géminivirus (Frischmuth et Stanley, 1993). La première consiste à exprimer des séquences antisens du gène AL1 du TGMV dans des plantes de tabac entraînant ainsi une réduction des symptômes dûs à ce virus (Day *et al.,* 1991). La deuxième approche repose sur l'inhibition du mouvement de l'ACMV *in planta* par l'expression d'une protéine de mouvement recombinante et défective du TGMV (von Arnim *et al.,* 1992).

Un des buts de la présente invention est de fournir un nouveau procédé d'obtention de plantes génétiquement transformées qui soient résistantes ou tolérantes aux virus à ADN pathogènes chez ces plantes.

Un autre but de la présente invention est de fournir des plantes transgéniques résistantes ou tolérantes aux virus à ADN.

Un autre but de la présente invention est de fournir des semences transgéniques susceptibles de donner lieu à la formation, par germination, de plantes transgéniques résistantes ou tolérantes aux virus à ADN. L'invention est illustrée à l'aide des figures suivantes:
- figure 1: organisation génomique du TYLCV de Sardaigne (TYLCVsar), encore désigné dans la suite de ce texte "STYLCV";
- figure 2: représentation des plasmides pTY Ala, His, Arg, du plasmide pBMC1-S et des plasmides pC1 Ala, His, Arg;
- figure 3: mutations construites dans le génome du STYLCV;
- figure 4: réplication des mutants dans le site de liaison aux NTP du STYLCV;
- figure 5: graphe représentant l'étude d'un effet *trans*-dominant de la mutation K en R, test d'activité NPT II;
- figure 6: graphe représentant l'effet de la transfection des génomes viraux mutants (pTYAla, pTYHis, pTYArg) sur la réplication du virus sauvage (pTYLCV);
- figure 7: graphe représentant l'effet de la cotransfection de constructions permettant l'expression à haut niveau des protéines C1 mutées (pC1Ala, pC1His, pC1Arg) sur la réplication du virus sauvage (pTYNéo);
- figure 8: représentation des plasmides pTYLCV et pGEXC1;
- figure 9: clonage de l'ORF C1 en aval du promoteur 35S dans le plasmide pGA492;
- figure 10: transformation de disques foliaires de *N. benthamiana* et obtention de plantes transgéniques exprimant l'ORF C1 sauvage ou muté;
- figure 11: schéma simplifié d'explication de l'obtention des plantes de la première génération F1;
- figure 12: réplication de l'ADN du STYLCV dans des protoplastes de tomate;
- figure 13: séquences nucléotidiques mutées C1* (dérivées par mutation de la séquence, à savoir l'ORF C1, codant pourde la protéine C1 ou Rep du STYLCV) codant pour les protéines mutées Rep^{K227A}, Rep^{K227H} et Rep^{K227R}, encore désignées dans la suite de ce texte par mutation Ala, His et Arg respectivement.

L'invention a pour objet l'utilisation de la séquence nucléotidique mutée codant pour une protéine C1 inactive correspondant à la protéine C1 active (encore désignée protéine Rep ou AL1) d'un poids moléculaire d'environ 40 kDa, essentielle pour la réplication du génome des géminivirus, dans laquelle la lysine située entre les positions 220 et 235 de ladite protéine C1 active est remplacée par l'alanine, l'arginine ou l'histidine, pour l'obtention de plantes résistantes ou tolérantes aux géminivirus.

Les séquences nucléotidiques mutées selon l'invention correspondent aux séquences nucléotidiques codant pour une protéine C1 des géminivirus suivants:
- le sous-groupe I (membre-type MSV pour Maize streak virus), comprenant les isolats suivants:
   .. MSV, ou virus des stries du maïs,
   .. CSMV (pour *Chloris* striate mosaic virus), ou virus de la mosaïque striée du *Chloris*,
   .. DSV (pour *Digitaria* streak virus), ou virus des stries du *Digitaria,*
   .. MiSV (pour *Miscanthus* streak virus), ou virus des stries du *Miscanthus*,
   .. WDV (pour Wheat dwarf virus), ou virus du nanisme du blé,
   .. PanSV-Ken (pour *Panicum* streak virus-Kenya),
   .. SSV-N (pour Sugarcane streak virus-Natal), ou virus Natal des stries de la canne à sucre,
- le sous-groupe II (membre-type BCTV pour Beet curly top virus), comprenant les isolats suivants:
   .. BCTV, ou virus de l'enroulement de l'apex de la betterave,
   .. TPCTV (pour Tomato pseudo-curly top virus), ou virus du pseudo-enroulement de l'apex de la betterave,
   .. BSDV (pour Bean summer death virus), ou virus de la mort estivale du haricot,
   .. TYDV (pour Tobacco yellow dwarf virus), ou virus du nanisme et jaunissement du tabac,
   .. TLRV (pour Tomato leafroll virus), ou virus de l'enroulement des feuilles de tomate,
- le sous-groupe III (membre-type BGMV pour Bean golden mosaic virus), comprenant les isolats suivants:
   .. BGMV, ou virus de la mosaïque dorée du haricot,
   .. AbMV (pour *Abutilon* mosaic virus), ou virus de la mosaïque de l'*Abutilon*,
   .. ACMV (pour African Cassava mosaic virus), ou virus de la mosaïque du manioc africain,
   .. CLCV (pour Cotton leaf crumple virus), ou virus du froissement des feuilles de coton,
   .. EuMV (pour *Euphorbia* mosaic virus) ou virus de la mosaïque de l'Euphorbe,
   .. HYVMV (pour Honeysuckle yellow vein mosaic virus), ou virus de la mosaïque et nervures jaunes du chèvrefeuille,
   .. ICMV (pour Indian Cassava mosaic virus), ou virus de la mosaïque du manioc indien,
   .. MLCV (pour Melon leaf curl virus), ou virus de l'enroulement des feuilles du melon,
   .. MYMV (pour Mungbean yellow mosaic virus), ou virus de la mosaïque jaune du haricot,
   .. PYMV (pour Potato yellow mosaic virus), ou virus de la mosaïque jaune de la pomme de terre,
   .. SLCV (pour Squash leaf curl virus), ou virus de l'enroulement des feuilles de la courge,
   .. TGMV (pour Tomato golden mosaic virus), ou virus de la mosaïque dorée de la tomate,
   .. TLCV (pour Tomato leaf curl virus), ou virus de l'enroulement de la feuille de tomate, notamment TLCV-Aus et TLCV-Ind pour Tomato leaf curl virus Australia et India respectivement,
   .. TYDV (pour Tomato Yellow Dwarf Virus), ou virus du nanisme et jaunissement de la tomate,
   .. TYLCV (pour Tomato yellow leaf curl virus), ou virus de l'enroulement et du jaunissement de la feuille de la tomate, notamment le STYLCV, pour TYLCV de Sardaigne, dont les sous-ensembles, notamment STYLCV-Sp (Espagne), comme le STYLCV-Sp-Murcia, le STYLCV-Si (Sicile) ou encore l'ITYLCV, pour TYLCV d'Israël, dont notamment les sous-ensembles ITYLCV-Eg (Egypte) et ITYLCV-Jo (Jordanie), ThTYLCV pour TYLCV de Thaïlande,
   .. AToLCV (pour Australian tomato leaf curl virus),
   .. IToLCV (pour Indian tomato leaf curl virus), dont notamment les sous-espèces IToLCV-ND (New Delhi) et IToLCV-Bg (Bengalore),
   .. TYMV (pour Tomato yellow mosaic virus), ou virus de la mosaïque jaune de la tomate,
   .. Tom GV (pour Tomato geminivirus MX3),
   .. ToLCrV (pour Tomato leaf crumple virus),
   .. ToMoV-Flo (pour Tomato mottle virus Florida),
   .. Virus du "Chino del Tomate,
   .. WCSV (pour Watermelon chlorotic stunt), ou virus du rabougrissement et de la chlorose de la pastèque,
   .. CLCV (pour Cotton leaf curl virus), ou virus de l'enroulement des feuilles du coton,
   .. CGMV (pour Cowpea golden mosaic virus), ou virus de la mosaïque dorée du *Vigna,*
   .. EYMV (pour Eggplant yellow mosaic virus), ou virus de la mosaïque jaune de l'aubergine,
   .. BCaMV (pour Bean calico mosaic virus MoV2),
   .. PhVCMV (pour Bean calico mosaic virus *P. vulgaris*),
   .. BDMV (pour Bean dwarf mosaic virus),
   .. PepGV (pour Pepper geminivirus MX1),
   .. PHV (pour Pepper huasteco virus),
   .. BYVMV (pour Bhendi (Okra) yellow vein mosaic virus),
   .. CYVMV (pour Croton yellow vein mosaic virus),
   .. HYMV (pour Horsegram yellow mosaic virus),
   .. DYMV (pour Dolichos yellow mosaic virus),
   .. PMTV-T (pour Pepper mild tigre virus-T),
   .. SGMV (pour Serrano golden mosaic virus),
   .. JMV (pour Jatropa mosaic virus),
   .. LIYV (pour Lettuce infectious yellow virus).

Pour une revue des virus à ADN, notamment simple brin, phytopathogènes comportant des séquences nucléotidiques dont sont susceptibles d'être dérivées par mutation les séquences nucléotidiques de l'invention, on peut se référer aux ouvrages suivants:
- *"Classification and Nomenclature of Viruses",* Fifth Report of the International Committee on Taxonomy of Viruses, Edited by Francki, Fauquet, Knudson and Brown; Archives of Virology, Supplementum 2, Springer-Verlag Wien, New York, 1991,
- *" Viruses of Tropical Plants",* Alan Brunt, Karen Crabtree and Adrian Gibbs, 1990, Edited by C.A.B. International, Wallingford, Oxon, OX10 8DE, UK,
- "Virus Taxonomy, the VIth Report of the ICTV"; Murphy FA, Fauquet CM, Bishop DHL, Ghabrial SA, Jarvis AW, Martelli GP, Mayo MA, Summers MD (eds) (1995), Springer-Verlag, Wien, New-York, pp. 570.

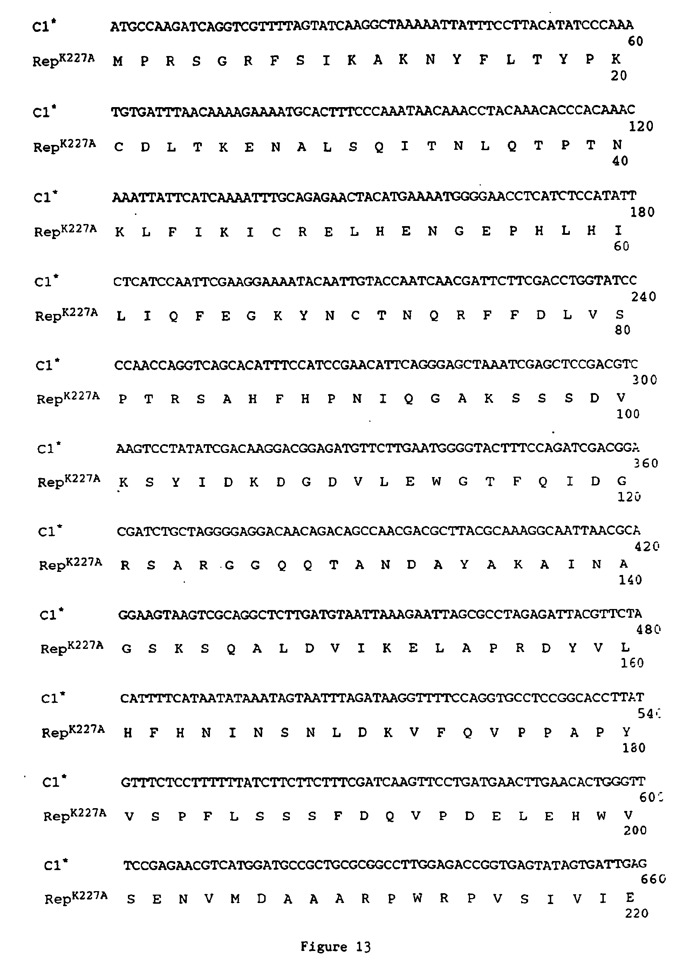

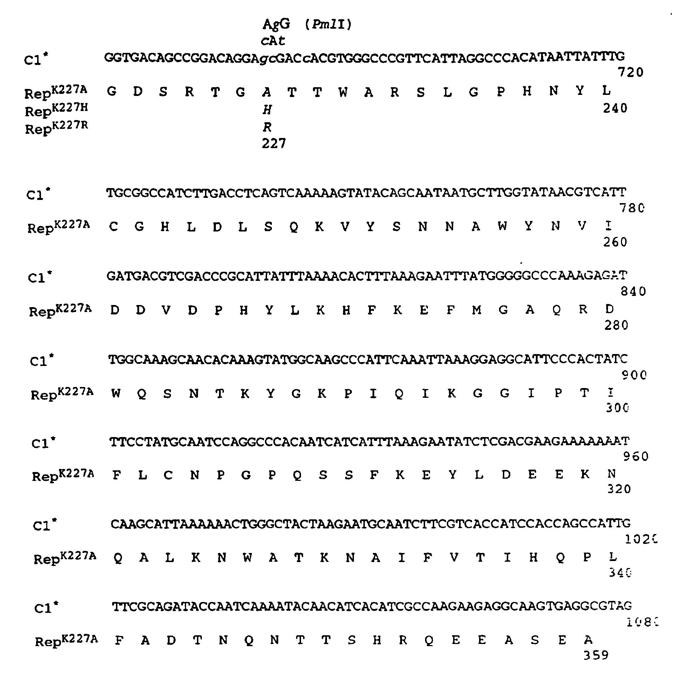

L'invention a plus particulièrement pour objet l'utilisation des séquences nucléotidiques mutées codant pour une protéine C1 inactive correspondant à la protéine C1 active des différents isolats du virus TYLC, telles que les séquences nucléotidiques C1* représentées sur la figure 13, dans lesquelles la lysine en position 227 est remplacée par l'alanine, l'arginine ou l'histidine, (à l'exception d'un isolat israëlien du virus TYLC (ITYLCV) pour lequel il semblerait que la lysine en question soit en position 225), pour l'obtention de plantes, notamment de tomates, résistantes ou tolérantes aux virus TYLC.

De préférence, la protéine C1 inactive susmentionnée des géminivirus, notamment des virus TYLC, correspond à la protéine C1 active, dans laquelle la lysine, située à l'une des positions indiquées ci-dessus, est remplacée par une alanine.

L'invention a également pour objet l'utilisation d'au moins une séquence nucléotidique mutée telle que définie ci-dessus, pour la transformation de cellules de plantes en vue de l'obtention de plantes transgéniques résistantes ou tolérantes aux géminivirus.

Par plantes transgéniques résistantes aux géminivirus, on entend toute plante chez laquelle, après infection par lesdits virus, on ne détecte pas d'une part, les symptômes pathologiques caractéristiques de l'infection d'une plante non transgénique sensible auxdits virus, et d'autre part l'ADN desdits virus au sein des cellules desdites plantes transgéniques.

Par plantes transgéniques tolérantes aux géminivirus, on entend toute plante chez laquelle, après infection par lesdits virus, on ne détecte pas les symptômes pathologiques caractéristiques de l'infection d'une plante non transgénique sensible auxdits virus, mais chez lesquelles de l'ADN desdits virus peut être détecté dans les cellules desdites plantes transgéniques (dans des quantités plus faibles que dans le cas de l'infection d'une plante sensible à ces virus).

Les séquences nucléotidiques mutées selon l'invention sont avantageusement utilisées pour la transformation de protoplastes, ou encore pour la construction de vecteurs, tels que des plasmides, ou pour la transformation de bactéries telles que *Agrobacterium tumefaciens,* susceptibles de transformer à leur tour des cellules de plantes.

L'invention a également pour objet l'utilisation susmentionnée de toute séquence nucléotidique mutée telle que décrite ci-dessus, dérivée par mutation d'une séquence nucléotidique présente dans le génome d'un géminivirus, pour l'obtention de plantes transgéniques résistantes ou tolérantes à une ou plusieurs sous-espèces et/ou espèces virales appartenant aux géminivirus, et plus particulièrement à une ou plusieurs sous-espèces et/ou espèces virales appartenant à un même sous-groupe au sein des géminivirus tel que le sous-groupe III, ou appartenant à un même membre d'un sous-groupe déterminé au sein des géminivirus, tel que les TYLCV.

A ce titre, l'invention a plus particulièrement pour objet l'utilisation susmentionnée de la séquence nucléotidique mutée codant pour une protéine C1 inactive correspondant à la protéine C1 active de l'espèce STYLCV, dans laquelle, la lysine en position 227 est remplacée par l'alanine, l'arginine ou l'histidine (telle que représentée sur la figure 13), pour l'obtention de plantes, notamment de tomates, résistantes ou tolérantes aux géminivirus tels que ceux appartenant aux TYLCV, notamment aux virus comme le STYLCV et l'ITYLCV, notamment l'ITYLCV-Jo, ou encore des géminivirus non TYLCV, notamment l'ACMV ou le BGMV.

L'invention a également pour objet tout procédé d'obtention de plantes, notamment de tomates, transgéniques résistantes ou tolérantes aux géminivirus, caractérisé en ce qu'il comprend une étape de transformation de cellules de plantes, à l'aide d'au moins une séquence nucléotidique mutée selon l'invention, le cas échéant, insérée dans un vecteur ou une bactérie tels que définis ci-dessus, suivie de la régénération des plantes à partir des cellules ainsi transformées.

A titre d'illustration, un procédé tel que décrit ci-desssus consiste à introduire une séquence nucléotidique mutée selon l'invention dans une cellule de plante, notamment dans un protoplaste, le cas échéant par l'intermédiaire d'un vecteur (tel qu'un plasmide), la plante transgénique étant obtenue par division des cellules ainsi transformées (procédé par utilisation d'ADN nu).

Un autre procédé consiste à transformer des cellules de plantes à l'aide de bactéries telles qu'*Agrobacterium tumefaciens,* elles-mêmes transformées par au moins une séquence nucléotidique mutée selon l'invention, notamment suivant la méthode de coculture ou des disques foliaires.

Pour une revue des méthodes utilisables pour l'obtention de plantes transgéniques de l'invention, on peut se reporter à l'article de Potrykus paru dans Biotechnology, juin 1990, 535-542.

Le procédé selon l'invention, est avantageusement réalisé par transformation des cellules d'un fragment d'une plante, notamment de disques foliaires, à l'aide de bactéries *Agrobacterium tumefaciens,* transformées par un vecteur tel que défini ci-dessus, suivie de la régénération des plantes à partir des cellules ainsi transformées, notamment à partir de bourgeons formés à la périphérie des disques foliaires susmentionnés.

L'invention concerne également les cellules transgéniques de plantes possédant un ADN hétérologue intégré de façon stable dans leur génome, ledit ADN hétérologue contenant le cas échéant un promoteur reconnu par les polymérases desdites cellules de plantes, et au moins une séquence nucléotidique mutée telle que définie ci-dessus, ladite séquence nucléotidique mutée étant capable d'inhiber totalement ou partiellement la réplication et/ou la diffusion et/ou la distribution de géminivirus, notamment capable de coder, sous le contrôle dudit promoteur, pour une protéine susceptible d'inhiber totalement ou partiellement la réplication et/ou la diffusion et/ou la distribution de géminivirus au sein desdites cellules transformées,
les cellules de *Nicotina tabaccum* cultivar BY2 (pour bright yellow 2, décrites par Nagata et al, 1992) étant exclues.

L'invention vise plus particulièrement les cellules de plantes telles que définies ci-dessus, qui peuvent être régénérées en une plante transgénique résistante ou tolérante à une ou plusieurs souches de géminivirus, et capable de produire des semences, elles-mêmes résistantes ou tolérantes auxdits virus.

Avantageusement, les cellules de plantes selon l'invention, sont transformées selon un procédé tel que décrit ci-dessus.

L'invention a également pour objet les semences transgéniques possédant un ADN hétérologue intégré de façon stable dans le génome de leurs cellules, ledit ADN hétérologue contenant le cas échéant un promoteur reconnu par les polymérases desdites cellules, et au moins une séquence nucléotidique mutée telle que définie ci-dessus, ladite séquence nucléotidique mutée étant capable d'inhiber totalement ou partiellement la réplication et/ou la diffusion et/ou la distribution de géminivirus, notamment capable de coder, sous le contrôle dudit promoteur, pour une protéine susceptible d'inhiber totalement ou partiellement la réplication et/ou la diffusion et/ou la distribution de géminivirus au sein desdites cellules transformées.

Avantageusement, les semences selon l'invention, sont capables de germer en une plante résistante ou tolérante aux géminivirus, et plus particulièrement à ceux définis ci-dessus.

De préférence, les semences selon l'invention, sont transformées par un procédé tel que décrit ci-dessus.

L'invention a également pour objet les plantes transgéniques résistantes ou tolérantes aux géminivirus comportant un ADN hétérologue intégré de façon stable dans le génome de leurs cellules, ledit ADN hétérologue contenant le cas échéant un promoteur reconnu par les polymérases desdites cellules de plantes, et au moins une séquence nucléotidique mutée telle que définie ci-dessus, ladite séquence nucléotidique mutée étant capable d'inhiber totalement ou partiellement la réplication et/ou la diffusion et/ou la distribution de géminivirus, notamment capable de coder, sous le contrôle dudit promoteur, pour une protéine susceptible d'inhiber totalement ou partiellement la réplication et/ou la diffusion et/ou la distribution de géminivirus au sein desdites cellules transformées,
les plantes de *Nicotina benthamiana* ainsi transformées étant exclues.

Avantageusement, les plantes selon l'invention, sont capables de produire des semences résistantes aux géminivirus.

L'invention vise plus particulièrement les plantes susmentionnées, telles qu'obtenues par mise en oeuvre d'un procédé tel que décrit ci-dessus.

Les plantes transgéniques selon l'invention résistantes ou tolérantes aux géminivirus, telles qu'obtenues par transformation de leurs cellules avec au moins une séquence nucléotidique mutée selon l'invention, sont avantageusement choisies parmi les plantes suivantes: *Ageratum* spp., notamment *Ageratum conyzoides, Arabidopsis* spp., notamment *Arabidopsis thaliana, Arachis* spp., notamment *Arachis hypogaea, Asparagus* spp., notamment *Asparagus officinalis, Avena* spp., notamment *Avena sativa* et *Avena byzantina, Beta* spp., notamment *Beta vulgaris, Brassica* spp., notamment *Brassica oleracea* vars *capitata, Brassica napus* et *Brassica rapa, Cajanus* spp., notamment *Cajanus cajan, Cannabis* spp., notamment *Cannabis sativa, Capsicum* spp., notamment *Capsicum annuum, Castanospermum* spp., notamment *Castanospermum australe, Citrullus* spp., notamment *Citrullus colocynthis* et *Citrullus lanatus, Cocos* spp., notamment *Cocos nucifera, Cola* spp., notamment *Cola chlamydantha* et *Cola gigantea* var. *glabrescens, Croton* spp., notamment *Croton lobatus, Cucumis* spp., notamment *Cucumis melo* var. *cantalupensis* et *Cucumis melo, Curcubita* spp., notamment *Cucurbita maxima, Cucurbita moschata,* et *Cucurbita pepo, Cynanchum* spp., notamment *Cynanchum acutum, Datura* spp., notamment *Datura stramonium, Dioscorea* spp., notamment *Dioscorea alata, Euphorbia* spp., notamment *Euphorbia fulgens, Euphorbia heterophylla, Euphorbia peplus* et *Euphorbia pulcherrima, Glycine* spp., notamment *Glycine max* et *Glycine hispida, Gossypium* spp., notamment *Gossypium barbadense, Gossypium hirsutum, Gossypium thurberi* et *Gossypium herbaceum, Helianthus* spp., notamment *Helianthus annuus, Hibiscus* spp., notamment *Hibiscus cannabinus, Hibiscus esculentus* et *Hibiscus sabdariffa, Hordeum* spp., notamment *Hordeum vulgare* et *Hordeum distichon, Indigofera* spp., notamment *Indigofera hirsuta, Ipomoea* spp., notamment *Ipomoea batatas, Jatropha* spp., notamment *Jatropha gossypifolia, Jatropha multifida* et *Jatropha podagrica, Lablab* spp., notamment *Lablab purpureus, Linum* spp., notamment *Linum usitatissimum, Lonicera* spp., notamment *Lonicera japonica, Lycopersicon* spp., notamment *Lycopersicon esculentum, Lycopersicon hirsutum* et *Lycopersicon pimpinellifolium, Macroptilium* spp., notamment *Macroptilium lathyroides, Macrotyloma* spp., notamment *Macrotyloma uniflorum, Malva* spp., notamment *Malva parviflora, Malvastrum* spp., notamment *Malvastrum coromandelianum, Manihot* spp., notamment *Manihot esculenta* et *Manihot glaziovii, Medicago* spp., notamment *Medicago sativa, Musa* spp., notamment *Musa sapientum, Nicotiana* spp., notamment *Nicotiana tabacum, Oryza* spp., notamment *Oryza sativa, Phaseolus* spp., notamment *Phaseolus acutifolius, Phaseolus lunatus* et *Phaseolus vulgaris*, *Pisum* spp., notamment *Pisum sativum, Raphanus* spp., notamment *Raphanus sativus, Saccharum* spp., notamment *Saccharum officinarum, Secale* spp., notamment *Secale cereale, Sida* spp., notamment *Sida rhombifolia, Solanum* spp., notamment *Solanum melongena* et *Solanum tuberosum, Sorghum* spp., notamment *Sorghum bicolor* et *Sorghum vulgare, Spinacia* spp., notamment *Spinacia oleracea, Theobroma* spp., notamment *Theobroma cacao,* les Triticales (croisements blé x seigle), *Triticum* spp., notamment *Triticum aestivum, Triticum durum* et *Triticum vulgare, Vicia* spp., notamment *Vicia faba* et *Vicia sativa, Vigna* spp., notamment *Vigna angularis, Vigna mungo, Vigna radiata* et *Vigna unguiculata, Vitis* spp., notamment *Vitis vinifera, Zea* spp., notamment *Zea mays.*

L'invention sera davantage illustrée à l'aide de la description détaillée qui suit de l'obtention de *N. benthamiana* et de tomates transgéniques résistantes à l'infection par les TYLCV, et plus particulièrement par les STYLCV et les ITYLCV.

### I) OBTENTION DE MUTANTS NEGATIFS DOMINANTS DANS LE SITE DE LIAISON AUX NTP DE LA PROTEINE C1 DU TYLCV, UN GEMINIVIRUS

### A) MATÉRIEL ET MÉTHODES

### a) Mutagénèse dirigée

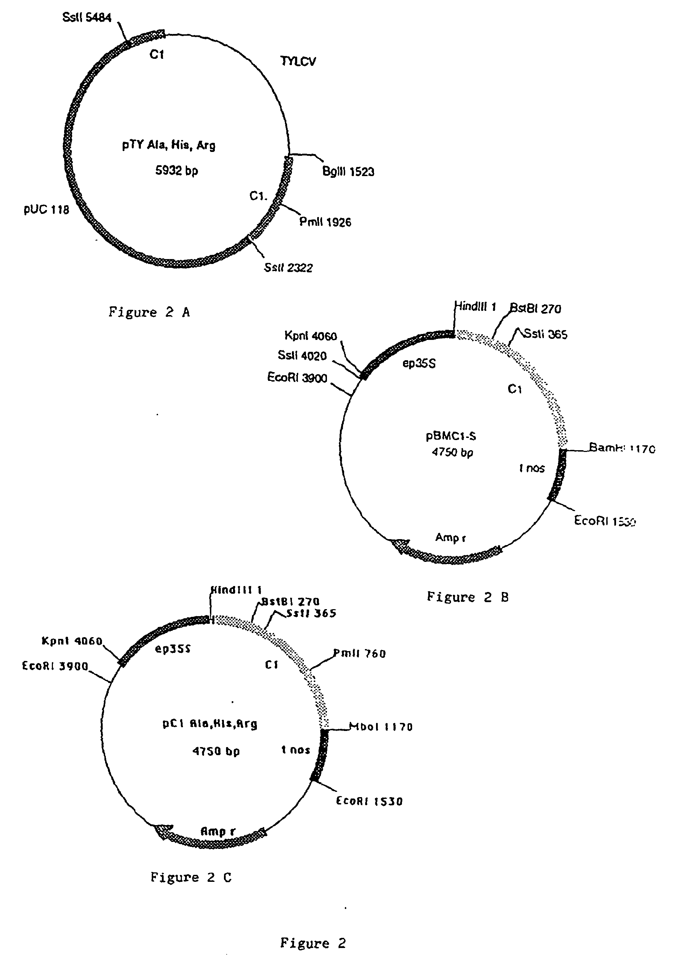

pTYLCV est un clone infectieux de l'isolat de Sardaigne du TYLCV (STYLCV) dans lequel le génome viral entier est cloné au site SstI du vecteur pUC118. La mutagénèse dirigée à été réalisée sur ce clone, produisant les plasmides pTYAla, pTYHis, pTYArg; portant respectivement la mutation correspondant au changement de K²²⁷ en A, H et R (Fig.2).

Principe (Kunkel, 1985) : Un oligonucléotide mutagène est hybridé à l'ADN simple-brin circulaire uridinylé soumis à la mutagenèse. La synthèse du deuxième brin s'effectue *in vitro,* puis la contre-sélection du brin uridinylé se fait par transformation dans *E. coli* et réparation de l'hybride double-brin *in vivo.* L'étape *in vitro* est réalisée en présence de fragments de restriction recouvrant le plasmide, de façon à minimiser la longueur du brin complémentaire néosynthétisé, ce qui diminue les risques d'introduction de nouvelles mutations.

### 1/-Obtention de l'ADN simple-brin uridynilé :

On transforme la souche *E*. *coli* BW313 (Hfr lysA dut-ung-thi-1 recA spoT1), infectée par le "phage helper" M13K07, par le plasmide à mutagéniser (pTYLCV). Ces bactéries produisent alors de l'ADN simple-brin uridynilé, la bactérie étant déficiente pour les fonctions dUTPase et Uracile-N-Glycosilase, et encapsidé sous forme simple-brin grâce aux fonctions codées par le phage M13K07 et à leur action en *trans* sur l'origine de réplication de M13 contenue dans pUC118. Les "phagemides" obtenus sont collectés par une première centrifugation de la culture bactérienne (10.000 rpm, 10 min), traitement du surnageant au PEG 8000 20% et NaCl 2,5M (300ml/ml), suivis d'une deuxième centrifugation (6000 rpm, 20 min). Les protéines sont extraites par traitement au phénol/chloroforme et l'ADN est précipité à l'éthanol (Sambrook et al, 1989).

### 2/-Hybridation avec l'oligonucléotide mutagène et synthèse du deuxième brin :

La séquence des oligonucléotides utilisés est la suivante :
mutation K en A : CCGGACAGGA**GC**GAC(**C**ACGTG)GGCC.
mutation K en H : CCGGACAGGA**C**A**T**AC(**C**ACGTG)GGCC.
mutation K en R : CCGGACAGGAA**G**GAC(**C**ACGTG)GGCC.

Les parenthèses indiquent l'emplacement du nouveau site de restriction P*ml*I.

Des fragments de restriction du pTYLCV ainsi que l'oligonucléotide phosphorylé (en excès molaire de 25 par rapport au fragment de restriction correspondant) sont hybridés sur l'ADN simple-brin uridynilé. Le deuxième brin est alors synthétisé (Klenow 10 unités, Ligase 800 unités, ATP 500 µM, dNTPs 500 µM, Tris 10mM, MgC12 5mM, DTT 500 µM). On transforme ensuite une souche *E. coli* DH5□ (supE44 □lacU169 (□80lacZ□M15) hsdR17 recA1 endA1 gyrA96 thi-1 relA1) qui va réparer l'ADN en dégradant préférentiellement le brin uridynilé. Le brin nouvellement synthétisé utilise comme matrice le brin complémentaire contenant la mutation, ce qui l'introduit ainsi sur les deux brins.

### b) Cassettes d'expression des ORF C1 mutées

L'expression à haut niveau des protéines C1 mutées est réalisée par sous-clonage des fragments portant la mutation dans le plasmide pBMC1-S, en plaçant les ORF C1 sous dépendance d'un promoteur fort, dérivé du promoteur 35S du virus de la mosaique du chou-fleur (CaMV). Le plasmide pBMC1-S contient l'ORF de C1 clonée dans un vecteur non réplicatif représenté sur la Fig.2.

Le site SstI₂₈₆₀ a été détruit de ce vecteur par l'ADN polymérase du phage T7. Le fragment H*in*dIII-S*ca*I, dans lequel le site SstI est supprimé, est substitué au fragment H*in*dIII-S*ca*I correspondant dans pBMC1-S: ainsi est obtenu le plasmide pBMC1-S(SstI). Ensuite, le fragment SstI₃₆₅-B*am*HI₁₁₇₀ de ce dernier plasmide a été remplacé par un fragment SstI-B*gl*II de pTYAla, pTYHis, ou pTYArg donnant respectivement les plasmides pC1Ala, pC1His et pC1Arg. On crée alors un nouveau site en position 1170, clivable par M*bo*I ou B*st*YI (Fig. 2).

### c) Détermination de la séquence des mutants obtenus

La méthode de terminaison de chaîne (Sanger et al, 1977; Sanger, 1981) (Kit Pharmacia -T7 Sequencing kit) a été utilisée avec pour matrice de l'ADN double-brin obtenu par maxipréparation d'ADN plasmidique (Sambrook et al, 1989). Les réactions sont déposées sur un gel d'acrylamide 6% dénaturant pour lequel on réalise un gradient d'épaisseur (0.2 mm en haut et 0.6 mm en bas des plaques) de façon à avoir une bonne résolution des bandes sur une plus grande partie du gel. La migration s'effectue en tampon TBE à 2000 V, avec un dispositif de thermostat permettant de maintenir les plaques à 50°C, ce qui donne une migration plus régulière.

### d) Lignée cellulaire et préparation de protoplastes

La lignée cellulaire BY2 est une suspension cellulaire dérivée de *Nicotiana tabacum* L cv Bright Yellow 2 (Kato et al, 1972; Nagata et al, 1992). Elle est diluée (2 : 100) tous les 7 jours dans un milieu dérivé du milieu de Murashige & Skoog (Sigma) supplémenté en KH₂PO₄ (200 mg/l), myo-Inositol (100 mg/l) thiamine-HCl (1mg/l), saccharose (30 g/l), 2,4-D (0,2mg/l) MES (2g/l), pH 5,8. La culture est maintenue en agitation constante (130 rpm) à 26°C et à l'obscurité.

Pour la préparation de protoplastes, les cellules d'une suspension de 100 ml de trois à quatre jours (ensemencement de 2 à 3 ml dans 100ml) sont récoltées par filtration, lavées dans du mannitol 0.4 M puis transférées dans 50 ml de la solution enzymatique suivante: 1% Cellulase Onozuka RS, 0.1% Pectolyase, mannitol 0.4 M, pH 4,5 à 5,5. La digestion de la paroi cellulaire se fait à 28°C, sous agitation (60 rpm) et à l'obscurité. L'obtention des protoplastes (1 à 2 h) est controlée sous microscope (apparition de cellules sphériques). Les protoplastes sont ensuite collectés par centrifugation (100g, 2 min), lavés, comptés en cellule de Fuchs-Rosenthal et repris en milieu MaMg (450 mM mannitol, 15 mM MgCl₂, 0.1 % MES, pH 5.6) à la densité finale de 2.10⁶ protoplastes dans 300 ml.

### e) Transfection des protoplastes

Chaque transfection est effectuée avec 2.10⁶ protoplastes et 15 mg d'ADN (plasmide circulaire ou ADN viral linéaire double-brin). Le mélange ADN/protoplastes est incubé 20 min avec 600 ml de PEG 1500 à 25% dans la solution suivante: 0,1 M MgCl₂, 6H₂O; 0,45 M Mannitol, 0,02M Hepes, pH 6. Les protoplastes sont ensuite rincés, repris dans 10 ml de milieu K3 (Nagy et al, 1976) et transferés dans deux boites de Petri de diamètre 5,5 cm. Les protoplastes transfectés sont incubés à 28°C en obscurité sans agitation et récoltés après 3 et 5 jours de culture.

### f) Extraction d'ADN total des cellules transfectées

Les cellules sont collectées par centrifugation (100 g, 5 min), lavées et remises en suspension dans 300 µl de tampon d'extraction (0.1M NaCl, 0.1M Tris-HCl pH8, 50 mM EDTA, 0.5% SDS) puis placées à -20°C.

Elles sont ensuite broyées dans ce tampon (broyeur Heidolph). Les protéines sont extraites par traitement au phénol-chloroforme (Sambrook et al, 1989) et les acides nucléiques sont précipités à l'éthanol (Sambrook et al, 1989). Les ADNs sont repris dans du tampon TE et leur concentration est déterminée par densité optique mesurée à 260 nm.

### g) Electrophorèse, transfert sur membrane et hybridation des ADNs extraits

L'éléctrophorèse en gel d'agarose 1% est réalisée sans bromure d'éthidium, en déposant 10 µg d'ADN par puits. Les conditions de migration sont les suivantes: 30 min à 60 volts puis environ 16 heures à 25 volts, en tampon TBE. Le gel est dépuriné en présence d'HCl 0,25 N et traité pour un transfert alcalin sur membrane Hybond-N (Amersham). La sonde utilisée correspond au génome total du STYLCV. Le marquage au ³²P est obtenu par amorçage aléatoire (Kit Amersham Megaprime).

### h) Extraction des proteines et test d'activité néomycine phosphotransférase

La méthode utilisée dérive de la méthode décrite par Reiss et al, 1984.

L'activité NPT II est décelée *in situ* sur un gel de protéines non dénaturant par la phosphorylation de la kanamycine au [□ -³²P] ATP.

Les protoplastes (une demi boite de Petri) sont centrifugés et repris dans un tampon contenant 60% glycérol, 8% ß-mercaptoéthanol, 100mM Tris pH6,8, 0,1% SDS, 1% bleu de bromophénol, 1% bleu de xylène-cyanol. Les protéines totales sont quantifiées par la méthode de Bradford (BIO-RAD); des quantités égales de protéines sont alors déposées sur gel d'acrylamide sans SDS (gel de tassement : acrylamide 3,5%; bisacrylamide 0,12 %; Tris-HCl 0.1 M pH6.8 / gel de séparation : acrylamide 10%; bisacrylamide 0,33%; Tris-HCl 0.4 M pH8.8). La migration est réalisée à 13,5 mA dans un tampon contenant 25 mM Tris-OH, pH 8,8; 191 mM glycine pendant 16 heures environ. Le gel est alors rincé à l'eau et équilibré dans le tampon de réaction (67 mM Tris-maléate; 42 mM MgCl₂; 400 mM NH₄Cl; pH 7.1). On coule ensuite sur le gel de protéines un gel d'agarose 1% contenant le sulfate de kanamycine (10,5 µg/ml) et 50 à 100 µCi [□-³²P] ATP. Après 30 min de réaction, la kanamycine phosphorylée est transférée par capillarité sur papier échangeur d'ions Whatman P81. Le papier P81 est ensuite traité (1mg/ml protéinase K, 1% SDS) pour diminuer les bandes parasites de phosphorylation de protéines cellulaires, rincé en tampon phosphate (10 mM K₂PO₄ pH 7.4) à 80°C séché et placé en autoradiographie.

### B) RÉSULTATS

### a) Obtention et séquençage des mutants dans le motif de liaison aux NTP de la protéine C1.

La protéine C1 du TYLCV contient le motif GX₄GKT de liaison aux NTP (résidus 221 à 228).

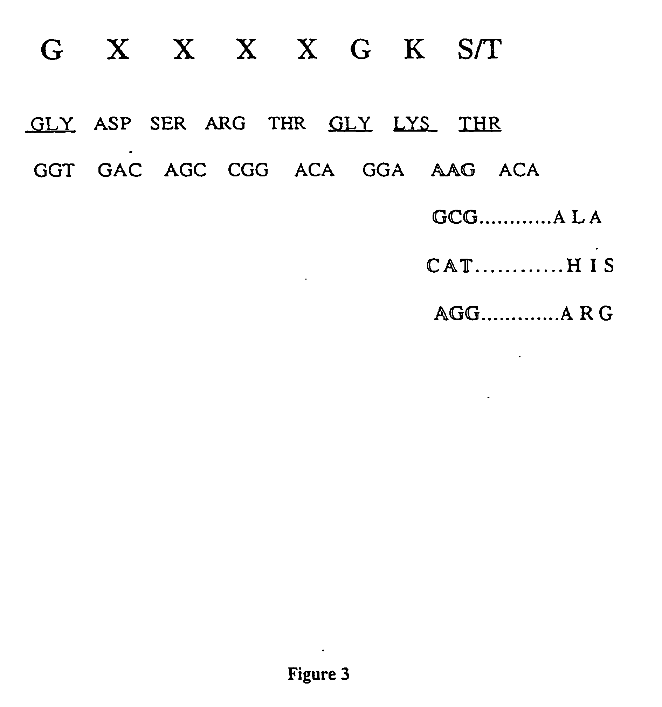

Trois mutations ont été introduites dans le clone infectieux pTYLCV pour remplacer la Lys²²⁷ (K) par trois acides aminés différents: Ala (A), His (H) et Arg (R) (Fig. 3). Les oligonucléotides mutagènes ont été conçus de manière à introduire au voisinage de la mutation recherchée un site de restriction unique pour l'enzyme P*ml*I (mutation silencieuse), afin de faciliter la sélection des clones présentant la mutation. Afin de confirmer la présence de la mutation désirée et de s'assurer qu'aucune autre mutation ne s'est produite dans cette région, plusieurs clones indépendants ont été séquencés pour chacune des mutations (Ala, His et Arg).

### b) Deux des mutants obtenus sont déficients pour leur réplication.

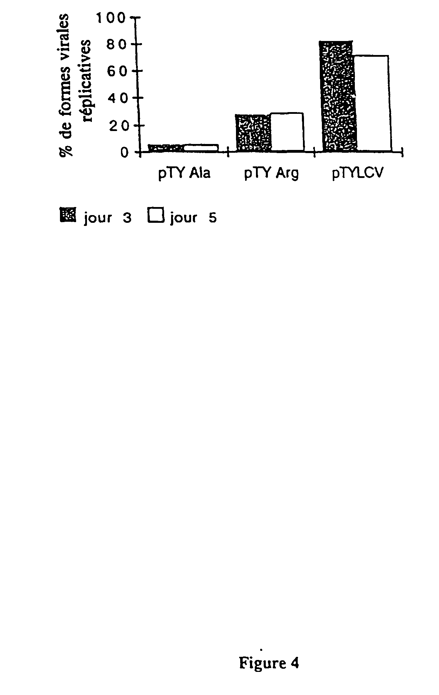

La réplication des mutants a été étudiée par transfection de protoplastes obtenus à partir de la suspension cellulaire de tabac BY2. Les differents génomes viraux sauvage (pTYLCV) ou mutés (pTYAla, pTYHis, pTYArg) ont été excisés de leur plasmide vecteur par l'enzyme Sst I, et sont donc transfectés sous forme double-brin linéaire. Après 3 et 5 jours de culture, on recherche l'apparition des formes réplicatives du virus par extraction des acides nucléiques totaux, éléctrophorèse, transfert sur membrane et hybridation avec une sonde correspondant à l'ADN viral. Les résultats sont présentés en Fig. 4 pour le clone viral infectieux et les mutants pTYAla et pTYArg. L'autoradiogramme résultant de l'analyse selon Southern a été quantifié à l'analyseur d'images (Bio-Image, Millipore). Les valeurs rapportées sur le graphe correspondent au rapport entre la densité optique (DO) des bandes correspondant aux formes réplicatives et la somme des DOs des bandes correspondant aux formes virales transfectées et aux formes réplicatives. On étudie donc le pourcentage de formes réplicatives par rapport aux formes virales totales.

La figure 4 représente le pourcentage de formes réplicatives après trois (en noir) et cinq (en blanc) jours de culture des cellules transfectées. pTYLCV: génome viral sauvage; pTYAla, pTYArg : génomes viraux mutés correspondant au changement de K en A et R respectivement.

On constate que le mutant pTYAla présente un taux de formes réplicatives bien inférieur à celui du virus sauvage (environ d'un facteur 12). Quant au mutant pTYArg, il présente une réduction d'un facteur 3 par rapport au sauvage. Un effet similaire a été observé lors d'une autre expérience. Les expériences n'ont pas été réalisées avec le mutant pTYHis.

La réplication des mutants pTYAla et pTYArg est altérée, ce qui indique que le site potentiel de liaison aux NTP joue un rôle important dans le fonctionnement de la protéine C1.

### c) Recherche d'un effet trans-dominant des protéines C1 mutées sur la réplication par le biais de l'expression du gène rapporteur NPT II :

L'effet *trans* -dominant sur la réplication de l'ADN viral peut être observé de deux façons, soit par accumulation d'ADN viral néosynthétisé, soit par accumulation d'une "protéine-rapporteur" codée par un virus recombinant. Le gène rapporteur utilisé ici est le gène codant pour la néomycine phospho-transférase II (NPT II), introduit à l'emplacement des phases de lecture V1 et V2 du TYLCV. Un tel génome viral remanié (pTYnéo) a les mêmes capacités de réplication qu'un génome non modifié dans des cellules isolées. Le test d'activité NPT II est quantitatif: le produit phosphorylé de la réaction, et donc l'intensité de la tache en autoradiographie dans le cas d'utilisation de □-³²P ATP, est proportionnel à la quantité d'enzyme présente (Reiss et al, 1984). En supposant que l'intensité de l'activité NPT II augmente avec le nombre de génomes viraux répliqués, nous avons étudié l'activité NPT II en présence des différentes constructions.

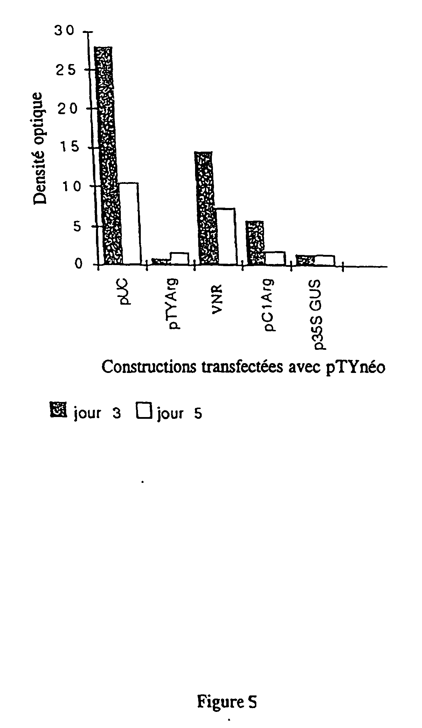

Afin de rechercher un effet trans-dominant, nous avons cotransfecté des protoplastes de tabac d'une part avec les génomes viraux sauvages et mutés (pTYLCV, pTYAla, pTYHis, pTYArg) et d'autre part avec les génomes viraux sauvages et les cassettes d'expression des ORF C1 mutées (pC1Ala, pC1His, pC1Arg). On constate une nette diminution de l'expression du gène NPT II en présence du virus mutant pTYArg (d'un facteur 37 à 3 jours). De même, l'expression de la protéine C1 mutée (pC1 Arg) à haut niveau inhibe la réplication d'un facteur 2,5 (Fig.5).

Nous pouvons voir cependant que, dans tous les cas, les valeurs de l'expression NPT II diminuent entre 3 et 5 jours. La construction portant le gène NPT II étant réplicative, on ne devrait pas observer une telle baisse de l'expression. Cette cinétique suggère que le promoteur de V1 et V2 est régulé en fonction du cycle viral. Cette méthode de détection de la réplication virale est donc trop indirecte et non appropriée, et ne sera pas poursuivie pour l'étude des mutants pTYHis et pTYAla.

### d) Les génomes viraux mutés dans le site de liaison aux NTP inhibent la réplication du génome sauvage.

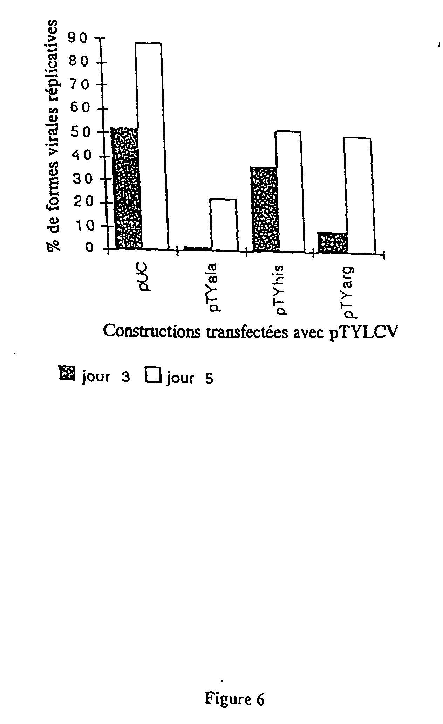

La réplication virale est étudiée cette fois par quantification des formes virales réplicatives accumulées dans les cellules cotransfectées avec les génomes viraux sauvages (pTYLCV) et mutés (pTYAla, pTYHis, pTYArg). La méthodologie utilisée est la même que pour l'étude de la réplication des mutants. La présence des virus mutants influe sur la réplication du virus sauvage (Fig. 6):
En effet, les mutants pTYAla et pTYArg diminuent la réplication virale d'un facteur 30 et 5,5 respectivement. Pour le mutant pTYHis, l'effet n'est pas très important (facteur 1,4). Ces résultats ne sont le fruit que d'une seule expérience, sauf pour le mutant pTYArg où l'experience a été répétée et a donné des résultats concordants.

Nous pouvons voir, avec les mutants pTYAla et pTYArg, qu'à 5 jours, la différence dans le taux de formes réplicatives entre le virus sauvage et les mutants est moins prononcée qu'à 3 jours. Ceci peut être expliqué par une accumulation progressive de molécules échappant à l'inhibition, qui ne serait donc pas complète dans ce système.

Les mutants pTYAla et pTYArg semblent donc inhiber la réplication du virus sauvage. Toutefois, il est difficile d'attribuer de façon rigoureuse cet effet inhibiteur aux protéines C1 mutées. En effet, un phénomène de dilution des protéines C1 fonctionnelles doit avoir lieu et interférer avec l'efficacité de réplication. La proteine C1 produite par le génome sauvage va en effet assurer la réplication de son propre génome mais aussi celle des génomes mutants, et donc se voir répartie sur deux fois plus de molécules matrices que dans le contrôle utilisé ici (pUC 118) où le génome viral sauvage est cotransfecté avec un plasmide auquel C1 ne se lie pas. Il sera intéressant de réaliser un contrôle supplémentaire, dans lequel un virus sauvage est cotransfecté avec un virus muté ne produisant aucune protéine C1 (par délétion de l'ORF C1 ou par introduction d'un codon STOP). Si la réplication virale est également diminuée, il s'agit alors d'un phénomène de titration des protéines C1 et non d'un effet négatif dominant.

### e) Les protéines C1 mutées surexprimées ont un effet négatif dominant: elles sont capables d'inhiber en trans la réplication du génome viral sauvage.

Afin de voir si les protéines C1 mutées dans le site de liaison aux NTP exprimées à haut niveau peuvent inhiber la réplication du génome viral sauvage, les ORFs correspondantes ont été placées sous contrôle d'un promoteur fort (pC1Ala, pC1His, pC1Arg). Les constructions ainsi obtenues ont été testées pour leur effet sur la réplication par cotransfection de protoplastes avec un génome viral sauvage.

Les plasmides pC1Ala et pC1His exercent une inhibition prononcée de la réplication du virus sauvage (diminution d'un facteur 38,5 et 14,5 respectivement par rapport au témoin utilisé). D'autres expériences ont montré une variation dans le même sens, pC1His ayant cette fois un effet plus fort que pC1Ala. Quant au plasmide pC1Arg, son effet n'est pas significatif puisque le facteur de diminution n'est que de 1,4. Ceci tend à faire valoir l'hypothèse de la dilution de molécules dans le cas de la cotransfection de génomes viraux entiers. En effet, avec cette même mutation, on pouvait voir un effet de diminution de la réplication virale que, curieusement, on ne retrouve pas dans le cas d'une surexpression.

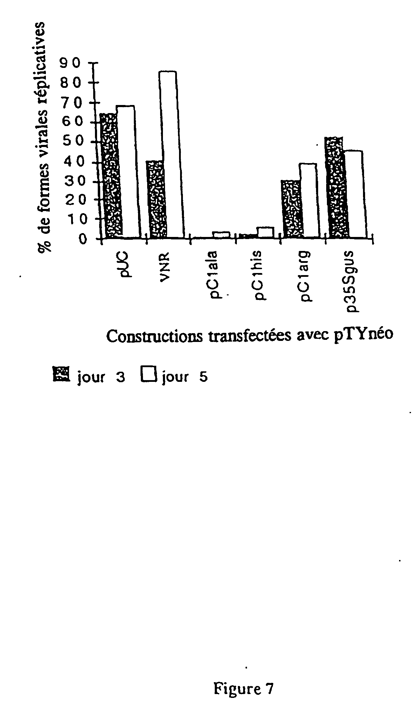

Une cotransfection contrôle a été réalisée avec pTYnéo et une cassette d'expression de la protéine GUS (□-glucuronidase), le promoteur alors utilisé étant le promoteur 35S du CaMV. Cette transfection a été réalisée dans le but d'évaluer les conséquences de la surexpression d'une protéine quelconque. Sur les deux expériences réalisées, les résultats ont été antagonistes puisque dans un cas l'effet semble nul alors que dans l'autre cela a provoqué une chute des formes réplicatives d'un facteur 4 (Fig. 5 et Fig. 7).

### Conclusion

L'altération du site potentiel de liaison aux NTP de la protéine C1 du TYLCV résulte en l'obtention de génomes viraux déficients pour leur réplication. Ceci démontre l'importance de ce site dans le fonctionnement de la protéine C1 et suggère fortement que la protéine C1 est sans doute capable de se lier à un NTP. De plus, les génomes viraux mutés (pTYAla et pTYArg) peuvent freiner la réplication de leur homologue sauvage. Un effet similaire a pu être mis en évidence avec un vecteur d'expression des protéines C1 mutées, pour les mutations Ala et His.

Il semble donc que le phénotype négatif des protéines C1 mutées soit effectivement dominant sur leur homologue sauvage, et conduise ainsi à une inhibition de la réplication virale. En effet, l'inhibition de la réplication observée dans le cas de la cotransfection de génomes viraux entiers ne peut être expliquée uniquement par un effet de dilution des protéines C1 sauvages puisque le mutant pTYHis n'induit pas de variation très importante. Toutefois, l'effet de la surexpression d'une protéine exogène (GUS) reste à définir.

Plusieurs mécanismes peuvent rendre compte d'un effet négatif dominant des protéines C1 mutées. Il est possible que la protéine C1 soit multimèrique et que les sous-unités mutées s'associent à leur homologue sauvage, rendant non fonctionnels des multimères hétérologues. Une autre possibilité est que les protéines C1 mutées entrent en compétition avec les protéines sauvages pour un substrat limitant. Ce substat pourrait être une protéine cellulaire, ou l'ADN viral lui-même. Le complexe hypothétique ainsi formé serait transitoire et productif quand il contient la protéine C1 sauvage, mais incapable d'évoluer plus loin s'il n'y a pas liaison aux NTP ou hydrolyse de ceux-ci.

L'obtention de phénotypes négatifs dominants dans le cas de la cotransfection de génomes viraux est un fait surprenant. En effet, il est difficile de concevoir un effet d'inhibition si radical avec des protéines C1 non mutées et mutées exprimées à un même niveau. Il pourrait y avoir surexpression spontanée des protéines C1 mutées, ce qui peut avoir lieu si la protéine C1 régule sa propre expression. En effet, il est concevable que la protéine C1 interagisse normalement avec un cofacteur cellullaire pour réaliser un rétro-contrôle négatif sur sa production; on a d'ailleurs pu voir que les transcrits de C1 sont présents en faible quantité par rapport aux transcrits codés par le brin viral. Les protéines C1 mutées ont peut-être perdu cette capacité d'interagir avec un cofacteur pour former un complexe de régulation négative. Il en résulterait alors une surexpression des protéines mutées, qui s'amplifient peu à peu et acquièrent alors la capacité d'inhiber les protéines sauvages, comme c'est le cas pour les protéines Rep de l'Adeno Associated Virus (AAV) (Chejanovsky et Carter, 1990).

### II) MISE EN EVIDENCE D'UNE ACTIVITE ATPase IN VITRO CHEZ LA PROTEINE C1 D'UN GEMINIVIRUS, LE STYLCV. ETUDE DE MUTATIONS DANS SON SITE DE LIAISON AUX NTP

### A) MATERIELS ET METHODES

Plusieurs méthodes classiques de biologie moléculaire ont été employées suivant les protocoles indiqués par Sambrook et al.(1989), Ausubel et al.(1989), ou par les fournisseurs; elles ne seront pas détaillées ici.

### 1. Etude biochimique de l'activité ATPase in vitro de la protéine C1 sauvage ou mutée

### 1.1 Obtention de la protéine C1 sauvage ou mutée

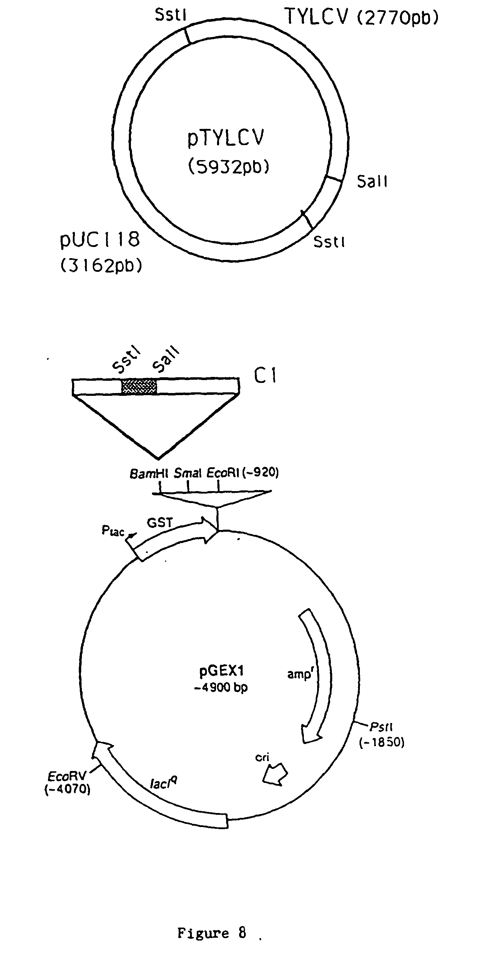

Une protéine de fusion GST-C1 "sauvage" a été obtenue. La construction exprimée dans *E.coli* est placée dans le plasmide pGEX3(Amrad), sous le contrôle d'un promoteur lac inductible par l'IPTG (voir figure 8).

Pour obtenir la même construction avec les différentes mutations étudiées, le fragment de 500 pb SalI-SstI du plasmide pGEX-C1 est remplacé par le fragment correspondant des plasmides pTY-Ala, Arg, His et Pml (figure 8). L'obtention de ces plasmides est décrite dans le paragraphe 2 ci-après.

Le vecteur pGEX-C1, ainsi que les différents inserts, sont purifiés après digestion par les enzymes SalI et SstI sur gel d'agarose 1% à bas point de fusion. La ligation s'effectue grâce à ligase T4 (Ligase: 400 unités; vecteur: 50ng; insert: 150ng) pendant la nuit à 16°C. La souche d'*E.coli* DH5□ est transformée avec les constructions obtenues, puis l'ADN bactérien est extrait par lyse alcaline. Les constructions sont digérées par l'enzyme PmlI, afin de mettre en évidence l'existence du site correspondant chez les mutants.

Après culture des bactéries en présence de l'inducteur (IPTG 0,2mM), les protéines sont extraites par traitement aux ultrasons ("sonication") (VibraCell 3x30", réglage 4,5). Les protéines contenant une partie GST fonctionnelle sont retenues sur des billes d'agarose-glutathion, lavées avec du tampon MTPBS# (#: voir annexe) pour éliminer les protéines bactériennes contaminantes, puis éluées dans une solution de glutathion 5mM, Tris pH8 15mM.

La présence de la protéine GST-C1 sauvage ou mutée est contrôlée avant et après purification par dépot d'un aliquot sur gel dénaturant Laemmli 10%^{#} et coloration au bleu de Coomassie^{#}.

### 1.2 Etude de l'activité ATPase in vitro de la protéine C1 sauvage ou mutée (d'après Traut, 1990)

Vingt ml de tampon ATPase(x2) (composition: voir ci-après) sont mélangés à 20 ml de fraction protéique purifiée (environ 50ng). Le mélange est mis à incuber à 25°C. La réaction est arrêtée après le temps voulu par ajout de 200ml de charbon actif (7,5% de charbon actif dans HCl 50mM, H₃PO₄ 5 mM).

Le charbon actif lie l'ATP, mais non le phosphate inorganique libéré par une éventuelle activité ATPase.

Le charbon est ensuite éliminé par centrifugation (5' à 13000 rpm en tube Eppendorf). La radioactivité du surnageant est mesurée au compteur à scintillation (Beckman LS 7500) après ajout de 4 ml de liquide de scintillation.
- Tampon ATPase x2:: Pipes/NaOH pH 7,0 50 mM;NaCl 200 mM;
MgCl₂10 mM; NP40 0,02%; ATP 10 mM.

A 400µl de cette solution sont ajoutés avant l'emploi 0,37 MBq (10mCi) de γ-³²P-ATP

### 2 Mutagenèse dirigée

### 2.1 Choix des mutants

L'obtention des plasmides pTYAla, pTYHis et pTYArg, est décrite dans le paragraphe I)A)a) ci-dessus. Deux autres mutants ont été obtenus:
- un mutant "non-sens" dans lequel un codon Ochre (TAA) est introduit 30 nucléotides en aval du codon d'initiation de l'ORF C1. Ce mutant permet d'étudier l'effet de l'ARN de C1 seul, sans production de la protéine correspondante. Il est appelé mutant Stop.
- un mutant Pml, dans lequel seul le site PmlI, créé dans les mutants Ala, Arg et His pour faciliter leur sélection, est introduit. Cette construction permet de confirmer l'absence d'effet de cette mutation silencieuse pour la protéine C1.

### 2.2 Obtention des mutants Pml et Stop

Le principe de la méthode employée est décrite par Kunkel en 1985: la mutation recherchée est obtenue par hybridation d'un oligonucléotide mutagène avec l'ADN viral simple-brin puis synthèse *in vitro* du brin complémentaire. L'ADN non muté est ensuite contre-sélectionné (comme décrit ci-dessus).

### Deux oligonucléotides mutagènes ont été utilisés:

Oligonucléotide "Stop":
   5' GTATCAAGGCT(AGATCT)TAATTCCTTACATATCCC 3'
Oligonucléotide "Pml":
   5' CCGGACAGGAAAGAC(CACGTG)GGCC 3'

Le nouveau site de restriction créé lors de la mutagenèse (BglII pour le mutant "Stop", PmlI pour le mutant "Pml"), est indiqué entre parenthèses. Le codon non-sens Ochre (TAA) introduit est souligné.

### 2.3 Séquençage des mutants obtenus

Le séquençage des mutants a été effectué au moyen du kit Pharmacia (Sequenase), qui utilise la méthode de terminaison de chaîne de Sanger et al (1977).

Les réactions obtenues sont déposées sur un gel d'acrylamide 6% dénaturant d'épaisseur constante 0,4mm. La migration s'effectue en tampon TBE (Sambrook et al., 1989) à 60W. L'ADN marqué par de l'α³³P-dCTP ou α³⁵S-dCTP est révélé par autoradiographie.

### 3. Etude de la réplication de l'ADN viral dans des protoplastes de tabac

(Comme décrit ci-dessus)

### 3.1 Obtention des protoplastes

(Comme décrit ci-dessus)

### 3.2 Transfection des protoplastes et Southern blot

(Comme décrit ci-dessus)

### 4 Obtention de plantes transgéniques possédant un gène qui code pour la protéine C1 sauvage ou mutée

### 4.1 Constructions introduites

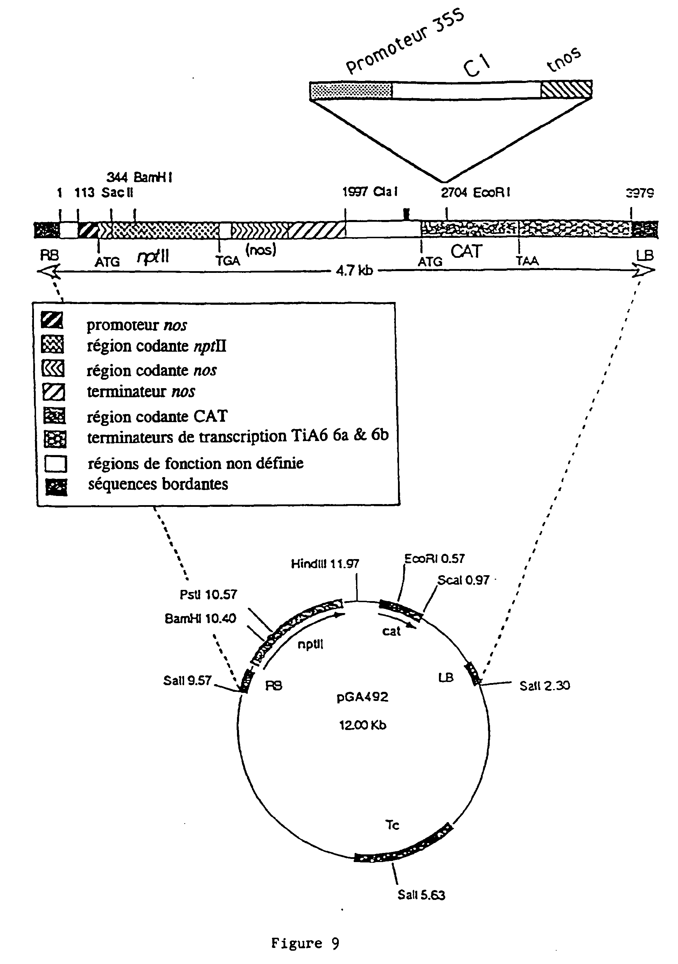

L'ORF C1 entier, sauvage ou muté, est cloné dans le vecteur binaire pGA492 (An et al., 1987), en aval du promoteur 35S du virus de la mosaïque du chou-fleur, ce qui permet une expression constitutive de l'ORF C1 dans les plantes transformées. La construction employée est détaillée sur la figure 9.

La construction ³⁵S-C1 ainsi que le gène NPTII conférant la résistance à la kanamycine, situés entre les bordures du T-DNA d'*Agrobacterium tumefaciens,* sont transférés dans le génôme des cellules végétales où ils s'intègrent de manière non contrôlée (place et nombre de copies).

### 4.2 Transformation de disques foliaires de Nicotiana benthamiana et obtention de plantes transgéniques

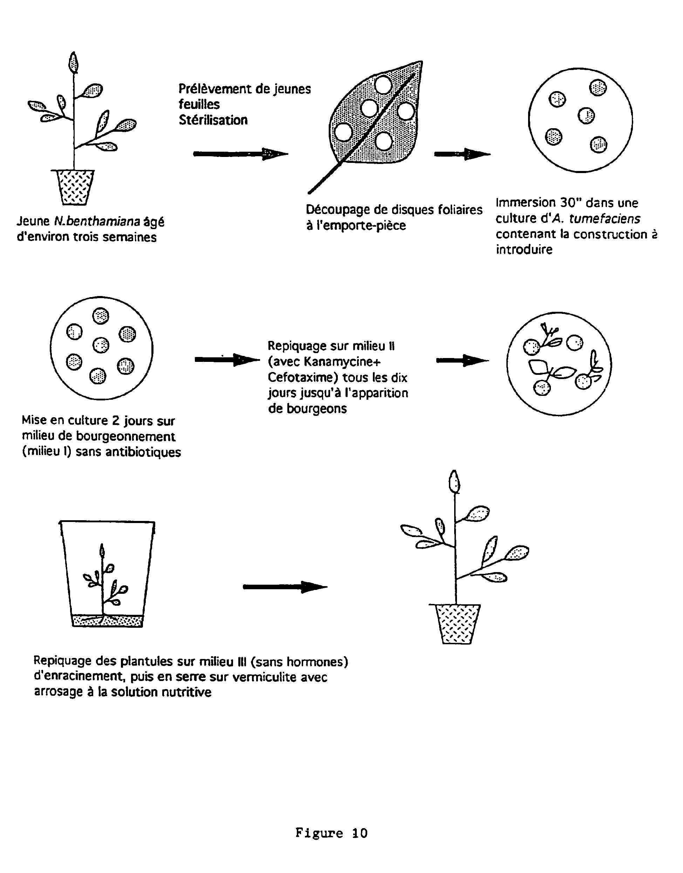

La figure 10 indique le protocole suivi pour la transformation de *N.benthamiana* par *A.tumefaciens* et la régénération de plantes transgéniques. Des plantules peuvent être acclimatées en serre environ deux mois après la transformation des disques foliaires.

### 4.3 Contrôle de l'expression de l'ORF C1 dans les plantes obtenues

Les plantes obtenues ont été maintenues, pendant toute leur période de culture *in vitro,* sur un milieu sélectif (Kanamycine 70mg/l), ce qui permet en principe de conserver uniquement celles qui ont intégré la construction recherchée.

Toutefois certains contrôles sont à effectuer pour s'en assurer.

Un "test NPTII" comme décrit ci-dessus a été effectué à partir d'extrait des plantes régénérées acclimatées en serre.

### B) RESULTATS

### 1.Activité ATPase in vitro de la protéine C1 sauvage ou mutée.

### 1.1 Activité ATPase de la protéine C1 sauvage

La protéine C1 utilisée est une protéine de fusion GST-C1.

La méthode utilisée pour mesurer l'activité ATPase de la protéine C1 permet de mettre en évidence la libération de phosphate inorganique à partir d'ATP. Dans le cas de la protéine GST-C1 sauvage, plusieurs expériences ont montré l'existence d'une telle activité: de 20 à 40% de la radioactivité totale introduite se trouvent libérés dans le surnageant à la fin de la réaction décrite précédemment, ce qui indique l'hydrolyse d'un pourcentage avoisinant de l'ATP introduit. Lors d'expériences-témoin avec la GST seule, ou avec le tampon de l'enzyme, 2 à 4% seulement de la radioactivité introduite se retrouvent dans le surnageant lors d'expériences menées en parallèle, dans les mêmes conditions.

Ces résultats semblent indiquer une activité ATPase de la protéine C1 elle-même. La technique employée ne permet pas de déterminer si l'activité de la protéine C1 conduit à l'obtention d'ADP ou d'AMP. Le pourcentage limite d'ATP hydrolysé peut être lié soit à une concentration de produit libéré inhibant le fonctionnement de l'enzyme, soit à la dégradation de celle-ci après 15 à 30 mn de réaction. Pour déterminer la cause de cette limite, des expériences de variation de concentration de l'enzyme ou de l'ATP sont à effectuer. Il reste aussi à déterminer si la protéine C1 est capable d'hydrolyser d'autres nucléotides, en particulier le GTP.

Si l'activité ATPase détectée est effectivement celle de C1, et non d'un contaminant d'origine bactérienne, la protéine C1 doit lier l'ATP. Le site de liaison aux NTP semble donc fonctionnel, ce qui permet de supposer que des mutations dans ce site peuvent être la cause de phénotypes négatifs dominants, comme c'est le cas dans d'autres modèles (Chejanovsky et Carter, 1990; Han et Sternberg 1991).

Les protéines mutées Ala, Arg, His et Pml ont donc été construites, exprimées dans *E.coli* et purifiées.

### 1.2 Obtention et activité ATPase des protéines C1 mutées

### * Obtention des protéines C1 portant les mutations Ala, Arg, His et Pml

Les protéines portant les mutations Ala, Arg, His et Pml ont été obtenues en utilisant le protocole décrit précédemment.

Les protéines GST ou GST-C1 obtenues sont extraites des bactéries et purifiées sur billes d'agarose-glutathion. Contrairement au cas de la GST seule, la quantité des protéines GST-C1 obtenue après purification est faible, et les contaminants encore nombreux. Toutefois, le degré de pureté et la concentration protéique semblent comparables pour les protéines C1 sauvage et mutée. Les protéines mutées ne semblent pas affectées dans leur stabilité par rapport à la protéine C1 sauvage.

### *Activité ATPase des protéines C1 mutées seules

L'activité ATPase des protéines mutées est mesurée selon le protocole utilisé pour la protéine C1 sauvage. L'activité ATPase des mutants Ala et His est comparable à celle de la GST seule, donc assimilable à un "bruit de fond". Le mutant Arg possède une activité qui atteint presque la moitié de celle de la protéine C1 sauvage. Le mutant Pml, qui est assimilable à la protéine sauvage puisque la mutation introduite est silencieuse, possède effectivement une activité ATPase comparable à celle de la protéine sauvage.

La réduction de l'activité ATPase chez le mutant Arg et sa perte totale chez les mutants Ala et His montrent l'importance d'une mutation ponctuelle dans le site supposé de liaison aux NTP de la protéine C1 du TYLCV. Ce site semble donc fonctionnel chez la protéine C1, et la lysine joue un rôle clé dans son fonctionnement.

La purification limitée de la protéine C1 laisse envisager la possibilité d'une activité ATPase liée à la présence de contaminants bactériens. Néanmoins, il semble peu probable que ces contaminants éventuels se trouvent éliminés de façon spécifique chez les protéines mutées.

Pour éliminer totalement l'hypothèse d'une activité liée à des contaminants bactériens, la méthode envisagée est le marquage de l'ATPase avec du γ³⁵S-ATP, capable de se lier aux enzymes mais non d'être hydrolysé. Après migration sur gel et autoradiographie, un marquage devrait apparaître pour une protéine de la taille de la fusion GST-C1 (65 kDa), puis après clivage de la protéine de fusion par une entérokinase, pour une protéine ayant le poids moléculaire apparent de la protéine C1 (42 kDa).

Cette méthode devrait aussi permettre de déterminer si les mutants ayant perdu leur activité ATPase sont toujours capables de lier l'ATP, comme c'est le cas pour la protéine RAD3 de levure (Sung et al.,1988).

### * Activité ATPase des protéines C1 sauvage et mutées

L'activité ATPase d'un mélange de protéines C1 sauvage et mutées a été étudiée. Le mélange C1 sauvage et GST est considéré comme un témoin, aucun effet négatif dominant de la GST n'étant attendu.

Les protéines mutées semblent n'avoir aucun effet sur le fonctionnement de la protéine sauvage, l'activité totale obtenue étant à peu près égale à la somme des activités de chacun des composants du mélange.

Les résultats obtenus avec les protéines C1 sauvage et mutées semblent indiquer une activité ATPase de la protéine C1 sauvage, en rapport avec la liaison de l'ATP au site défini par Walker (1982).

L'hydrolyse de l'ATP fournit probablement l'énergie nécessaire à une autre activité de C1, celle-ci restant à déterminer.

L'activité ATPase ne semble pas liée au clivage de l'ADN simple-brin, car celui-ci ne nécessite pas l'apport d'ATP. De plus, des protéines ayant perdu leur capacité de clivage en raison d'une mutation située dans un domaine supposé indépendant du site de liaison aux NTP ont conservé leur activité ATPase.

### III) RESISTANCE DERIVEE DU PATHOGENE: UNE PROTEINE NEGATIVE DOMINANTE PROTEGE DES PLANTES DE NICOTIANA BENTHAMIANA CONTRE LE VIRUS STYLC

### A) MATERIELS ET METHODES

### 1. ETUDE DE LA REPLICATION DE L'ADN VIRAL DANS DES PROTOPLASTES

### 1a. Transfection de protoplastes de tomate

Une suspension cellulaire de l'hybride interspécifique *Lycopersicon esculentum x L. pennellii*, est cultivée pendant quatre jours dans du milieu MSI* (*: voir annexe ci-après) à 25°C et avec agitation à 100 RPM. Les cellules sont récoltées par centrifugation (200 g, 5 min.) et lavées deux fois avec du milieu CPW*. Les parois cellulaires sont ensuite digérées avec trois volumes de solution enzymatique* pendant 3 heures avec agitation, à 25°C et à l'obscurité.

Les protoplastes ainsi obtenus sont ensuite filtrés deux fois sur tamis de 300 µm puis 140 µm, récoltés par centrifugation (90 g, 5 min.) et lavés deux fois avec du milieu TM2*. Les protoplastes sont comptés en cellule de Fuchs-Rosenthal et repris à une concentration de 106 protoplastes/ml.

A 5x10⁵ protoplastes, sont ajoutés 50 µg d'ADN de thymus de veau, 10 µg d'ADN viral double brin linéarisé par *Sst*I et 0.5 ml de solution PEG 1500 (40% dans du milieu F*). La suspension de protoplastes est ensuite diluée à des intervalles de 10 min. avec le milieu F* (volume final 10 ml). Les protoplastes sont récoltés (90 g, 5 min.) et cultivés à une concentration de 105 /ml dans du milieu TM2* à lumière diffuse et à 25°C. Les cellules sont récoltées sept jours plus tard pour l'extraction de l'ADN total.

### 1b. Extraction de l'ADN total de protoplastes transfectés

(Comme décrit ci-dessus)

### 1c. Analyse de l'ADN par Southern blot

(Comme décrit ci-dessus)

### 2. RECHERCHE DE PLANTES TRANSGENIQUES RESISTANTES AU STYLCV

### 2a. Agroinoculation de plantes transgéniques (régénérants primaires)

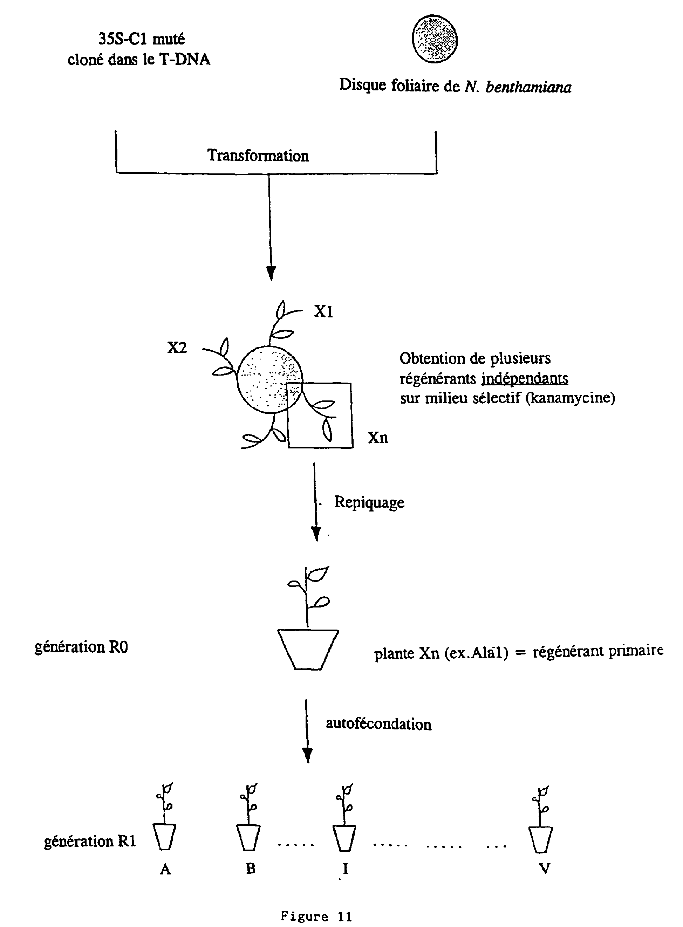

Les plantes transgéniques de *N. benthamiana* (Ala, His, Arg et Stop) ont été obtenues par transformation avec les différents gènes C1 mutés (Ala, His, Arg et Stop) (Desbiez, DEA 1993). La descendance obtenue après autofécondation (F1) des premiers régénérants (R) a été semée sans sélection et les plantes obtenues (F1), sont testées pour leur sensibilité ou résistance vis à vis du STYLCV sauvage; voir Fig.11.

Comme le STYLCV ne peut pas être transmis mécaniquement, la technique d'agroinoculation ou agroinfection (Grimsley *et al.,* 1986) a été utilisée pour infecter les plantes F1 transgéniques de *N. benthamiana.* Cette technique permet d'introduire le génome viral dans les plantes grâce au T-DNA d'*Agrobacterium tumefaciens.*

Le STYLCV sauvage a été cloné sous forme de 1.8 mer dans le vecteur binaire pBin19, devenant ainsi le plasmide pBIN19/TYLCV-S 1.8 mer (Kheyr-Pour *et al.,* 1991). Les bactéries transformées ont été sélectionnées sur milieu YEB* solide additionné de kanamycine (100 µg/ml), de rifampicine (150 µg/ml) et de néomycine (20 µg/ml). Les *A. tumefaciens* LBA4404/pBin19/TYLCV-S 1.8 mer ont été cultivés dans 20 ml de YEB* liquide (additionné d'antibiotiques comme indiqué ci-dessus), à 28°C avec agitation (200 RPM) et à l'obscurité. Après 48h d'incubation, les bactéries ont été lavées 2 fois à l'eau stérile puis remises en suspension dans 10 ml d'eau stérile.

Pour l'agroinoculation, des plantes âgées d'environ trois à quatre semaines sont utilisées. La suspension d'*A. tumefaciens* LBA4404/pBin19/TYLCV-S 1.8 mer est injectée à l'aide d'une seringue de 1ml au niveau des pétioles de trois feuilles jeunes. Les plantes sont placées dans des enceintes à 24°C, 16h de photopériode et 70% d'hygrométrie (avec une désinfection des eaux de drainage, pour éviter la contamination de l'environnement).

Pour chaque expérience 25 plantes sont agroinoculées. Deux plantes transgéniques et 2 plantes de *N. benthamiana* normales sont inoculées avec de l'eau stérile (contrôle négatif). De plus deux plantes de *N. benthamiana* normales sont inoculées dans les mêmes conditions avec la souche d'*A. tumefaciens* décrite ci-dessus (contrôle positif). Les plantes sont suivies régulièrement pour observer l'apparition de symptômes. Un mois après l'agroinoculation, les premiers symptômes caractéristiques (enroulement et jaunissement des feuilles) apparaissent sur les plantes témoins non transgéniques.

### 2b. Détection des différentes formes d'ADN viral

La présence d'ADN viral simple brin dans les plantes agroinoculées est détectée par la méthode du "squash foliaire" (Navot *et al.,* 1989): une feuille jeune est écrasée sur une membrane de nylon, et l'ADN en est fixé aux UV. Sans dénaturation préalable, la membrane est hybridée avec une sonde correspondant à l'ADN total STYLCV marqué au [α-32P] dCTP.

Pour confirmer la présence des autres formes d'ADN viral, un Southern blot est réalisé. L'ADN total est extrait selon la méthode de Matzeit *et al.* (1991), suivie d'une série d'extractions phénoliques, et enfin l'ADN est précipité à l'éthanol. Cinq µg d'ADN sont déposés dans un gel d'agarose à 1% (TAEx1*) à 2V/cm et analysés par Southern Blot (voir ci-dessus § 1.c).

### 2c. Agroinoculation de disques foliaires

Une culture d'A. *tumefaciens* LBA4404/pBin19/TYLCV-S 1.8 mer est maintenue pendant 48h (voir ci-dessus agroinoculation de plantes transgéniques). Les agrobactéries sont lavées et remises en suspension dans du milieu MS 30*. Pour chaque plante, deux jeunes feuilles sont décontaminées au Dichloroisocyanuranate de sodium (Bayrochlor, 1 comprimé par litre) pendant 15 min. Des disques foliaires sont découpés et immergés pendant 1 min. dans la suspension d'agrobactéries. Les disques sont alors séchés sur papier stérile, puis déposés sur le milieu A*. Après 48h, les disques sont transférés sur le milieu B*. L'ADN total est extrait sept jours après l'agroinoculation (voir ci-dessous).

### 2d. Extraction de l'ADN total de plantes transgéniques ou de disques foliaires agroinoculés avec le STYLCV

Un gramme de feuilles jeunes (ou de disques foliaires) est broyé dans l'azote liquide et remis en suspension dans 5 ml de solution TNES*. Deux extractions phénoliques sont effectuées pour éliminer les débris cellulaires et les protéines. Les acides nucléiques (ADN et ARN) sont précipités en présence d'acétate de sodium 0.3M (pH 5.2) et d'éthanol 70% (% final), séchés et repris dans 200 µl de TE (Tris 10 mM, EDTA 1 mM) stérile. Les ARNS sont éliminés par un traitement à la RNase A (concentration finale 200 µg/ml) pendant 1h à 37°C. La densité optique à 260 nm est mesurée. Cinq µg d'ADN sont déposés dans un gel d'agarose à 1% dans du TAEx1 (migration 2V/cm, 15h) et analysés par Southern Blot.

### 3. DETERMINATION DU NOMBRE D'INSERTS DANS LES PLANTES F1 ALA1

### 3a. Ségrégation in vitro sur kanamycine

Pour déterminer la concentration en kanamycine nécessaire pour arrêter la croissance des plantes, une gamme de concentration est réalisée pour *N. benthamiana* non tranformé (70, 100, 150, 200 et 300 µg/ml). Les graines F1 sont semées sur le milieu MS30* contenant de la kanamycine à 150 µg/ml et ' régulièrement observées pour la décoloration des feuilles.

### 3b. Southern blot

Cinq µg d'ADN génomique extraits de feuilles de plantes transgéniques F1 Ala1 avant agroinoculation sont digérés par *Hin*dIII (le choix de cette enzyme repose sur le fait qu'elle coupe une seule fois au niveau de l'insert 35S-C1). Les fragments d'ADN digérés sont séparés en gel d'agarose à 1% dans du TAEx1* (5 V/cm pendant 5h), puis transférés sur membrane de nylon Hybond-N (Amersham) pendant 6h par capillarité avec du SSCx20*. La membrane est ensuite hybridée avec une sonde ADN C1 *Pfl*MI*-Bgl*II (obtenue par coupure avec *Pfl*MI et *Bgl*II de l'ADN viral cloné dans pUC118 au niveau du site *Sst*I et séparation en gel d'agarose à bas point de fusion) marquée par amorçage aléatoire au [α-³²P] dCTP et traitée de la même manière que ci-dessus (voir analyse de l'ADN par Southern § 1.c).

### 4. ANALYSE DES PROTEINES PAR WESTERN BLOT

### 4a. Extraction des protéines totales de plantes

Cinq cent mg de feuilles jeunes sont broyés dans l'azote liquide, et les protéines totales sont précipitées à l'acide trichloroacétique 10% (4 ml/g). Le culot est lavé une fois à l'acétone 100% puis trois fois à l'acétone 90%, avant d'être séché toute la nuit au Speed-Vac (Savant). Le culot est repris dans 400 µl de tampon Tris-Glycine (25mM Tris, 250mM Glycine). La concentration en protéines totales est déterminée à 280 nm. A 20 µl du surnageant (contenant 50 µg de protéines totales) sont ajoutés 4 µl de tampon 6xSDS* et le tout est porté à 100°C pendant 4 min. et immédiatement chargé sur un gel de polyacrylamide (12.5%) en présence de SDS 0.1%. La migration se poursuit pendant 1h à 20 V/cm. Le gel est ensuite transféré sur membrane de nylon Hybond-C extra (Amersham), en présence de tampon Tris-Glycine, pendant 16h à 2 V/cm.

### 4b. Préadsorption de l'anticorps anti-C1 avec l'extrait protéique de N. benthamiana non transgénique

L'anticorps polyclonal anti-C1 a été préparé au laboratoire par purification sur gel de polyacrylamide de la bande correspondant à la protéine de fusion GST-C1 (produite chez *E. coli)* et inoculation à un lapin. Dix µl d'anticorps polyclonaux anti-C1 sont incubés avec 50 µl d'extrait protéique total de *N. benthamiana* non transgénique toute la nuit à température ambiante. La solution est ensuite centrifugée à 17000 g pendant 5 min., à température ambiante et le surnageant récupéré.

### 4c. Incubation de la membrane avec l'anticorps anti-C1 préadsorbé

La membrane est incubée pendant une heure avec une solution de PBS*, Tween20 0.1%, Lait écrémé Gloria 5% (pour saturer les sites de fixation non spécifiques de la membrane à l'anticorps). Les anticorps anti-C1 préalablement préadsorbés sont ajoutés à la membrane pendant 2h à température ambiante (dilution 1/10000).Puis trois lavages dans du PBS, Tween20 0.1% sont effectués. La membrane est alors incubée dans du sérum de chèvre anti-IgG-lapin conjugué à la phosphatase alcaline (Sigma) (dilution 1/9500) pendant 2h à température ambiante. La membrane est ensuite lavée dans du PBS, Tween20 0.1%. La révélation du complexe antigène-anticorps se fait dans 15 ml de tampon substrat* en présence de 100 µl de Nitro Blue Tetrazolium (NBT: solution mère à 50 mg/ml, Sigma) et de 50 µl de 5-Bromo-4-Chloro-3-Indoyl Phosphate (BCIP: solution mère à 50 mg/ml, Sigma). Un précipité de couleur brune apparaît et la réaction est arrêtée dans l'eau bidistillée, le tout à température ambiante et à la lumière.

### B) RESULTATS

### a) REPLICATION DES GENOMES VIRAUX SAUVAGE ET MUTES DANS DES PROTOPLASTES DE TOMATE

Pour examiner un éventuel effet négatif dominant *in vivo* de la protéine C1 mutée au niveau de la P-loop sur la réplication de l'ADN viral sauvage, les génomes viraux (sauvage et muté) sont cotransfectés dans des protoplastes de tomate. L'intérêt d'un tel système est qu'il permet d'étudier la réplication de l'ADN viral indépendamment des phénomènes de mouvement systémique du virus.

Le plasmide pTY renferme le génome double brin viral sauvage entier du STYLCV cloné dans le site *Sst*I du vecteur pUC118. Les plasmides pTYAla, pTYHis et pTYArg sont identiques à pTY, sauf qu'ils portent respectivement une mutation ponctuelle remplaçant la Lys227 en alanine, histidine et arginine. Le plasmide pTYStop contient une mutation "non sens" 30 nt en aval du codon d'initiation de la traduction de l'ORF C1. Grâce aux ligases cellulaires, les monomères de génomes viraux introduits linéarisés par *Sst*I sont recircularisés et servent ainsi de formes réplicatives intermédiaires dans le cycle viral.

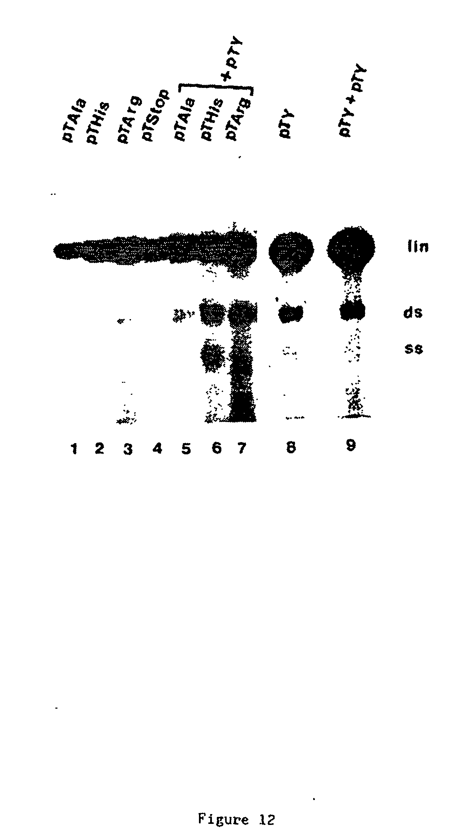

Quand les protoplastes de tomate sont transfectés avec de l'ADN viral sauvage seul, les différentes formes de l'ADN viral simple brin (ss) et double brin superenroulé (ds), s'accumulent et sont détectées. Ceci témoigne d'une réplication *de novo* du génome viral sauvage dans les protoplastes (voir Fig. 12). Le mutant pTYStop, comme attendu ne se réplique pas (car il n'y a pas traduction de l'ARN). Le mutant pTYArg se réplique mais moins bien que le sauvage. En revanche, les mutants pTYAla et pTYHis (qui sont déficients pour leur activité ATPase *in vitro)* ne se répliquent pas (ou se répliquent à un niveau non détectable) (Fig.11). Ces résultats suggèrent que la P-loop (et probablement l'activité ATPase) est nécessaire à la réplication de l'ADN viral et confirment les résultats présentés sur la figure 4 susmentionnée.

Quand l'ADN viral sauvage est cotransfecté avec les ADNs viraux mutés des résultats intéressants sont obtenus. En présence de pTYArg ou pTYHis, la réplication de l'ADN viral sauvage n'est apparemment pas altérée (présence des formes ss et ds). En revanche, pTYAla affecte considérablement la réplication de l'ADN viral sauvage puisque la forme ss est absente (ou non détectable) et la forme ds est présente en quantité plus faible (Fig.12); Cette forme ds est probablement générée par la circularisation de l'ADN viral linéaire introduit grâce aux ligases cellulaires. Ces résultats montrent clairement un effet négatif dominant du génome viral muté pTYAla sur la réplication de l'ADN viral sauvage, ce qui confirme les résultats présentés sur la figure 6 susmentionnée.

### b) AGROINOCULATION DE PLANTES F1 PAR LE STYLCV SAUVAGE

Afin de voir si les plantes transformées par les différents gènes C1 mutés sont résistantes au STYLCV sauvage, des expériences d'agroinoculation sont réalisées (Grimsley *et al.,* 1986). Des plantes de *N. benthamiana* ont été transformées par les diffèrents gènes C1 mutés (C1-Ala, C1-His, C1-Arg et C1-Stop) placés sous le contrôle du promoteur 35S du Cauliflower Mosaic Virus (CaMV). Les régénérants correspondants ont été respectivement obtenus (Ala, His, Arg et Stop) (Desbiez, DEA 1993). La première descendance F1 de ces plantes obtenue par autofécondation est semée et les plantes âgées d'environ un mois sont agroinculées avec le STYLCV. A la suite de l'agroinoculation, des monomères de virus sont libérés du 1.8-mer probablement par recombinaison homologue. Les monomères viraux libérés se répliquent et infectent la plante de manière systémique. Trois à quatre semaines après l'agroinoculation, les premiers symptômes (enroulement des feuilles) apparaîssent. La présence de l'ADN viral ss est détectée par la technique du squash foliaire (Navot *et al*., 1989), et confirmée par analyse de l'ADN total de ces plantes par Southern blot.

Les *N. benthamiana* non transgéniques (témoin positif) sont malades et présentent en squash foliaire un fort signal d'hybridation avec une sonde ADN total de STYLCV. Toutes les plantes Arg1, His21 et Stop6 sont malades et présentent toutes un fort signal d'hybridation avec la même sonde. Par contre, parmi 14 plantes Ala1 agroinoculées dans les mêmes conditions, sept ne présentent ni symptômes ni hybridation avec la sonde. L'analyse de l'ADN total par Southern blot montre pour ces plantes une absence totale des différentes formes de l'ADN viral, alors que les plantes sensibles présentent bien les différentes formes de l'ADN viral (ss, ds et forme circulaire relachée (oc)).

Pour confirmer ces résultats, des disques foliaires de plantes potentiellement résistantes sont agroinoculées avec le STYLCV. Sept jours après l'agroinoculation, les diffèrentes formes de l'ADN viral (ss et ds) sont détectées dans les disques foliaires de *N. benthamiana* non transgénique. Ceci témoigne d'une bonne infectivité du clone utilisé pour inoculer les disques foliaires. Trois plantes montrent une réplication très réduite par à rapport au témoin positif: ce sont des plantes résistantes (absence de symptômes, mais réplication résiduelle de l'ADN viral). Deux plantes ne présentent aucune forme d'ADN viral: ce sont des plantes totalement résistantes. Curieusement deux plantes présentent les différentes formes de l'ADN viral, alors qu'aucun signal n'était visible ni en squash ni en Southern blot. Ceci pourrait s'expliquer par la "pression en inoculum" utilisée dans les expériences d'agroinoculation de disques foliaires. Cette "pression en inoculum" est plus élevée par rapport à celle utilisée dans l'agroinoculation de plantes entières, et donc conduirait à une modification du rapport protéines C1 mutée: sauvage en faveur de la protéine sauvage, et par conséquent à la réplication de l'ADN viral. D'un autre côté, ce comportement (ambigu) pourrait correspondre à une inhibition du mouvement (à longue distance) du virus *in planta,* puisque l'agroinoculation de disques foliaires permet de s'affranchir des phénomènes de mouvement à longue distance.

Par ailleurs, trois autres descendances F1 de plantes Ala issues de régénérants indépendants (Ala14, Ala30 et Ala44) sont disponibles. L'agroinoculation de 35 plantes F1 Ala30 avec le STYLCV a révélé l'existence de 29 plantes potentiellement résistantes. Ce résultat conforte celui obtenu avec la descendance F1 de Ala1.

### c) CARACTERISATION MOLECULAIRE DES PLANTES ALA1 RESISTANTES AU TYLCV

La présence et le nombre de copies du gène C1-Ala présentes dans le génome des plantes Ala1 est déterminée par deux approches complémentaires. La première, génétique, repose sur l'étude de la ségrégation du phénotype résistance à la kanamycine (kanR). Ce phénotype est conféré par le gène néomycine phosphotransférase (NPTII du transposon Tn5) introduit avec le gène C1 muté pour faciliter la sélection des transformants. La deuxième, moléculaire, consiste à digérer l'ADN génomique et à effectuer un Southern.

L'expression du gène C1-Ala est analysée au niveau protéique par Western blot.

### 1. Détermination du nombre de copies du gène C1-Ala

### * approche génétique

Une gamme de concentration en kanamycine est réalisée pour N. *benthamiana* afin de déterminer la concentration nécessaire pour arrêter la croissance des plantes (décoloration des feuilles). Les résultats ont montré que pour obtenir des effets visibles, il fallait utiliser une concentration en kanamycine supérieure à 100 mg/l. Une concentration de 150 mg/l est donc utilisée.

Une ségrégation de type 3 kan^{R}:1 kanS correspondrait théoriquement à l'insertion d'une copie unique de T-DNA dans le génome de plante, alors qu'une ségrégation de type 15 kan^{R}: 1 kan^{S} correspondrait à l'insertion de deux copies de T-DNA.

Parmi 89 plantes Alal semées, 7 sont kanS et 82 sont kanR. Le test 02 montre que ces valeurs sont en accord avec une ségrégation de type 15:1 (□2 =0.45 et a <0.001,donc l'écart est non significatif et il y a moins d'une chance sur mille pour que notre hypothèse ne soit pas valable). Ces résultats suggèrent que le régénérant primaire Ala1 renfermait au moins deux copies de T-DNA dont les gènes NPTII sont exprimés.

Cette méthode est néanmoins criticable pour deux raisons:
- Le phénotype kanR est conféré par le gène NPTII, or il se pourrait qu'un remaniement ou une perte de ce gène se soit produit: on aurait alors un phénotype kan^{S} bien que le gène C1 soit présent. La situation inverse pourrait également se produire, avec perte du gène C1 et présence du gène NPTII (kan^{R}).
- Si la plante renferme plus de deux copies du gène C1, les résultats de la ségrégation deviennent difficiles à interpréter. Il faut soit augmenter la taille de l'échantillonnage, soit étudier la ségrégation kan^{R}/kan^{S} de la descendance F2.

### * approche moléculaire

Afin de déterminer le nombre exact de copies du gène C1-Ala présentes dans les plantes transformées Ala1, l'ADN de ces plantes est digéré par *Hind*III. Comme cette enzyme ne coupe qu'une fois au niveau du gène C1, le nombre de bandes qui sera révélé correspond au nombre d'inserts C1-Ala. La taille minimale attendue est de 1530 pb. Pour avoir une digestion enzymatique complète, l'enzyme utilisée est largement en excès (10 fois celle recommandée par le fournisseur) et le temps de digestion est de cinq heures au lieu d'une heure. La digestion est ensuite vérifiée sur gel.

Trois bandes sont révélées: 1600 pb, 1800 pb et 2200 pb. Ces résultats montrent que les plantes analysées sont bien transgéniques, puisqu'elles renferment une ou deux copies du gène C1-Ala. Ils suggèrent aussi que le régénérant primaire (Ala1) renfermait au moins trois copies du gène C1.

### 2. Détection de la protéine C1-Ala dans les plantes transgéniques

La protéine C1-Ala dans les plantes transgéniques Alal est analysée par Western blot. Une expérience préliminaire a révélé de nombreuses bandes non spécifiques même dans un extrait protéique de *N. benthamiana* non transgénique. Par conséquent, une étape de préadsorption de l'anticorps anti-C1 avec un extrait protéique total de *N. benthamiana* non transgénique est réalisée avant incubation de l'anticorps anti-C1 avec la membrane. Cette étape est rendue nécessaire pour épuiser la solution d'anticorps anti-C1 en anticorps qui reconnaissent de manière aspécifique les antigènes de *N. benthamiana.*

L'anticorps dirigé contre la protéine C1 reconnaît une bande d'environ 40 kDa, taille attendue pour cette protéine. Cette bande est présente chez les plantes transgéniques Ala1, D, S, U, et V, mais est absente chez *N. benthamiana* non transgénique et chez la plante Ala1 B sensible au STYLCV. Rappelons que la plante Ala1 serait "résistante" d'après le squash mais "sensible" d'après les résultats d'agroinoculation de disques foliaires. Ce comportement "ambigu" pourrait correspondre à la production de cette bande de 40 kDa.

Ces résultats démontrent que la protéine C1-Ala est exprimée dans les plantes transgéniques Ala1. Ils montrent une corrélation entre l'expression de la protéine mutée C1-Ala et la résistance au STYLCV.

### Conclusion

### a) La P-loop de la protéine C1 est essentielle à la réplication

Les protoplastes offrent un moyen puissant et rapide pour l'étude de la réplication de l'ADN viral, indépendamment des processus de mouvement systémique du virus. A la suite de transfection de protoplastes par le génome viral sauvage, les différentes formes de l'ADN viral simple brin (ss) et double brin superenroulé (ds) s'accumulent et sont détectées. La forme ds correspond à une synthèse *de novo,* plutôt qu'à une ligation de l'ADN viral linéaire introduit. Ceci a été montré pour le WDV (Matzeit *et al*., 1991). Les auteurs ont utilisé une enzyme de restriction (*Dpn*I) qui reconnaît et coupe seulement la séquence GAmTC dont l'adénine est méthylée en position N6. L'ADN viral amplifié chez *E. coli* est digéré par *DpnI,* alors que la forme ds de cellules de blé transfectées est résistante à la digestion. Ceci montre que la forme ds est néosynthétisée dans les cellules transfectées. Toutefois, une déméthylation de l'ADN introduit dans les cellules transfectées n'est pas à exclure. L'utilisation de plasmide contenant un dimère en tandem (au lieu d'un ADN linéarisé) aboutit à la formation des mêmes formes (ss et ds) qui correspondent donc à une synthèse *de novo.*

Une activité ATPase *in vitro* a été mise en évidence chez la protéine C1 du STYLCV sauvage (Desbiez, DEA 1993). Cette activité est absente chez les protéines mutées C1-Ala et C1-His, et réduite chez la protéine mutée C1-Arg. Ici, nous montrons une nette corrélation entre l'activité ATPase et la réplication de ces mutants dans les protoplastes de tomate. En effet, les deux mutants pTYAla et pTYHis ne se répliquent pas dans les protoplastes de tomate transfectés. Ces résultats montrent que le site de liaison au NTP (P-loop) est impliqué, directement ou indirectement, dans la réplication de l'ADN viral. En revanche, le mutant pTYArg (qui porte une substitution de la lysine 227 en arginine) se réplique mais moins bien que le sauvage. Le remplacement du résidu lysine 227 par l'arginine, acide aminé proche par sa structure et sa charge (positive à pH physiologique) du résidu lysine, semble affecter légèrement l'activité de le protéine C1. Ceci n'est pas le cas pour les résidus alanine et histidine. Ces résultats suggèrent un rôle essentiel de la lysine 227 de la P-loop dans la réplication de l'ADN viral.

De nombreux exemples, dans d'autres systèmes animaux et viraux, ont souligné l'importance de cette P-loop et particulièrement du résidu conservé lysine. La protéine RAD3 de *Saccharomyces cerevisiae* est impliquée dans la réparation des lésions de l'ADN. Une substitution de la lysine 48 de la P-loop par un résidu arginine entraîne une perte des activités ATPase et ADN hélicase de cette protéine (Sung *et al*., 1988). La protéine Rep de l'AAV est impliquée dans la réplication de l'ADN viral. Une substitution de la lysine 340 par une histidine produit un virus mutant incapable de se répliquer (Chejanovsky *et al*., 1990).

### b) La protéine C1 du TYLCV: quels rôles dans la réplication de l'ADN viral?

La protéine C1 (ou son homolgue AL1 chez les géminivirus à génome bipartite) est essentielle à la réplication de l'ADN viral. Parmi les différents géminivirus, c'est la protéine AL1 du TGMV qui est la plus étudiée. Cependant, les rôles de cette protéine dans la réplication ne sont pas bien élucidés. Il a été montré que la protéine AL1 du TGMV reconnaît spécifiquement une séquence d'environ 50 nt au niveau de la région commune, et préférentiellement l'ADN simple brin (Lazarowitz *et al.,* 1992b; Thömmes *et al.,* 1993). Cette interaction spécifique est nécessaire à l'initiation de la réplication. Pour la protéine C1 du TYLCV, il a été montré qu'elle possédait une activité endonucléase site spécifique au niveau du nonanucléotide très conservé dans la structure potentielle en tige-boucle de tous les géminivirus connus. La protéine AL1 du TGMV possède également une activité d'autorégulation négative sur sa propre transcription (Sunter *et al*., 1993). Le domaine protéique responsable de l'activité de répression a été identifié pour la protéine AL1 du TGMV. La signification de cette rétrorégulation demeure inconnue. L'hypothèse que nous avançons est que la protéine C1 serait suffisante pour le cycle viral en très faible quantité, d'où le phénomène de rétrorégulation observé. Il se pourrait que la surexpression de la protéine C1 soit préjudiciable à la cellule hôte et par conséquent au virus lui-même qui ne fait qu'utiliser la machinerie cellulaire.

La présence chez la protéine C1 (et AL1) d'un motif conservé GxxxxGKT/S, correspondant au site de liaison aux NTP (P-loop), a conduit à attribuer à cette protéine un rôle potentiel d'ADN hélicase (Gorbalenya et Koonin, 1993). Cette activité d'ADN hélicase pourrait, par exemple, faciliter la fixation du complexe cellulaire d'ADN polymérase comme c'est le cas pour l'antigène T de SV40 (Dodson *et al*., 1987). La mise en évidence d'une activité ATPase *in vitro* chez la protéine C1 du TYLCV a renforcé cette hypothèse. Cependant, toutes les ADN hélicases connues, à l'exception de celle du virus de la vaccine (Traktman *et al.,* 1993), possèdent une activité ATPase ADN-dépendante. Des essais *in vitro* de l'activité ATPase de la protéine C1 de TYLCV ont montré qu'elle était ADN (simple et double brin) indépendante. Il est possible que les conditions utilisées ne soient pas optimales pour mettre en évidence cette dépendance. D'autre part, la protéine C1 utilisée est sous forme de fusion GST-C1 exprimée dans un procaryote *E. coli.* Par conséquent, des modifications post-traductionnelles (phosphorylation, glycosylation, myristylation etc.) et/ou des oligomérisations pourraient être nécessaires à l'expression de cette activité ADN hélicase potentielle (ou topoisomérase ou autre(s) inconnue(s)), et ces modifications ne se produisent pas chez *E. coli.* Enfin, il est possible également que la fusion avec la GST influence le fonctionnement normal de la protéine C1. Il est intéressant de noter qu'au moins deux sites potentiellement phosphorylables existent au niveau de la P-loop: RXXT (site consensus des protéines kinases calmoduline-dépendante et RTKG site consensus reconnu par les protéines kinases de type C (Hofer *et al.,* 1992).

L'activité ATPase déterminée *in vitro* possède une activité spécifique (a.s.) de l'ordre de 3x10⁻² mol d'ATP hydrolysé/ min/ mol de proteine, qui est très faible comparée aux ATPases adénylate kinases par exemple, qui ont une a.s. de l'ordre de 2x10⁶ (Hampton et Slotin, 1975). Si une activité ATPase existe bien *in vivo* et avec la valeur d'a.s. comparable à celle déterminée *in vitro,* elle ne servirait sans doute pas à fournir l'énergie nécessaire à une fonction, par exemple hélicase ou topoisomérase, mais permettrait vraisemblablement un changement de conformation de la protéine C1 elle-même, induisant soit la formation d'un multimère de C1, soit l'interaction avec des facteurs de l'hôte. La protéine Hsp70 *d'E. coli* possède une activité du type "chaperon", c'est-à-dire capable de se lier à d'autres protéines (Buchberger et al., 1994). Cette fonction est couplée à une activité ATPase avec une a.s. de l'ordre de 3.5x10⁻². La fixation, et probablement l'hydrolyse de l'ATP, induiraient un changement de conformation de la protéine Hsp70 nécessaire à son activité de "chaperon". Il est possible qu'un changement de conformation ait lieu également chez la protéine C1 et qu'il soit nécessaire à son activité.

### c) Effet négatif dominant sur la réplication de l'ADN viral sauvage

Quand les protoplastes de tomate sont cotransfectés avec pTY et pTYArg, la réplication de l'ADN viral n'est pas altérée: l'ADN de pTY se réplique et celui de pTYArg se réplique également seul, à un niveau moindre que le génome sauvage. Le même résultat est observé avec pTY et pTYHis, bien que ce dernier ne se réplique pas seul dans les protoplastes de tomate. La protéine C1-His n'a donc pas d'effet dominant détectable sur la réplication du sauvage. Il est possible que la substitution du résidu lysine par l'acide aminé histidine ait un effet sur l'oligomérisation ou son interaction potentielles avec d'autres facteurs (voir plus loin). D'un autre côté, on peut penser que la protéine C1 sauvage "complémenterait" la déficience en réplication du mutant pTYHis. Pour montrer cela, il faudrait cotransfecter des protoplastes de tomate avec d'une part pTYHis et d'autre part une construction plasmidique renfermant l'ORF C1 sauvage sous contrôle de promoteur fort (35S du CaMV par exemple). Si notre hypothèse est vraie, on devrait observer une réplication de l'ADN viral muté pTYHis.

La cotransfection des protoplastes de tomate avec pTY et pTYAla, dont le génome ne se réplique pas seul, provoque une réduction notable de la réplication du sauvage. Ce résultat correspond à un effet négatif dominant *en trans* du mutant pTYAla sur la réplication du sauvage, et rejoint des résultats préliminaires et similaires obtenus avec des protoplastes de tabac (Mettouchi, DEA 1992). Récemment, un exemple de mutation négative dominante a été obtenu pour un virus à ARN, le Potato Virus X (PVX) (Longstaff *et al.,* 1993). La protéine de 188 K du PVX contient un motif GDD conservé parmi les ARN polymérases. L'expression de cette protéine modifiée au niveau du motif GDD dans des plantes transgéniques de *N. tabacum* (cv. Samsun NN) entraîne une résistance au PVX (absence d'accumulation de l'ARN viral).

Deux modèles peuvent rendre compte de l'effet négatif dominant observé (Herskowitz, 1987). Soit la protéine mutée C1-Ala entre en compétition avec la protéine sauvage correspondante pour un substrat ou un facteur (activateur? répresseur?) se trouvant en quantité limitante, soit elle s'associe avec la protéine correspondante sauvage (ou un facteur hôte) pour former un hétéromultimère non fonctionnel (c'est-à-dire inactif ou très vite dégradable).

L'effet négatif dominant de la protéine mutée sur la protéine sauvage a été recherché *in vitro* pour l'activité ATPase. Les résultats obtenus n'ont montré aucun effet négatif concernant l'utilisation de l'ATP. Toutefois, aucune conclusion relative à un effet *in vivo* ne peut être avancée car, la protéine C1 est sous forme de fusion GST-C1 exprimée chez *E. coli.* Si ce modèle de titration d'un facteur limitant (par exemple un activateur ou un répresseur), par la protéine mutée, a effectivement lieu *in vivo,* il suffirait de surexprimer la protéine sauvage sous promoteur fort pour contrebalancer l'effet négatif dominant (à condition, bien sûr, que la protéine mutée n'ait pas une très grande affinité pour le facteur limitant).

Le deuxième modèle propose la formation d'un hétéromultimère non fonctionnel entre la protéine sauvage et mutée (et/ou un autre facteur de l'hôte). On peut penser que l'hétéromultimère ne reconnaît plus la séquence spécifique de la région intergénique, qu'il aurait perdu la capacité d'autorégulation négative, qu'il serait instable ou très vite dégradable. Pour vérifier ce deuxième modèle, il faudrait d'abord mettre en évidence dans un système eucaryote l'existence de tels oligomères ou hétéromultimères de la protéine C1.

En l'absence de fonctions biochimiques clairement attribuées à la protéine C1, il est difficile de choisir entre les deux modèles. L'identification de facteur(s) de l'hôte interagissant avec la protéine C1 ainsi que la détermination des structures tertiaires et quaternaires de celle-ci permettraient de mieux choisir le modèle approprié pour rendre compte de l'effet négatif dominant observé et par conséquent, élucider les rôles de la protéine C1 dans la réplication de l'ADN viral.

### d) Des plantes transgéniques exprimant C1-Ala sont résistantes au TYLCV

Deux types de résultats ont été obtenus lorsqu'a été analysée la résistance au TYLCV de la descendance F1 des plantes transformées avec les diffèrents gènes C1 mutés. Les plantes F1 His21, Arg1 et Stop6 sont toutes sensibles au STYLCV. La descendance F1 Ala1 montre, pour cinq plantes sur 14 analysées, une résistance à ce virus. Ces résultats confortent ceux obtenus avec les expériences de cotransfection, dans lesquelles seul le mutant pTYAla inhibe la réplication du génome sauvage.

Nous avons utilisé trois méthodes expérimentales pour tester la résistance de ces plantes vis-à-vis du TYLCV. Le premier niveau repose sur la détection de la forme virale simple brin (ss) par la méthode du squash foliaire. Le deuxième niveau consiste à extraire l'ADN total de la plante et à analyser les diffèrentes formes de l'ADN viral par Southern blot. Enfin, le troisième niveau fait appel à la méthode d'agroinoculation de disques foliaires de ces plantes par le STYLCV. Cette dernière méthode est beaucoup utilisée dans les programmes de sélection de résistance au TYLCV (et aux géminivirus en général).

Utilisant ces trois approches, nous avons pu obtenir deux types de réponse. Trois plantes Ala1 présentent une résistance incomplète au STYLCV, puisque l'agroinoculation de disques foliaires de ces plantes conduit à un faible niveau de réplication par rapport aux plantes sensibles. Deux plantes Ala1 montrent une résistance totale (aucune réplication n'est observée avec les trois expériences).

Curieusement, deux plantes Alal, qui, si l'on s'en tient aux résultats du squash et du Southem, seraient résistantes, se comportent comme les plantes sensibles (avec un niveau élevé de réplication) après agroinoculation de disques foliaires. Nous avons interprété ce phénotype comme correspondant soit a une "pression en inoculum" élevée modifiant le rapport protéine C1 mutée: protéine C1 sauvage, soit probablement à une inhibition de mouvement viral *in planta.*

Notons que les sept plantes Ala1 précitées ne présentent aucun symptôme caractéristique du TYLCV même trois mois après leur agroinoculation.

Ces résultats suggèrent un effet négatif dominant *in planta* et en *trans* de la protéine C1-Ala mutée surexprimée sur la réplication de l'ADN viral sauvage. Le mécanisme rendant compte de cet effet *in planta* est probablement le même que celui ayant lieu dans les protoplastes.

Nous avons analysé la présence et l'expression du gène C1-Ala introduit dans ces plantes résistantes. Le gène est effectivement présent en une ou deux copies chez les plantes résistantes mais aussi chez certaines plantes sensibles analysées. Une corrélation entre d'une part la présence et le nombre de copies du gène C1-Ala et d'autre part la résistance ne peut pas être établie. Ceci peut être dû soit à un effet de position (insertion dans une région "silencieuse" du génome), soit à un remaniement du gène entraînant une sensibilité au TYLCV.

Les plantes Ala1 résistantes au TYLCV expriment toutes la protéine C1-Ala, alors qu'une plante sensible, à l'exception de l'une d'elles, ne l'exprime pas. En attendant d'analyser davantage de plantes Ala1 (et d'autres plantes F1 Ala disponibles), on peut dire qu'il y a une corrélation entre l'expression de la protéine et la résistance.

### ANNEXE

### milieux pour protoplastes de tomate

| milieu MSI | | milieu CPW | |
|---|---|---|---|
| Sels MS (Sigma) | 4.6 g | mannitol | 440 mM |
| vitamines Morel | 1 ml | MES | 100 mM |
| saccharose | 20 g | CaCl₂ | 10 mM |
| myoinositol | 100 mg | KH₂PO₄ | 0.19 mM |
| 2,4 D | 1 mg | KNO₃ | 1 mM |
| CaCl₂, 2 H₂O | 7 mM | MgSO₄ | 1 mM |
| H₂O (q.s.p.) | 11 | H₂O (q.s.p.) | 1 l |
| pH 5.7 | | pH 5.7 | |

| solution enzymatique pour protoplastes de tabac | |
|---|---|
| (dans du CPW) | |
| cellulase | 2% |
| macérozyme | 0.1% |
| pectolyase | 0.01% |

| milieu TM2 | | milieu F | |
|---|---|---|---|
| macroéléments | 100 ml | NaCl | 140 mM |
| microéléments | 1 ml | KCl | 5 mM |
| vitamine | 1 ml | Na₂HPO₄ | 0.75 mM |
| saccharose | 68.4 g | glucose | 54 mM |
| mannitol | 4.6 g | CaCl₂ | 150 mM |
| xylitol | 3.8 g | MES | 1 mM |
| sorbitol | 4.6 g | pH 5.5 | |

### tampons d'extraction

Tampon TE: TRIS 10 mM, EDTA 1 mM, pH 7,5 - 8

| Tampon TNES | | Tampon X | |
|---|---|---|---|
| (extraction d'ADN de plantes) | | (extraction d'ADN de protoplastes de tabac et de tomate) | |
| Tris-HCl (pH8) | 100 mM | Tris-HCl (pH8) | 100 mM |
| NaCl | 100 mM | NaCl | 100 mM |
| EDTA | 10 mM | EDTA | 50 mM |
| SDS | 1% | SDS | 0.5% |
| | | | |

### milieux de culture

| milieu YEB (pour les agrobactéries) | | milieu MS30 | |
|---|---|---|---|
| Bacto-beef extract | 5 g | sels MS (macro et micro éléments) (Sigma) | 4.6 g |
| Bacto-yeast extract | 1 g | saccharose | 30 g |
| peptone | 6 g | H₂O (q.s.p.) | 1 1 |
| saccharose | 5 g | pH 5.8 | |
| Mg SO4 | 0.5 g | | |
| H₂O (q.s.p.) | 1 1 | (MS = Murashige et Skoog) | |
| pH 7.2 | | | |

| milieu A | | milieu B | |
|---|---|---|---|
| MS30 + | ANA 0.1 g/l | milieu A + | |
| | BAP 1 g/l | kanamycine | 70 mg/l |
| | Vitamines de Morel | céfotaxime | 500 mg/l |

### solutions pour Southern

| TAEx1 | | SSCx20 | |
|---|---|---|---|
| Tris-acétate | 0.04 M | NaCl | 175 g |
| EDTA | 0.001 M | Na-citrate | 88 g |
| | | H₂O (q.s.p.) | 1 l |

| TBE x 1 | |
|---|---|
| Tris-borate | 0,09 M |
| EDTA | 0,002 M |

| solution d' hybridation | |
|---|---|
| NaH₂PO₄ | 0.5 M |
| SDS | 7% |
| EDTA | 1 mM |
| BSA | 10 g/l |

### solutions pour Western

| PBS | | Tampon substrat | |
|---|---|---|---|
| (Phosphate-Buffered Saline) | | (pour phosphatase alcaline) | |
| NaCl | 140 mM | NaCl | 100 mM |
| KCl | 2.7 mM | MgCl2 | 5 mM |
| Na2HPO4 | 10 mM | Tris-HCl (pH 9.5) | 100 mM |
| KH2PO4 | 1.8 mM | | |
| pH 7.3 | | | |

| Tampon 6xSDS | |
|---|---|
| Tris-HCl (pH 6.8) | 350mM |
| SDS | 0.3% |
| glycérol | 36% |
| DTT | 9% |
| Bleu de bromophénol | 0.012% |

### Milieux et gels utilisés pour l'étude de la protéine Rep in vitro

| **Milieu MTPBS** | |
|---|---|
| * NaCl | 100 mM |
| * Na₂HPO₄ | 16 mM |
| * NaH₂Po₄ | 4 mM |
| pH 7,3 | |

### Composition des gels utilisés: Gel Laemmli 10%

| Gel de tassement (2,5 ml) | | Gel résolutif (5 ml) | |
|---|---|---|---|
| H2O | 1,9 ml | H2O | 1,67 ml |
| Tris 1 M pH 6,8 | 0,31 ml | Tris 1 M pH 8,8 | 1,63 ml |
| SDS 20% | 30 µl | SDS | 30 µl |
| Acrylamide-bisacrylamide 30% | 0,32 ml | Acrylamide-bisacrylamide 30% | 1,65 ml |
| APS 10% | 25 µl | APS | 25 µl |
| TEMED | 4 µl | TEMED | 4 µl |

### Solution de Bleu de Coomassie:

* 2,5 mg/l de Bleu de Coomassie Brillant R250
* 20% Ethanol
* 10% Acide acétique

Les gels sont mis à colorer la nuit dans cette solution, puis l'excès de coloration est éliminé par rinçage dans un mélange d'éthanol 20% - acide acétique 10% pendant au moins une heure.

| | | | | | |
|---|---|---|---|---|---|
| **Milieu I:** | MS30 + | ANA | 0,1 mg/l | | |
| | | BAP | 1 mg/l | | |
| | | Vitamines de Morel: | | Panthoténate de Ca | 1 mg/l |
| | | | | Méso-Inositol | 100 mg/l |
| | | | | Biotine | 0,01 mg/l |
| | | | | Acide nicotinique | 1 mg/l |
| | | | | Pyridoxine (Vit. B6) | 1 mg/l |
| | | | | Thiamine (Vit. B1) | 1 mg/l |

| | | |
|---|---|---|
| **Milieu II:** Milieu I + | Kanamycine | 70 mg/l |
| | Cefotaxime | 500 mg/l |

**Milieu III:** Milieu II sans ANA et BA

Les trois milieux sont utilisés solides (agar 9 g/l).

### IV) CONSTRUCTION DE TOMATES TRANSGENIQUES (L. esculentum) EXPRIMANT LES PROTEINES REP MUTEES ET PROTEGEES CONTRE LES PATHOLOGIES PROVOQUEES PAR LES STYLCV

### 1) Protocole utilisé pour la transformation génétique de la tomate (Lycopersicon esculentum)

Le protocole suivi s'inspire de deux publications.

D'une part celle de S. McCormick et al. (1986): leaf disk transformation of cultivated tomato (Lycopersicon esculentum) using Agrobacterium tumefaciens. Plant Cell Reports 5:81-84, d'autre part, celle de J.J. Fillati et al. (1987): Efficient transfert of a glyphosate tolerance gene into tomato using a binary Agrobacterium tumefaciens.vector. Bio/technology 5:726-730.

Les explants utilisés sont les jeunes cotylédons encore en croissance, avant l'émergence des premières feuilles.

Ils proviennent de germinations stériles in vitro.

Les souches d'Agrobacterium contenant les plasmides qui renferment le gène d'intérêt (à savoir les séquences nucléotidiques mutées C1* telles que représentées sur la figure 13) sont cultivées en milieu liquide pendant deux jours à 28°C, à l'obscurité et avec agitation.

La succession des opérations est la suivante :
Jour 1 : mise en culture des bactéries en milieu minimum avec antibiotiques. Préparation de boîtes de Pétri (milieu solide) contenant une suspension cellulaire de tabac en pleine croissance: il s'agit des couches-nourrices.
Jour 2 : découpage minutieux des fragments de cotylédons (deux fragments par cotylédon), en évitant toute oxydation des tissus (sous l'eau). Mise en culture de ces fragments, face supérieure dirigée vers le bas, sur les couches-nourrices recouvertes au préalable d'un papier filtre Whatman n° 1. Cette incubation doit se poursuivre pendant une durée minimale de 8 heures, à 25°C, en lumière atténuée.
Jour 3 : centrifugation des agrobactéries et reprise de celles-ci dans un milieu adapté aux tissus végétaux en prenant le soin d'ajuster la DO (à 590 nm) de la suspension entre 0,4 et 0,5.
Immersion rapide des explants de tomate dans cette suspension et mise en culture de ceux-ci sur les mêmes couches-nourrices : il s'agit de la co-culture plantes-bactéries, qui se poursuit pendant 40 heures, à 25°C, en lumière atténuée.
Jour 5 : transfert des explants sur un milieu permettant la régénération de bourgeons (régulateurs de croissance) et la sélection des cellules transformées (antibiotiques appropriés). Les fragments de cotylédons sont alors déposés selon une polarité normale. Ils sont transférés sur de nouveaux milieux toutes les deux à trois semaines, jusqu'à l'apparition de bourgeons.

Lorsque les bourgeons sont bien développés, ils sont mis sur un milieu permettant l'enracinement. Bien enracinées, les plantes sont acclimatées en serre.

Milieux utilisés pour la transformation de la tomate :
Milieux pour bactéries :
Milieu minimal pour la culture des Agrobacterium, pour 1 litre :

| | |
|---|---|
| K₂HPO₄ | 10,5 g |
| K₂HPO₄ | 4,5 g |
| (NH₄)₂SO₄ | 1,0 g |
| Citrate de sodium, 2H₂O | 0,5 g |

Autoclaver à 120°C, 30 mn, dans un volume total de 990 ml. Avant emploi, ajouter les composés suivants, stériles: stérilisés également à l'autoclave

| | |
|---|---|
| MgSO₄, H₂O 1M | 1 ml (120°C, 30 mn) |
| Glucose 20% | 10 ml (110°C, 30 mn) |

Stérilisé par filtration (0,45 µm).
Tétracycline à une concentration finale de 5 mg/l pour le vecteur utilisé.
Milieu liquide pour remettre en suspension les agrobactéries :
Eléments minéraux de Murashige et Skoog, déjà décrits ci-dessus pour la transformation de Nicotiana benthamiana:

| | |
|---|---|
| Saccharose | 30 g |
| pH | 5,8 |

Autoclaver à 110°C, pendant 20 mn.
Milieux pour plantes :
Milieu pour la germination des graines de tomate :
Pour 1 litre :
Elément minéraux de Murashige et Skoog

| | |
|---|---|
| Saccharose | 30 g |
| Agarose | 6 g |
| pH | 5,8 |

Autoclaver à 110°C, pendant 20 mn. Verser dans des tubes de culture stériles.
Milieu pour la suspension BY2 :
Déjà décrit ci-dessus pour l'entretien de cette suspension cellulaire utilisée également pour la transfection de protoplastes de tabac.
Ce milieu est simplement solidifié avec 0,6% d'agarose et coulé en boîtes de Pétri. Un ml de suspension est étalé sur sa surface.

Milieu permettant la sélection des cellules transformées et la régénération de bourgeons:
Pour 1 litre :
Eléments minéraux de Murashige et Skoog

| | |
|---|---|
| Myo Inositol | 100 mg |
| Saccharose | 20 g |
| Gelrite (Serva) | 2 g |
| pH | 6,0 |

Autoclaver à 110°C, pendant 20 mn.

Avant emploi, ajouter les composés suivants, stérilisés par filtration :
Riboside de zéatine 2 mg
Vitamines de Nitsch :

| | |
|---|---|
| Thiamine | 0,5 mg |
| Glycine | 2,0 mg |
| Acide nicotinique | 5,0 mg |
| Pyridoxine | 0,5 mg |
| Acide folique | 0,5 mg |
| Biotine | 0,05 mg |

Une solution concentrée 1000 fois de ce mélange est préparée, fractionnée en volumes de 1 ml et stockée à -20°C.

Pour 1 1 de milieu, ajouter 1 ml de solution-stock.

### Antibiotiques :

| | |
|---|---|
| Céfotaxime | 500 mg (pour éliminer les agrobactéries) |
| Kanamycine | 100 mg (sélection des cellules transformées) |

Milieu d'enracinement :
Pour 1 litre:
Eléments minéraux de Murashige et Skoog dilués de moitié

| | |
|---|---|
| Saccharose | 5 g |
| Gelrite | 2 g |
| pH 6,0 | |

Autoclaver à 110°C, pendant 20 mn.

Avant emploi, ajouter les composés suivants, stérilisés par filtration :

| | |
|---|---|
| Céfotaxime | 200 mg |
| Kanamycine | 50 mg. |

### 2) Agroinoculation de tomates (Lycopersicon esculentum) avec le STYLCV

Les expériences d'agroinoculation ont été réalisées suivant le procédé décrit ci-dessus pour *N. benthamiana.*

Plantes inoculées : régénérants primaires T0 parfaitement enracinés in vitro sur le milieu de culture in vitro sélectif contenant de la kanamycine (50 mg/l).

Résultats obtenus après hybridation moléculaire selon la technique de squash blot en utilisant comme sonde l'ADN total du STYLCV, marqué au ³²P.

Conclusion générale :sur un ensemble de 11 régénérants primaires T0 indépendants, 4 obtenus avec la construction Ala et 7 avec la construction His, 4 (2 avec l'une des constructions et 2 avec l'autre) sont dépourvus d'ADN viral après agroinoculation et sont donc de bons candidats pour une résistance au virus STYLCV (voir le Tableau 1 ci-joint).

**Tableau 1**

| Régérants primaires T0 inoculés | | Effectif total inoculé | Essai du 21/07/95 | | Effectif total inoculé | Essai du 11/08/95 | |
|---|---|---|---|---|---|---|---|
| | | | S | R | | S | R |
| Cultivar I Ala | a | 9 | 4 | 5 | 25 | 16 | 9 |
| | b | 1 | 1 | 0 | 2 | 2 | 0 |
| | c | 1 | 1 | 0 | 3 | 1 | 2 |
| Cultivar II | a | non testé | | | 1 | 1 | 0 |
| | | | | | | | |
| Cultivar I His | a | 1 | 0 | 1 | 2 | 2 | 0 |
| Cultivar II | a | 1 | 0 | 1 | 1 | 1 | 0 |
| | b | 1 | 1 | 0 | 1 | 1 | 0 |
| | c | 1 | 1 | 0 | 2 | 0 | 2 |
| Cultivar III | a | 1 | 1 | 0 | 2 | 2 | 0 |
| Cultivar IV | a | 2 | 1 | 1 | . 5 | 3 | 2 |
| | b | 1 | 1 | 0 | 1 | 1 | 1 0 |

| | | | | | | | |
|---|---|---|---|---|---|---|---|
| S : plante sensible, présence d'ADN viral sur le squash blot | | | | | | | |
| R : plante résistante, absence d'ADN. | | | | | | | |

Les effectifs sont faibles car il s'agit des premières plantes de tomates transgéniques obtenues, acclimatées peu à peu en serre.

### V) AGROINOCULATION DE NICOTIANA BENTHAMIANA AVEC L'ISOLAT JORDANIEN DU ITYLCV (ITYLCV-Jo):

Les expériences d'agroinoculation ont été effectuées selon le protocole décrit ci-dessus.

Plantes inoculées :descendance T2, ou deuxième génération obtenue après autofécondation de plantes T1 comportant les mutations Ala et His qui sont résistantes au STYLCV, et qui produisent en quantité importante la protéine Rep mutée.

Résultats obtenus après hybridation moléculaire selon la technique de squash blot en utilisant comme sonde l'ADN total du ITYLCV-Jo, marqué au ³²P.

Conclusions générales : dans un ensemble de plantes T2 issues de quatre plantes T1 sélectionnées pour la présence de Rep et la résistance au STYLCV et lors de deux répétitions de la même expérience, certaines d'entre elles montrent une résistance face à un autre virus, le ITYLCV-Jo, dont la séquence nucléotidique ne présente que 75% d'homologie avec celle du STYLCV.

La résistance conférée par la production d'une protéine Rep altérée codée par une séquence C1 mutée du STYLCV semble donc induire une résistance vis-à-vis d'un autre géminivirus, le ITYLCV-Jo (voir le Tableau 2 joint). De plus, ces virus sont suffisamment éloignés pourqu'il ne puisse pas y avoir complémentation croisée pour les fonctions de la protéine Rep (Jupin I., Héricourt F., Benz B. et Gronenborn B. (1995), DNA replication specificity of TYLCV geminivirus is mediated by the aminoterminal 116 amino acids of the Rep protein, FEBS Letters, 362: 116-120.

**Tableau 2**

| Plantes T1 | | Nombre de T2 inoculées | Essai du 23/06/95 | | Nombre de T2 inoculées | Essai du 03/08/95 | |
|---|---|---|---|---|---|---|---|
| | | | S | R | | S | R |
| His | a | 11 (10) | 1 (6) | 10 (4) | 12 | 11 | 1. |
| | b | 10 (12) | 5(10) | 5(2) | 23 | 21 | 2 |
| | | | | | | | |
| Ala | a | 11(8) | 6(4) | 5(4) | 22 | 18 | 4 |
| | b | 10(9) | 7(2) | 4(7) | 23 | 23 | 0 |

| | | | | | | | |
|---|---|---|---|---|---|---|---|
| S : plante sensible, présence d'ADN viral sur le squash blot | | | | | | | |
| R : plante résistante, absence d'ADN. | | | | | | | |
| (×) : résultats obtenus en parallèle après inoculation du STYLCV | | | | | | | |

### Légendes des figures:

- figure 1: organisation génomique du STYLCV. Les flèches noires représentent les ORFs des brins viraux (V1 et V2) et complémentaires (C1, C2, C3, C4). La position de la structure en épingle à cheveux est notée par (¡). L'ORF V1 semble coder pour une protéine impliquée dans le mouvement systémique, l'ORF V2 code pour la protéine de capside, l'ORF C1 code pour la seule protéine indispensable à la réplication, l'ORF C2 code pour un activateur de transcription des ORFs du brin viral, les ORF C3 et C4 n'ont pas de fonctions connues;
- figure 2: représentation schématique des plasmides pTY Ala, His, Arg, du plasmide pBMC-S et des plasmides pC1 Ala, His, Arg; les principaux sites de restriction de ces différents plasmides sont indiqués;
- figure 3: les mutations touchent le codon de la lysine en position 227 (codée par AAG) et résultent en sa substitution par Ala (codée par GCG), His (codée par CAT) et Arg (codée par AGG);
- figure 4: réplication des mutants dans le site de liaison aux NTP du STYLCV; l'autoradiogramme résultant de l'analyse selon Southern est quantifié à l'aide d'un analyseur d'images (Bio-Images, Millipore), qui attribue aux taches une valeur de densité optique. Le graphe représente le pourcentage de formes réplicatives après trois (en noir) et cinq (en blanc) jours de culture des cellules transfectées. pTYLCV: génome viral sauvage; pTYAla, pTYArg: génomes viraux mutés correspondant au changement de K en A et R respectivement;
- figure 5: graphe issu de la quantification de l'autoradiogramme obtenu lors de l'étude d'un effet trans-dominant de la mutation K en R, par un test d'activité NPT II. Les constructions indiquées sur le document ont été cotransfectées avec le génome viral recombinant codant pour la néomycine phosphotransférase (pTYNéo). L'activité NPT II est recherchée après 3 et 5 jours de culture des cellules transfectées. Les intensités ont été normalisées par rapport au signal correspondant à la cotransfection du génome recombinant avec le vecteur pUC.
- figure 6: graphe issu de la quantification de l'autoradiogramme obtenu lors de l'étude de l'effet de la transfection des génomes viraux mutés (pTYAla, pTYHis, pTYArg) sur la réplication du génome viral (pTYLCV). le pourcentage de formes réplicatives est représenté pour les différents échantillons après 3 et 5 jours de culture des cellules transfectées. Le témoin correspond à la cotransfection du virus sauvage avec du vecteur pUC de façon à transfecter la même quantité d'ADN;
- figure 7: graphe issu de la quantification de l'autoradiogramme obtenu lors de l'étude de l'effet de la cotransfection de constructions permettant l'expression à haut niveau des protéines C1 mutées (pC1Ala, pC1His, pC1Arg) sur la réplication du génome viral (ici pTYNéo);
- figure 8: les plasmides pTYLCV et pTY-Ala, Arg, His, Pm1, Stop sont composés du plasmide pUC118 dans lequel est cloné au site unique SstI le génome entier du TYLCV sauvage ou muté. Le plasmide pGEXC1 est constitué du plasmide pGEX3 (Amrad) dans lequel est cloné au site BamHI le gène C1. Le répresseur lac, produit du gène lacI, se lie au promoteur Ptac et réprime l'expression de la protéine de fusion GST-C1. En présence d'IPTG, l'expression de la protéine de fusion est déréprimée;
- figure 9: clonage de l'ORF C1 en aval du promoteur 35S dans le plasmide pGA492. Ce plasmide binaire possède les éléments (bordures du T-DNA d'A. *tumefaciens)* nécessaires en *cis* au transfert d'ADN dans les plantes. Les autres éléments sont fournis par un plasmide auxiliaire présent dans la souche d'A. *tumefaciens* LBA4404. Les plasmides contenant les mutations introduites ont été obtenus en remplaçant un fragment SstI-BgIII (SstI-PflmI pour le mutant Stop) du gène C1 sauvage par un gène muté. La construction est ensuite clonée au site unique EcoRI du plasmide pGA492;
- figure 10: transformation de disques foliaires de *N. benthamiana* et obtention de plantes transgéniques exprimant l'ORF C1 sauvage ou muté. Partant d'un jeune plant de *N. benthamiana* âgé d'environ trois semaines, on effectue un prélèvement de jeunes feuilles et une stérilisation. Les disques foliaires sont découpés à l'emporte-pièce, puis immergés 30 secondes dans une culture d'*A*. *tumefaciens* contenant la construction à introduire. Puis les disques sont mis en culture 2 jours sur milieu de bourgeonnement (milieu I) sans antibiotiques. On effectue un repiquage sur milieu II (avec Kanamycine + Cefotaxime) tous les dix jours jusqu'à l'apparition de bourgeons. Les plantules obtenues sont repiquées sur milieu III (sans hormones) d'enracinement, puis en serre sur vermiculite avec arrosage à la solution nutritive. La composition des milieux I, II et III est fournie en annexe.
- figure 11: schéma simplifié d'explication de l'obtention des plantes de la première génération F1;
- figure 12: réplication de l'ADN viral dans des protoplastes de tomate. Les protoplastes sont soit transfectés (puits 1 à 4 et 8), soit cotransfectés (puits 5 à 7 et 9) avec le génome viral sauvage ou muté. Sept jours après, l'ADN total est extrait, séparé dans un gel d'agarose et transféré sur membrane nylon. L'hybridation se fait avec une sonde correspondant à l'ADN total de TYLCV-Sar marquée au ³²P. Exposition de 15 h à -70°C. ss: forme simple brin; ds: forme superenroulée double brin; lin: forme linéaire.
- figure 13: séquences en acides nucléiques et en acides aminés des ORF C1 mutés (C1*) du STYLCV et des protéines Rep altérées correspondantes. La séquence nucléotidique commence à l'ATG initiateur. La protéine Rep comprend 359 acides aminés. Les acides nucléiques et les acides aminés sauvages sont indiqués par des majuscules. Les acides nucléiques mutés sont indiqués par des minuscules et les acides aminés mutés sont indiqués en majuscules et en italique. Le codon n° 227 (AAG) codant pour la lysine (K) de la protéine Rep sauvage a été remplacé par le codon gcG pour obtenir la protéine Rep K227A, où l'alanine remplace la lysine. Ce même codon a été remplacé par le codon cAt pour obtenir la protéine Rep K227H, dans laquelle l'histidine remplace la lysine. Enfin, le remplacement de ce codon par le codon AgG permet d'obtenir la protéine Rep K227R, où l'arginine remplace la lysine. (PmlI) indique le site de restriction cACGTG introduit pour la sélection des mutants. Il s'agit d'une mutation silencieuse qui n'altère pas la séquence protéique.

### BIBLIOGRAPHIE

Accotto G.P., Donson J., and Mullineaux P.M. 1989. Mapping of Digitaria streak virus transcripts reveals different RNA species from the same transcription unit [published erratum appears in EMBO J 1989 Nov;8(11):3542]. *Embo J,* **8:** 1033-9.
Allex D., Laterrot H., Kheyr-Pour A., Machoux G., et Hostachy B. 1994. La maladie des feuilles jaunes en cuillère de la tomate: une virose à venir? *PHM Revue Horticole* n° **350**: 13-17.
An G., 1987. Binary Ti vectors for plant transformation and promoter analysis. *Methods in Enzymology* **153**: 292-305.
Ausubel F.M., Brent, R., Kingston R.E., Moore D.D., Seidman J.G., Smith J.A., Struhl K., 1989. *Current Protocols in Molecular Biology.* Wiley Interscience, N.Y.
Buchberger A., Valencia A., McMacken R., Sander C. and Bukau B. 1994. The chaperone function of DnaK requires the coupling of ATPase activity with substrate binding through residue E171. *Embo J*., **13**: 1687-1695.
Chejanovsky N. and Carter B.J. 1990. Mutation of consensus purine binding site in the adeno-associated virus rep gene generates a dominant negative phenotype for DNA replication. *J. Virol.,* **64**: 1764-70.
Czosnek H., Navot N., and Laterrot H. 1990. Geographical distribution of Tomato Yellow Leaf Curl Virus. A first survey using a specific DNA probe. *Phytopath. Medit.,* **29**: 1-6.
Davies JW. and Stanley J. 1989. Geminivirus genes and vectors. *Trends Genet.,* **5**: 77-81.
Day A.G., Bejarano E.R., Buck K.W., Burrell M. and Lichtenstein C.P. 1991. Expression of an antisense viral gene in transgenic tobacco confers resistance to the DNA virus tomato golden mosaic virus. *Proc. Natl. Acad. Sci. USA*, **88**: 6721-6725.
Desbiez C. 1993. Mise en évidence d'une activité ATPase chez la protéine Rep d'un géminivirus. Etude de mutations dans son site de liaison aux NTP. Rapport de DEA de Phytopathologie. Université Paris VI, Université Paris XI et Institut National Agronomique Paris-Grignon.
Dodson M., Dean F.B., Bullock P., Harrison E. and Hurwitz J. 1987. Unwinding of duplex DNA from the SV40 origin of replication by T antigen. *Science,* **238**: 964-967.
Elmer J.S., Brand L., Sunter G., Gardiner W.E, Bisaro D.M. and Rogers S.G. 1988. Genetic analysis of tomato golden mosaic virus II. Requirement for the product of the highly conserved AL1 coding sequence for replication. *Nucleic Acids Res.,* **16**: 7043-7060.
Eshed Y., Abu-Abied M., Saranga Y. and Zamir D. 1992. *Lycopersicon esculentum* lines containing small overlapping introgressions from *L. pennellii. Theor. Appl. Genet.,* **83**: 1027-1034.
Fontes EPB., Luckow VA. and Hanley-Bowdoin L. 1992. A gemenivirus replication protein is a sequence-specific DNA binding protein. *The Plant Cell* **4**: 597-608.
Francki R.I.B., Hatta T., Boccardo G., and Randles J.W.. 1980. The composition of chlorosis striate virus, a geminivirus. *Virology*, **101:** 233.
Frischmuth T. and Stanley J. 1993. Strategies for the control of geminiviruses diseases. *Seminars in* Virology. **4**: 329-337.
Gorbalenya A.E., and Koonin E.V. 1989. Viral proteins containing the purine NTP-binding sequence pattern. *Nucleic Acids Res.,* **17**: 8413-8440.
Gorbalenya A. E. and Koonin E. V. 1993. Helicases: amino acid sequence comparisons and structure-function relationships. *Current Opinion in Structural Biology,* **3**: 419-429.
Grimsley N., Hohn B., Hohn T., and Walden R. 1986. "Agroinfection" an alternative route of viral infection of plants by using the Ti plasmid. *Proc Natl Acad Sci U S A,* **83**: 3282-3286.
Hampton A. and Slotin L.A. 1975. Inactivation of rabbit, pig and carp adenylate kinases by N6-o- and p-Fluorobenzoyladenosine 5'-triphosphates. *Biochemistry*, **14**: 5438-44.
Han M., Sternberg P.W., 1991. Analysis of dominant negative mutations of the *Caenorhabditis elegans let-60* ras gene. *Genes & Developement* **5**, 2188-2198.
Hanley-Bowdoin L., Elmer J.S., and Rogers S.G. 1988. Transient expression of heterologous RNAs using tomato golden mosaic virus. *Nucleic Acids Res **16**:* 10511-18.
Harrison B.D. 1985. Advances in geminivirus research. *Annu. Rev. Phytopathol.,* **23:** 30-139.
Herskowitz I. 1987. Functional inactivation of genes by dominant negative mutations. *Nature,* **329**: 219-222.
Hofer J.M.I., Dekker E.L., Reynolds H.V., Woolston C.P., Cox B.S and Mullineaux P.M. 1992. Coordinate regulation of replication and virion sense gene expression in wheat dwarf virus. *The Plant Cell,* **4**: 213-223.
Hostachy B., Allex D. 1993. Un nouveau défi pour les maraîchers des Antilles françaises: un géminivirus de la tomate transmis par Bemisia tabaci, *Phytoma,* **456**: 24-28.
Kato K., Matsumoto T., Koiwai A., Mizusaki S., Nishida K., Noguchi M., and Tamaki E., 1972. Liquid Suspension Culture of Tobacco Cells. *Ferment. Technol. Today,* 689-695.
Kheyr-Pour, A., Bendahmane, M., Matzeit, V., Accotto, G.P., Crespi, S., and Gronenbom, B. 1991. Tomato yellow leaf curl virus from Sardinia is a whitefly-transmitted monopartite geminivirus. *Nucleic Acids Res*, **19:** 6763-9.
Kunkel T.A. 1985. Rapid and efficient site-specific mutagenesis without phenotypic selection. *Proc. Natl. Acad. Sci*., **82:** 488-492.
Lazarowitz S.G. 1992a. Geminiviruses: genome structure and gene function. *Critical Reviews in Plant Sciences,* **11:** 327-349.
Lazarowitz S.G., Leeju C.W., Rogers S.G. and Elmer S. 1992b. Sequence-specific interaction with the viral AL1 protein identifies a geminivirus DNA replication origin. *The Plant Cell*, **4**: 799-809.
Longstaff M., Brignetti G., Boccard F., Chapman S. and Baulcombe D. 1993. Extreme resistance to potato virus infection in plants expressing a modified component of putative viral replicase. *Embo J*., **12**: 379-386.
Matzeit V., Schaefer S., Kammann M., Schalk H., Schell J. and Gronenbom B. 1991. Wheat dwarf virus vectors replicate and express foreign genes in cells of monocotyledonous plants. *The Plant Cell,* **3**: 247-258.
Mettouchi A. 1992. Mutants négatifs dominants dans le site de liaison de NTP de la protéine C1 du virus de la courbure et du jaunissement des feuilles de la tomate. Rapport de DEA de Microbiologie. PASTEUR.
Nagata T., Nemoto Y. and Haseawa S., 1992. Tobacco BY-2 Cell Line as the "Hela" Cell in the Cell Biology of Higher Plants. *International revue of Cytology:* **132***.*
Nagy, J.I. and Maliga P., 1976. Callus induction and plant regeneration from mesophyl protoplasts of *Nicotina Sylvestris. Z. Pflanzenphysiol.:* **78**: 453-455.
Navot N., Ber R. and Czosnek H. 1989. Rapid detection of tomato yellow leaf curl virus in squashes of plants and insect vectors. *Phytopathology,* **79:** 562-568.
Navot N., Pichersky E., Zeidan M., Zamir D. and Czosnek H. 1991. Tomato yellow leaf curl virus: a whitefly-transmitted geminivirus with a single genomic component. *Virology*, **185**: 151-161.
Reiss B., Sprengel R., Will H., Schaller H., 1984. A new sensitive method for qualitative and quantitative assay of neomycin phosphotransferase in crude cell extracts. *Gene* **30**: 211-218.
Rochester D.E., Kositratana, W., and Beachy, R.N. 1990. Systemic movement and symptom production following agroinoculation with a single DNA of tomato yellow leaf curl geminivirus (Thailand). *Virology*, **178**: 520-6.
Sambrook, Fritsch and Maniatis. Molecular cloning A laboratory manual. *Cold Spring Harbor: Cold Spring Harbor Laboratory Press,* 1989.
Sanger F., Nicklen S., and Coulson A.R., 1977. DNA sequencing with chain-terminating inhibitors. *Proc. Natl. Acad. Sci.,* **74**: 5463-5467.
Sanger F., 1981. Determination of Nucleotide Sequences in DNA. Science, **214,** 1205-1210.
Saunders K., Lucy A. and Stanley J. 1991. DNA forms of the geminivirus African cassava mosaic virus consistent with a rolling circle mechanism of replication. *Nucleic Acids Res.* **19**: 2325-2330.
Stenger D.C., Revington G.N., Stevenson M.C. and Bisaro, D.M. 1991. Replicational release of geminivirus genomes from tandemly repeated copies: evidence for rolling-circle replication of a plant viral DNA. *Proc Natl Acad Sci U S A,* **88**: 8029-33.
Sung P., Hooggins D., Prakash L. and Prakash S. 1988. Mutation of Lysine-48 in the yeast RAD3 protein abolishes its ATPase and DNA helicase activities but not the ability to bind ATP. *Embo J.,* **7**: 3263-3269.
Sung P., Hoggins D., Prakash L., Prakash S., 1988. Mutation of Lys.68 to Arg. in yeast RAD3 protein abolishes its ATPase and DNA helicase activities but not the ability to bind ATP. *EMBO J*., **7**: 3263-3269.
Sunter G., Hartitz M.D. and Bisaro D.M. 1993. Tomato golden mosaic virus leftward gene expression: autoregulation of geminivirus replication protein. *Virology,* **195**: 275-80.
Thömmes P., Osman T.A.M., Hayes R.J. and Buck K.W. 1993. TGMV replication protein AL1 preferentially binds to single-stranded DNA from the common region. *FEBS,* **319:** 95-99.
Townsend R., Watts J., Stanley J., 1986. Synthetis of viral DNA forms in *Nicotina plumbaginifolia* protoplasts inoculated with cassava latent virus (CLV); evidence for independent replication of one component of the CLV genome. *Nucleic Acids Res.,* **14**: 1253-1266.
Traktman P., McDonald W., Klemperer N. and Ghosh R. 1993. Molecular genetics and biochemical analysis of vaccinia virus DNA replication. *in* Abstract of the IXth International Congress of Virology of Glasgow (Scotland), 8-13 August.
Von Arnim A. and Stanley J. 1992. Inhibition of African cassava mosaic virus systemic infection by a movement protein from the related geminivirus tomato golden mosaic virus. *Virology,* **187**: 555-64.
Walker J.E., Saraste M., Runswick M.J., Gay N.J., 1982. Distantly related sequences in the α- and β-subunits of ATP synthase, kinases and other ATP-requiring enzymes and a common nucleotide-binding site. *EMBO J*., **1**: 945-951.
Zakay Y., Navot N., Zeidan M., Rabinowitch H., Czosnek H. and Zamir D. 1990. Screening of Lycopersicon accessions for resistance to tomato yellow leaf curl virus: presence of viral DNA and symptom development. *Plant Dis.,* **75:** 279-281.

## Revendications

1. Utilisation d'une séquence nucléotidique mutée codant pour une protéine C1 inactive correspondant à la protéine C1 active des géminivirus, dans laquelle la lysine située entre les positions 220 et 235 de ladite protéine C1 active est remplacée par l'alanine, l'arginine ou l'histidine, et de préférence par l'alanine, pour l'obtention de plantes résistantes ou tolérantes aux géminivirus.

2. Utilisation selon la revendication 1, **caractérisée en ce que** la protéine C1 inactive correspond à la protéine C1 active de virus TYLC, dans laquelle la lysine en position 227 est substituée par une alanine.

3. Utilisation selon la revendication 1 ou 2, **caractérisée en ce qu'**au moins une séquence nucléotidique mutée est utilisée pour la transformation de cellules de plantes en vue de l'obtention de plantes transgéniques résistantes ou tolérantes aux géminivirus.

4. Utilisation selon l'une des revendications 1 à 3, **caractérisée en ce que** les séquences nucléotidiques mutées sont utilisées pour la transformation de protoplastes, ou encore pour la construction de vecteurs, tels que des plasmides, ou pour la transformation de bactéries telles que *Agrobacterium tumefaciens,* susceptibles de transformer à leur tour des cellules de plantes.

5. Procédé d'obtention de plantes transgéniques résistantes aux géminivirus, **caractérisé en ce qu'**il comprend une étape de transformation de cellules de plantes, à l'aide d'au moins une séquence nucléotidique mutée telle que définie dans la revendication 1 ou 2, le cas échéant, insérée dans un vecteur ou une bactérie, tels que définis dans la revendication 4, suivie de la régénération des plantes à partir des cellules ainsi transformées.

6. Procédé selon la revendication 5, **caractérisé en ce qu'**il est réalisé par transformation des cellules d'un fragment d'une plante, notamment de disques foliaires, à l'aide de bactéries *Agrobacterium tumefaciens,* elles-mêmes transformées par au moins une séquence nucléotidique mutée selon la revendication 1 ou 2, suivie de la régénération des plantes à partir des cellules ainsi transformées, notamment à partir de bourgeons formés à la périphérie des disques foliaires susmentionnés.

7. Cellules transgéniques de plantes possédant un ADN hétérologue intégré de façon stable dans leur génome, ledit ADN hétérologue contenant un promoteur reconnu par les polymérases desdites cellules de plantes, et au moins une séquence nucléotidique mutée telle que définie dans la revendication 1 ou 2, ladite séquence nucléotidique mutée étant capable d'inhiber totalement ou partiellement la réplication et/ou la diffusion et/ou la distribution de géminivirus, notamment capable de coder, sous le contrôle dudit promoteur, pour une protéine susceptible d'inhiber totalement ou partiellement la réplication et/ou la diffusion et/ou la distribution de géminivirus au sein desdites cellules transformées,
les cellules de *Nicotina tabaccum* cultivar BY2 (pour bright yellow 2) étant exclues.

8. Cellules de plantes selon la revendication 7, qui peuvent être régénérées en une plante transgénique résistante ou tolérante à une ou plusieurs souches de géminivirus, et capable de produire des semences, elles-mêmes résistantes ou tolérantes auxdits virus.

9. Cellules de plantes selon la revendication 7 ou la revendication 8, qui sont transformées selon le procédé de la revendication 5 ou la revendication 6.

10. Semences transgéniques possédant un ADN hétérologue intégré de façon stable dans le génome de leurs cellules, ledit ADN hétérologue contenant le cas échéant un promoteur reconnu par les polymérases desdites cellules, et au moins une séquence nucléotidique mutée, telle que définie dans la revendication 1 ou 2, ladite séquence nucléotidique mutée étant capable d'inhiber totalement ou partiellement la réplication et/ou la diffusion et/ou la distribution de géminivirus, notamment capable de coder, sous le contrôle dudit promoteur, pour une protéine susceptible d'inhiber totalement ou partiellement la réplication et/ou la diffusion et/ou la distribution de géminivirus phytopathogènes au sein desdites cellules transformées.

11. Semences selon la revendication 10, qui sont capables de germer en une plante résistante aux géminivirus.

12. Semences selon la revendication 10 ou 11, qui sont transformées par le procédé selon la revendication 5 ou la revendication 6.

13. Plantes transgéniques résistantes ou tolérantes aux géminivirus comportant un ADN hétérologue intégré de façon stable dans le génome de leurs cellules, ledit ADN hétérologue contenant le cas échéant un promoteur reconnu par les polymérases desdites cellules de plantes, et au moins une séquence nucléotidique mutée selon la revendication 1 ou 2, ladite séquence nucléotidique mutée étant capable d'inhiber totalement ou partiellement la réplication et/ou la diffusion et/ou la distribution de géminivirus, notamment capable de coder, sous le contrôle dudit promoteur, pour une protéine susceptible d'inhiber totalement ou partiellement la réplication et/ou la diffusion et/ou la distribution de géminivirus au sein desdites cellules transformées,
les plantes de *Nicotina benthamiana* ainsi transformées étant exclues.

14. Plantes selon la revendication 13, capables de produire des semences résistantes aux géminivirus.

15. Plantes selon la revendication 13 ou 14, telles qu'obtenues par mise en oeuvre d'un procédé selon la revendication 5 ou la revendication 6.

## Claims

1. Use of a mutated nucleotide sequence coding for an inactive C1 protein corresponding to the active C1 protein of the geminiviruses, in which the lysine situated between positions 220 and 235 of the said active C1 protein is replaced by alanine, arginine or histidine, and preferably by alanine, for obtaining plants that are resistant or tolerant to the geminiviruses.

2. Use according to claim 1, **characterized in that** the inactive C1 protein corresponds to the active C1 protein of the virus TYLC, in which the lysine at position 227 is substituted by an alanine.

3. Use according to claim 1 or 2, **characterized in that** at least one mutated nucleotide sequence is used for the transformation of plant cells in order to obtain transgenic plants that are resistant or tolerant to geminiviruses.

4. Use according to one of the claims 1 to 3, **characterized in that** the mutated nucleotide sequences are used for the transformation of protoplasts, or for the construction of vectors, such as plasmids, or for the transformation of bacteria such as *Agrobacterium tumefaciens*, capable in their turn of transforming plant cells.

5. A method of obtaining transgenic plants that are resistant to geminiviruses, **characterized in that** it includes a stage of transformation of plant cells, by means of at least one mutated nucleotide sequence as defined in claim 1 or 2, if necessary inserted in a vector or a bacterium, as defined in claim 4, followed by regeneration of the plants starting from the cells thus transformed.

6. A method according to claim 5, **characterized in that** it is carried out by transformation of the cells of a fragment of a plant, especially leaf discs, by means of bacteria *Agrobacterium tumefaciens*, themselves transformed by at least one mutated nucleotide sequence according to claim 1 or 2, followed by the regeneration of plants starting from the cells thus transformed, especially from buds formed at the periphery of the aforementioned leaf discs.

7. Transgenic cells of plants possessing a heterologous DNA stably integrated in their genome, the said heterologous DNA containing a promoter that is recognized by the polymerases of the said plant cells, and at least one mutated nucleotide sequence as defined in claim 1 or 2, the said mutated nucleotide sequence being capable of inhibiting totally or partially the replication and/or diffusion and/or distribution of geminiviruses, especially being capable of coding, under the control of the said promoter, for a protein that is able to inhibit totally or partially the replication and/or diffusion and/or distribution of geminiviruses within the said transformed cells,
the cells of *Nicotiana tabaccum* cultivar BY2 (bright yellow 2) being excluded.

8. Plant cells according to claim 7, which can be regenerated into a transgenic plant that is resistant or tolerant to one or more strains of geminiviruses, and is capable of producing seeds, which in turn are resistant or tolerant to the said viruses.

9. Plant cells according to claim 7 or claim 8, which are transformed according to the method of claim 5 or Claim 6.

10. Transgenic seeds possessing a heterologous DNA stably integrated in the genome of their cells, the said heterologous DNA containing if necessary a promoter that is recognized by the polymerases of the said cells, and at least one mutated nucleotide sequence, as defined in claim 1 or 2, the said mutated nucleotide sequence being capable of inhibiting totally or partially the replication and/or diffusion and/or distribution of geminiviruses, and especially being capable of coding, under the control of the said promoter, for a protein that is able to inhibit totally or partially the replication and/or diffusion and/or distribution of geminiviruses within the said transformed cells.

11. Seeds according to claim 10, which are capable of germinating into a plant that is resistant to geminiviruses.

12. Seeds according to claim 10 or 11, which are transformed by the method according to claim 5 or claim 6.

13. Transgenic plants that are resistant or tolerant to geminiviruses containing a heterologous DNA stably integrated in the genome of their cells, the said heterologous DNA containing if necessary a promoter that is recognized by the polymerases of the said plant cells, and at least one mutated nucleotide sequence according to claim 1 or 2, the said mutated nucleotide sequence being capable of inhibiting totally or partially the replication and/or diffusion and/or distribution of geminiviruses, and especially being capable of coding, under the control of the said promoter, for a protein that is able to inhibit totally or partially the replication and/or diffusion and/or distribution of geminiviruses within the said transformed cells,
plants of *Nicotiana benthamiana* thus transformed being excluded.

14. Plants according to claim 13, capable of producing seeds that are resistant to geminiviruses.

15. Plants according to claim 13 or 14, such as are obtained by carrying out a method according to claim 5 or claim 6.

## Patentansprüche

1. Verwendung einer mutierten Nucleotidsequenz, die für ein inaktives C1-Protein codiert, das dem aktiven C1-Protein von Geminiviren entspricht, worin das zwischen den Positionen 220 und 235 des aktiven C1-Proteins angeordnete Lysin durch Alanin, Arginin oder Histidin, vorzugsweise durch Alanin, ersetzt ist, um Pflanzen zu erhalten, die gegenüber Geminiviren resistent oder tolerant sind.

2. Verwendung nach Anspruch 1, **dadurch gekennzeichnet, dass** das inaktive C1-Protein dem aktiven C1-Protein des TYLC-Virus entspricht, worin das Lysin in der Position 227 durch Alanin ersetzt ist.

3. Verwendung nach Anspruch 1 oder 2, **dadurch gekennzeichnet, dass** mindestens eine mutierte Nucleotidsequenz für die Transformation von Pflanzenzellen im Hinblick auf die Gewinnung transgener Pflanzen, die gegenüber Geminiviren resistent oder tolerant sind, verwendet wird.

4. Verwendung nach einem der Ansprüche 1 bis 3, **dadurch gekennzeichnet, dass** die mutierten Nucleotidsequenzen für die Transformation von Protoplasten oder auch für die Konstruktion von Vektoren, wie Plasmiden, oder für die Transformation von Bakterien, wie *Agrobacterium tumefaciens*, die ihrerseits Pflanzenzellen transformieren können, verwendet werden.

5. Verfahren zur Gewinnung transgener Pflanzen, die gegenüber Geminiviren resistent sind, **dadurch gekennzeichnet, dass** es einen Schritt der Transformation von Pflanzenzellen mit Hilfe mindestens einer mutierten Nucleotidsequenz, wie in Anspruch 1 oder 2 definiert, gegebenenfalls inseriert in einen Vektor oder ein Bakterium, wie im Anspruch 4 definiert, gefolgt von der Regenerierung der Pflanzen, ausgehend von den so transformierten Zellen, beinhaltet.

6. Verfahren nach Anspruch 5, **dadurch gekennzeichnet, dass** es durch Transformation der Zellen eines Teils einer Pflanze, insbesondere Blattscheiben, mit Hilfe der Bakterien *Agrobacterium tumefaciens*, die ihrerseits mit mindestens einer mutierten Nucleotidsequenz nach Anspruch 1 oder 2 transformiert sind, gefolgt von Regenerierung der Pflanzen, ausgehend von so transformierten Zellen, insbesondere ausgehend von an der Peripherie der Blattscheiben gebildeten Knospen, durchgeführt wird.

7. Transgene Zellen von Pflanzen, die eine heterologe DNA stabil in ihr Genom integriert haben, wobei die heterologe DNA einen Promotor aufweist, der von den Polymerasen der Pflanzenzellen erkannt wird, und mindestens eine mutierte Nucleotidsequenz, wie in Anspruch 1 oder 2 definiert, wobei diese mutierte Nucleotidsequenz fähig ist, die Replikation und/oder Diffusion und/oder Verteilung von Geminivirus vollständig oder teilweise zu hemmen, insbesondere fähig ist, unter der Kontrolle des genannten Promotors für ein Protein zu codieren, das imstande ist, die Replikation und/oder Diffusion und/oder Verteilung von Geminivirus in den transformierten Zellen vollständig oder teilweise zu hemmen,
wobei die Zellen von *Nicotina tabaccum* Kultivar BY2 ("bright yellow 2") ausgeschlossen sind.

8. Pflanzenzellen nach Anspruch 7, die zu einer transgenen Pflanze regeneriert werden können, die gegen einen oder mehrere Geminivirus-Stämme resistent oder tolerant ist und fähig ist, Samen zu produzieren, die ihrerseits gegen die genannten Viren resistent oder tolerant sind.

9. Pflanzenzellen nach Anspruch 7 oder Anspruch 8, die nach dem Verfahren nach Anspruch 5 oder Anspruch 6 transformiert sind.

10. Transgene Samen, die eine heterologe DNA stabil in das Genom ihrer Zellen integriert haben, wobei die heterologe DNA gegebenenfalls einen Promotor aufweist, der von den Polymerasen der Zellen erkannt wird, und mindestens eine mutierte Nucleotidsequenz, wie in Anspruch 1 oder 2 definiert, wobei diese mutierte Nucleotidsequenz fähig ist, die Replikation und/oder Diffusion und/oder Verteilung von Geminivirus vollständig oder teilweise zu hemmen, insbesondere fähig ist, unter der Kontrolle des genannten Promotors für ein Protein zu codieren, das imstande ist, die Replikation und/oder Diffusion und/oder Verteilung von phytopathogenen Geminiviren in den transformierten Zellen vollständig oder teilweise zu hemmen.

11. Samen nach Anspruch 10, die fähig sind, in eine gegenüber Geminiviren resistente Pflanze zu keimen.

12. Samen nach Anspruch 10 oder 11, die nach dem Verfahren nach Anspruch 5 oder Anspruch 6 transformiert sind.

13. Transgene Pflanzen, die gegen Geminiviren resistent oder tolerant sind, die eine heterologe DNA stabil in das Genom ihrer Zellen integriert haben, wobei die heterologe DNA gegebenenfalls einen Promotor aufweist, der von den Polymerasen der Pflanzenzellen erkannt wird, und mindestens eine mutierte Nucleotidsequenz nach Anspruch 1 oder 2, wobei diese mutierte Nucleotidsequenz fähig ist, die Replikation und/oder Diffusion und/oder Verteilung von Geminivirus vollständig oder teilweise zu hemmen, insbesondere fähig ist, unter der Kontrolle des genannten Promotors für ein Protein zu codieren, das imstande ist, die Replikation und/oder Diffusion und/oder Verteilung von Geminivirus in den transformierten Zellen vollständig oder teilweise zu hemmen,
wobei die so transformierten Pflanzen von *Nicotina benthamiana* ausgeschlossen sind.

14. Pflanzen nach Anspruch 13, die fähig sind, gegenüber Geminiviren resistente Samen zu produzieren.

15. Pflanzen nach Anspruch 13 oder 14, wie sie durch Durchführung des Verfahrens nach Anspruch 5 oder Anspruch 6 erhalten werden.
